# EUROPEAN PATENT APPLICATION

(11) **EP 4 831 179 A1**
(43) Date of publication of application: **28.10.2026**
(21) Application number: 25171826.8
(22) Date of filing: 22.04.2025
(51) Int. Cl.: C07D 487/04, A61P 35/00, A61P 37/00, A61P 29/00, A61K 31/519

(54) **4,6 DISUBSTITUTED 7H PYRROLO[2,3 D]PYRIMIDINES AS CDK9 INHIBITORS FOR THERAPEUTIC USE**

(71) Applicant: Ustav Organicke Chemie a Biochemie AV CR v.v.i., 16000 Praha 6 (CZ)
(72) Inventor: Tichy, Michal, Praha (CZ); Hocek, Michal, Praha (CZ); Klecka, Martin, Praha (CZ); Hulek, Filip, Praha (CZ); Vaiedelich, Enzo, Praha (CZ); Mertlikova-Kaiserova, Helena, Praha (CZ); Matousova, Marika, Praha (CZ)
(74) Representative: Harber IP s.r.o.

(57) **Abstract**

The invention provides 4,6-disubstituted-7*H*-pyrrolo[2,3-*d*]pyrimidines of general formula I, 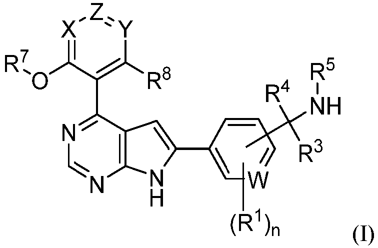 wherein all the variables are defined in the claims. The compounds of this invention are potent and selective inhibitors of CDK9 and are useful in the treatment of leukemias, solid tumors, lymphomas, neutrophilic dermatoses or netrophilic diseases.

## Description

### Field of the invention

The invention provides compounds as selective inhibitors of cyclin-dependent kinase 9 (CDK9), their preparation, pharmaceutical compositions containing these inhibitors and their use for treatment of cancer and/or neutrophilic diseases.

### Background of the Invention

Cyclin-dependent kinases (CDKs) are heterodimers composed of a catalytic kinase subunit and cyclin, a regulatory subunit. CDKs belong to a family of serine/threonine kinases that regulate cell cycle or transcription by phosphorylation of various substrates.

CDK9 together with cyclin T forms RNA polymerase II (RNA PolII) positive transcription elongation factor b (PTEFb), which promotes transcription elongation by phosphorylating Ser-2 of the RNA Pol II. Phosphorylation by CDK9 induces a conformational change that allows RNA Pol II to enter possessive elongation to efficiently transcribe full-length mRNAs. The expression of CDK9 is common in many hematological and solid malignancies where it promotes the expression of short-lived antiapoptotic proteins like MCL-1 and MYC. Increased MCL-1 activity is associated with poor prognosis with some solid tumours (*e.g.,* Shigemasa et al. Jpn. J. Cancer Res. 2002, 93, 542; Likui et al. J. Surg. Oncol. 2009, 100, 396; Nakano et al Anticancer Ress 2020, 40, 1007), some forms of chronic lymphocytic leukemia (*e.g.,* Awan et al. Blood, 2009, 113, 535*;* Johnston et al Leuk. Lymphoma 2004, 10, 2017) and multiple myeloma (Wuilleme-Toumi et al. Leukemia 2005, 19, 1248). Targeting transcription-associated CDK9 represents a therapeutic strategy for treating malignancies dependent on these antiapoptotic proteins, for example Acute Myelocytic Leukemia (AML), Relapsed/Refractory Chronic Lymphocytic Leukemia (CLL), Chronic Myelomonocytic Leukemia (CMML), sarcomas, solid tumors like breast (Human Epidermal Growth Factor Receptor 2 Negative Breast Cancer, Triple-negative breast cancer), lung, ovarian, melanoma and pancreatic cancers including Castration-Resistant Prostate Cancer (Anshabo, A. T. et al. Front. Oncol. 2021, 11, 678559), lymphomas (e.g. Hodgkin lymphoma; diffuse large B-cell lymphoma (Thieme et al. Mol. Cancer 2023, 22, 64); high-grade B-cell lymphoma (Morillo et al. Oncotarget 2023, 14, 749-752), cutaneous T-cell lymphoma (Luo Ch.-H. et al. Nature Comm. 2024, 15, 10594); Extranodal Marginal Zone B-Cell Lymphoma (Mucosa-Associated Lymphoid Tissue or MALT-Lymphoma) (Wang et al Blood Cancer J. 2021, 11, 14), and Peripheral T-Cell Lymphomas (PTCL) (Spinner et al Leukemia 2016, 30, 1520; Koch et al Blood 2019, 133, 566). Inhibition of CDK9 can lead to reactivation of suppressor genes to restrict tumour growth (Zhang et al Cell 2018, 175, 1244).

CDK9 inhibitors can also find therapeutic application in inflammatory neutrophilic diseases. Neutrophils rely on expression of MCL-1 for survival (Wang, K. et al. PloS One 2012, 7, e30128; Murphy and Caraher Immunol. Res. 2015, 62, 225). Downregulation of MCL-1 expression in neutrophils by CDK9 inhibitors rapidly induces apoptosis and promotes neutropenia. Neutrophilic diseases that would benefit from rapid, acute normalization of dysregulated neutrophil activity would include but not limited to autoimmune diseases (Fu et al MedComm 2021, 2, 402) such as rheumatoid arthritis (Wright, H. L. et al Nat. Rev. Rheumatol. 2014, 10, 593-601), systemic lupus erythematosus or antiphospholipid syndrome (Wirestam, L. et al Front. Imunol. 2019, 10, 2734), systemic sclerosis (Impellizzieri et al Allergy 2022, 77, 1274) and Sjögren's syndrome (Gudbjörnsson et al Ann. Rheum. Dis. 1991, 50, 685), and in other inflammatory disease such as cystic fibrosis (Yonker et al J. Cyst. Fibro. 2021, 20, 1062; Gray et al Thorax 2017, 210134), severe asthma (Jatakanon et al Am. J. Respir. Crit. Care Med. 1999, 160, 1532; Crisford et al Thorax 2020, 215986 ), chronic obstructive pulmonary disease (Butler et al COPD, 2018, 15, 392), bronchiectasis (Bedi et al Am. J. Respir. Crit. Care Med. 2018, 198, 880; Giam et al Eur. Respir. J. 2021, 58, 2003157), neutrophilic dermatoses such as psoriasis, prurigo nodularis, palmoplantar pustulosis, hidradenitis suppurativa and pyoderma gangrenosum (Weiss, E. H. et al Curr. Dermatology Reports 2022, 11, 89-102), diabetic nephropathy (Yang, X. et al Toxicol. Appl. Pharmacol. 2021, 416, 115465), regeneration after myocardial infarction (Kaveh, A. et al Development, 2022, 149, dev199636) or in treatment of acute liver failure (ALF), which was recently linked with upregulation of MYC (Kolodziejczyk, A. A. et al Nat. Med. 2020, 26, 1899-1911). CDK9 regulates mitochondrial function, where certain metabolic dysfunctions may be controlled by CDK9 inhibition (Arisan et al Curr. Pharm. Des. 2014, 20, 180; Itkonen et al Neoplasia 2019, 21, 713).

With all the above-mentioned functions and pathological roles of CDK9 activity, there is an urgent need for small molecule selective inhibitors of CDK9. Although CDK9 is a well-established therapeutic target, there is no FDA approved CDK9 inhibitor on the market. The most advanced and selective CDK9 inhibitor developed for oral administration, **KB0742,** was recently discontinued.

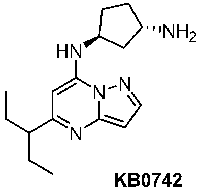

### Summary of the Invention

This invention describes novel 7H-pyrrolo[2,3-d]pyrimidines substituted at positions 4 and 6 of the general formula I which possess activity as potent and selective inhibitors of CDK9. These compounds show cytotoxic and cytostatic activity on cell lines of tumor origin that are dependent on MCL-1 and MYC for survival. The compounds of the invention also regulate neutrophil counts which makes them suitable for treatment of neutrophilic diseases, in particular inflammatory neutrophilic diseases.

The present invention addresses the need for orally available, potent and highly selective CDK9 inhibitors with favorable ADME properties, in vivo efficacy and reduced toxicity.

The combination of substituents in positions 4 and 6 of the 7*H*-pyrrolo[2,3-*d*]pyrimidine skeleton is necessary for the strong and selective binding of the compounds of the invention to CDK9. The main features of the substitution pattern are a 6-membered hetaryl ring (pyridine or pyrimidine) substituted by at least one *ortho*-alkoxy group, attached at the position 4 of the nucleobase, and a *meta-* or *para-*(aminomethyl)phenyl moiety at position 6 on the nucleobase.

One aspect of the present invention are 4,6-disubstituted-7*H*-pyrrolo[2,3-*d*]pyrimidines of general formula I 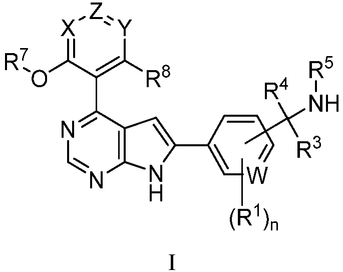 wherein
W is selected from CH, CR¹ or N;
R¹ is on each occurence independently selected from halogen, C1-C4 alkyl, hydroxy, C1-C4 alkoxy; or R¹ forms a ring with R³ or R⁵ as described herein below;
n = 0, 1 or 2;
the substituent -C(R³)(R⁴)-NH(R⁵) is preferably bound in position meta or para relative to the pyrrole ring attachment;
R³ is selected from C1-C6 alkyl, C1-C3 haloalkyl, C1-C3 hydroxyalkyl, C3-C8 cycloalkyl, C4-C8 cycloalkylalkyl, hydrogen;
or R³ and R¹ which is in a position adjacent to the position of the substituent -C(R³)(R⁴)-NH(R⁵), together with the carbon atoms linking them, form a 5- to 8-membered saturated cycloalkyl or heterocycloalkyl ring, wherein the heterocycloalkyl ring contains 1 or 2 heteroatoms selected from N, O, S, and wherein the ring formed by R³ and R¹ together with the carbon atoms linking them can optionally be substituted by one, two or three substituents selected from halogen (preferably fluorine), C1-C4 alkyl, amino, hydroxy, C1-C4 alkoxy;
R⁴ is selected from C1-C6 alkyl, C3-C8 cycloalkyl, C4-C8 cycloalkylalkyl, hydrogen;
or R³ and R⁴ together with the carbon atom to which they are bound form a 3- to 8-membered cycloalkyl or heterocycloalkyl or spirocycloalkyl ring, wherein the heterocycloalkyl ring contains 1 or 2 heteroatoms selected from N, O, S, and wherein the ring formed by R³ and R⁴ together with the carbon atom to which they are bound can optionally be substituted by one, two or three substituents selected from halogen (preferably fluorine), C1-C4 alkyl, amino, hydroxy, C1-C4 alkoxy;
or R³ and R⁵ together with the carbon and nitrogen atom linking them form a 5- to 8-membered (preferably 5- to 6-membered) saturated heterocycloalkyl ring, optionally containing 1 or 2 further heteroatoms selected from N, O, S, and optionally substituted by one, two or three substituents selected from halogen (preferably fluorine), C1-C4 alkyl, amino, hydroxy, C1-C4 alkoxy;
R⁵ is selected from C1-C6 alkyl, C1-C4 haloalkyl (preferably trifluoromethyl), C3-C8 cycloalkyl, C4-C8 cycloalkylalkyl, hydrogen;
or R⁵ and R¹ when R¹ is in a position adjacent to the position of the substituent -C(R³)(R⁴)-NH(R⁵), together with the carbon and nitrogen atoms linking them, form a 5- to 8-membered (preferably 5-to 6-membered) heterocycloalkyl ring, optionally containing 1 or 2 further heteroatoms selected from N, O, S, and optionally substituted by one, two or three substituents selected from halogen (preferably fluorine), C1-C4 alkyl, amino, hydroxy, C1-C4 alkoxy;
X, Y and Z are selected from CH, C-R⁶ and N, wherein one or two of X, Y, Z are N;
R⁶ is selected from halogen, C1-C4 alkyl, C1-C4 halogenalkyl, C1-C4 alkoxy, C1-C4 halogenalkoxy and hydrogen;
R⁷ is selected from C1-C4 alkyl, trifluoromethyl, C3-C6 cycloalkyl;
R⁸ is selected from C1-C4 alkyl, C1-C4 alkoxy, trifluoromethyl, hydrogen;
and pharmaceutically acceptable salts thereof.

In some embodiments, X and Y are N and Z is C.

In some embodiments, X is N and Y and Z are C.

In some embodiments, Z is N and X and Y are C.

In a preferred embodiment, R¹ is independently selected from fluorine, hydroxy, C1-C4 alkyl, C1-C4 alkoxy. More preferably, R¹ is independently selected from fluorine and methyl.

Preferably, n is 1.

In a preferred embodiment, R⁶ is selected from hydrogen, fluorine, chlorine, methyl, methoxy, trifluoromethyl, trifluoromethoxy. Preferably, only one R⁶ is present.

In a preferred embodiment, R³ is selected from C1-C6 alkyl, C1-C3 haloalkyl, C1-C4 hydroxyalkyl, C3-C6 cycloalkyl, C4-C6 cycloalkylmethyl. More preferably, R³ is selected from methyl, ethyl, *n-*propyl, 2-methylpropyl, butyl, *i*-propyl, trifluoromethyl, hydroxymethyl, cyclopropyl, cyclobutyl, cyclopentyl, cyclohexyl, cyclobutylmethyl.

In some embodiments, R³ and R¹ which is in a position adjacent to the position of the substituent - C(R³)(R⁴)-NH(R⁵), together with the carbon atoms linking them, form a 5- to 8-membered saturated cycloalkyl or heterocycloalkyl ring. The cycloalkyl ring is preferably selected from 5- or 6-membered cycloalkyl, more preferably the cycloalkyl ring is 6-membered cycloalkyl ring. The heterocycloalkyl ring contains 1 or 2 heteroatoms selected from N, O, S, preferably O, and preferably is a 6-membered ring.

The ring formed by R³ and R¹ together with the carbon atoms linking them is optionally substituted by one, two or three substituents, preferably by one or two substituents). The substituents are preferably selected from fluorine and C1-C3 alkyl, such as methyl.

More preferably, R³ and R¹ which is in a position adjacent to the position of the substituent -C(R³)(R⁴)-NH(R⁵), together with the carbon atoms linking them, form tetrahydropyran and cyclohexyl.

In some embodiments, R³ is selected from C1-C6 alkyl, C1-C3 haloalkyl, C1-C3 hydroxyalkyl, C3-C8 cycloalkyl, C4-C8 cycloalkylalkyl, and R⁴ is hydrogen. Preferably, R³ is selected from methyl, ethyl, propyl, 2-methylpropyl, butyl, isopropyl, hydroxymethyl, cyclopentyl, cyclohexyl, cyclobutylmethyl, trifluoromethyl, and R⁴ is hydrogen.

In some embodiments, R³ and R⁴ are the same and are selected from C1-C6 alkyl or hydrogen, more preferably, R³ and R⁴ are selected from methyl, ethyl, n-propyl, i-propyl, butyl and 2-methylpropyl.

In some embodiments, R³ and R⁴ together with the carbon atom to which they are bound form a 3- to 8-membered cycloalkane or heterocycloalkane or spirocycloalkane ring, wherein the heterocycloalkane ring contains 1 or 2 heteroatoms selected from N, O, S, preferably one heteroatom O. The ring can optionally be substituted by one or two halogens (preferably fluorine). More preferably, R³ and R⁴ together with the carbon atom to which they are bound form cyclopropane, cyclobutane, cyclopentane, cyclohexane, cycloheptane, tetrahydropyran, oxetane, tetrahydrofuran, spiro[2.3]hexane or 2,2-difluorocyclobutane ring.

When R³ forms a ring with R¹ or R⁵, R⁴ is preferably hydrogen.

In some embodiments, R³ and R⁵ together with the carbon and nitrogen atom to which they are attached form a 5- to 8-membered (preferably 5- to 6-membered) heterocycloalkane ring, optionally containing 1 or 2 further heteroatoms selected from N, O, S, preferably N. The ring may optionally be substituted by one, two or three substituents selected from halogen (preferably fluorine), methyl, amino, hydroxy, methoxy. More preferably, R³ and R⁵ together with the carbon and nitrogen atom to which they are attached form 5- to 6-membered heterocycloalkane ring. Most preferably, R³ and R⁵ together with the carbon and nitrogen atom to which they are attached form piperazine or pyrrolidine.

In some embodiments, R⁵ is selected from C1-C6 alkyl, trifluoromethyl, C3-C6 cycloalkyl, hydrogen. Most preferably, R⁵ is selected from hydrogen, methyl, ethyl, cyclopropyl, i-propyl and butyl.

In some embodiments, R⁵ and R¹ when R¹ is in a position adjacent to the position of the substituent - C(R³)(R⁴)-NH(R⁵), together with the carbon and nitrogen atoms linking them, form a 5- to 8-membered saturated heterocycloalkane ring, optionally containing 1 or 2 further heteroatoms selected from N, O, S, and optionally substituted by one, two or three substituents selected from halogen (preferably fluorine), C1-C4 alkyl, amino, hydroxy, C1-C4 alkoxy. More preferably, R⁵ and R¹ when it is in a position adjacent to the position of the substituent -C(R³)(R⁴)-NH(R⁵), together with the carbon and nitrogen atoms to which they are bound, form a 5- to 6-membered saturated heterocycloalkane ring, optionally containing 1 further heteroatom selected from N, O, S and optionally substituted by one, two or three substituents selected from halogen (preferably fluorine) and C1-C4 alkyl. Most preferably, R⁵ and R¹ when R¹ is in a position adjacent to the position of the substituent -C(R³)(R⁴)-NH(R⁵), together with the carbon and nitrogen atoms linking them, form 2-methylpiperidine, 3,3-dimethylpiperidine, piperidine, pyrrolidine.

In some embodiments, R⁷ is selected from C1-C4 alkyl, C3-C6 cycloalkyl. More preferably, R⁷ is selected from methyl, ethyl, trifluoromethyl, cyclopropyl and *i*-propyl.

In some embodiments, R⁸ is selected from C1-C4 alkoxy, and hydrogen. More preferably, R⁸ is selected from hydrogen or methoxy.

As described herein and unless otherwise indicated, the individual substituents have the following meanings:
"alkyl" is a linear or branched saturated hydrocarbon chain containing the number of carbons indicated at the place of use of the term. Alkyls may include, but are not limited to, methyl, ethyl, propyl, *iso*propyl, butyl, 2-(methyl)propyl, isobutyl, *tert-butyl.*
"cycloalkyl" is a saturated cyclic hydrocarbon residue, wherein all carbon atoms of the cycloalkyl are part of the cycle. The cycloalkyl comprises one cycle. Examples of cycloalkyl include cyclopropyl, cyclobutyl, cyclopentyl, cyclohexyl and cycloheptyl.
"cycloalkylalkyl" is a cycloalkyl residue bound via an alkylene linker. Example of cycloalkylalkyl is cycloalkylmethyl.
"halogen" means F, Cl, Br or I.
"amino" denotes -NH₂.
"alkoxy" refers to a group of -*O*-alkyl (-OR'), where R' corresponds to the definition of alkyl; an example of an alkoxy group is methoxy (CH₃O-) or ethoxy (CH₃CH₂O-).
"hydroxy" denotes -OH.

Cycloalkyl and heterocycloalkyl rings typically have 3 to 8 members, preferably 5 to 8 members, even more preferably 5 to 6 members.

As used herein, the term "pharmaceutically acceptable salts" refers to salts that retain the biological effectiveness and properties of the claimed compounds of general formula I according to this invention, and which are within reasonable medical judgment suitable for use in contact with the tissues of humans and lower animals without undue toxicity, irritation, allergic reactions, and the like, and have an acceptable benefit/risk ratio. In many cases, the compounds of the present invention are capable of forming acid and/or base salts by virtue of the presence of amino and/or carboxyl groups or groups similar thereto (e.g., phenol or hydroxyamic acid). Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Lists of additional suitable salts can be found, e.g., in Remington's Pharmaceutical Sciences, 20th ed., Mack Publishing Company, Easton, Pa., (1985).

In a preferred embodiment, the present invention provides the following 4,6-disubstituted-7*H-*pyrrolo[2,3-*d*]pyrimidines of formula **I**:

### Systematic name

{4-[4-(2,4-dimethoxypyrimidin-5-yl)-7*H*-pyrrolo[2,3-*d*]pyrimidin-6-yl]-2-fluorophenyl}methanamine,
1-{4-[4-(2,4-dimethoxypyrimidin-5-yl)-7*H*-pyrrolo[2,3-*d*]pyrimidin-6-yl]phenyl}propan-1-amine,
1-{4-[4-(2,4-dimethoxypyrimidin-5-yl)-7*H*-pyrrolo[2,3-*d*]pyrimidin-6-yl]phenyl}butan-1-amine,
4-{4-[4-(2,6-dimethoxypyrimidin-5-yl)-7*H*-pyrrolo[2,3-*d*]pyrimidin-6-yl]phenyl}-3,3-difluorocyclobutan-1-amine,
3-{4-[4-(2,4-dimethoxypyrimidin-5-yl)-7*H*-pyrrolo[2,3-*d*]pyrimidin-6-yl] }tetrahydro-2*H*-pyran-3-amine,
4-(2,4-dimethoxypyrimidin-5-yl)-6-[4-(piperazin-2-yl)phenyl]-7*H*-pyrrolo[2,3-*d*]pyrimidine,
1-{4-[4-(2,4-dimethoxypyrimidin-5-yl)-7*H*-pyrrolo[2,3-*d*]pyrimidin-6-yl]phenyl}-*N*-ethylpropan-1-amine,
6-[4-(2,4-dimethoxypyrimidin-5-yl)-7*H*-pyrrolo[2,3-*d*]pyrimidin-6-yl]-5-fluoro-1,2,3,4-tetrahydroisoquinoline,
1-{4-[4-(2,4-dimethoxypyrimidin-5-yl)-7*H*-pyrrolo[2,3-*d*]pyrimidin-6-yl]phenyl}cyclohexan-1-amine,
7-[4-(2,4-dimethoxypyrimidin-5-yl)-7*H*-pyrrolo[2,3-*d*]pyrimidin-6-yl]-6-fluoro-1,2,3,4-tetrahydroisoquinoline,
6-[4-(2,4-dimethoxypyrimidin-5-yl)-7*H*-pyrrolo[2,3-*d*]pyrimidin-6-yl]-4,4-dimethyl-1,2,3,4-tetrahydroisoquinoline,
1-{4-[4-(2,4-dimethoxypyrimidin-5-yl)-7*H*-pyrrolo[2,3-*d*]pyrimidin-6-yl]-3-fluorophenyl}-*N-*methylpropan-1-amine,
1-{4-[4-(2,4-dimethoxypyrimidin-5-yl)-7*H*-pyrrolo[2,3-*d*]pyrimidin-6-yl]-3-fluorophenyl}-*N*-methylbutan-1-amine,
1-{4-[4-(2,4-dimethoxypyrimidin-5-yl)-7*H*-pyrrolo[2,3-*d*]pyrimidin-6-yl]phenyl}cycloheptan-1-amine,
4-{4-[4-(2,4-dimethoxypyrimidin-5-yl)-7*H*-pyrrolo[2,3-*d*]pyrimidin-6-yl]phenyl}tetrahydro-2*H*-pyran-4-amine,
(*R*)-7-[4-(2,4-dimethoxypyrimidin-5-yl)-7*H*-pyrrolo[2,3-*d*]pyrimidin-6-yl]chroman-4-amine,
2-{3-[4-(2,4-dimethoxypyrimidin-5-yl)-7*H*-pyrrolo[2,3-*d*]pyrimidin-6-yl]phenyl}propan-2-amine,
1-{4-[4-(2,4-dimethoxypyrimidin-5-yl)-7*H*-pyrrolo[2,3-*d*]pyrimidin-6-yl]phenyl}-3-methylbutan-1-amine,
1-{3-[4-(2,4-dimethoxypyrimidin-5-yl)-7*H*-pyrrolo[2,3-*d*]pyrimidin-6-yl]phenyl}cyclopropan-1-amine,
3-{4-[4-(2,4-dimethoxypyrimidin-5-yl)-7*H*-pyrrolo[2,3-*d*]pyrimidin-6-yl]phenyl}pentan-3-amine,
1-{4-[4-(2,4-dimethoxypyrimidin-5-yl)-7*H*-pyrrolo[2,3-*d*]pyrimidin-6-yl]-3-fluorophenyl}cyclohexan-1-amine,
4-{4-[4-(2,4-dimethoxypyrimidin-5-yl)-7*H*-pyrrolo[2,3-*d*]pyrimidin-6-yl]phenyl}heptan-4-amine,
5-{4-[4-(2,4-dimethoxypyrimidin-5-yl)-7*H*-pyrrolo[2,3-*d*]pyrimidin-6-yl]phenyl}nonan-5-amine,
4-{4-[4-(2,4-dimethoxypyrimidin-5-yl)-7*H*-pyrrolo[2,3-*d*]pyrimidin-6-yl]phenyl}-2,6-dimethylheptan-4-amine,
5-{4-[4-(2,4-dimethoxypyrimidin-5-yl)-7*H*-pyrrolo[2,3-*d*]pyrimidin-6-yl]phenyl}spiro[2,3]hexan-5-amine,
3-{4-[4-(2,4-dimethoxypyrimidin-5-yl)-7*H*-pyrrolo[2,3-*d*]pyrimidin-6-yl]phenyl}-2,4-dimethylpentan-3-amine,
6-[4-(2,4-dimethoxypyrimidin-5-yl)-7*H*-pyrrolo[2,3-*d*]pyrimidin-6-yl]-1,2,3,4-tetrahydroisoquinoline,
4-(2,4-dimethoxypyrimidin-5-yl)-6-[(4-pyrrolidin-2-yl)phenyl]-7*H*-pyrrolo[2,3-*d*]pyrimidine,
7-[4-(2,4-dimethoxypyrimidin-5-yl)-7*H*-pyrrolo[2,3-*d*]pyrimidin-6-yl]-1,2,3,4-tetrahydroisoquinoline,
4-(2,4-dimethoxypyrimidin-5-yl)-6-(isoindolin-5-yl)-7*H*-pyrrolo[2,3-*d*]pyrimidine,
*N*-{3-[4-(2,4-dimethoxypyrimidin-5-y1)-7*H*-pyrrolo[2,3-*d*]pyrimidin-6-yl]benzyl}cyclopropanamine,
4-(2,4-dimethoxypyrimidin-5-yl)-6-[(3-pyrrolidin-2-yl)phenyl]-7*H*-pyrrolo[2,3-*d*]pyrimidine,
7-[4-(2,4-dimethoxypyrimidin-5-yl)-7*H*-pyrrolo[2,3-*d*]pyrimidin-6-yl]-3-methyl-1,2,3,4-tetrahydroisoquinoline,
1-{4-[4-(2,4-dimethoxypyrimidin-5-yl)-7*H*-pyrrolo[2,3-*d*]pyrimidin-6-yl]-3-fluorophenyl}-cyclopropan-1-amine,
3-{4-[4-(2,4-dimethoxypyrimidin-5-yl)-7*H*-pyrrolo[2,3-*d*]pyrimidin-6-yl]phenyl}tetrahydrofuran-3-amine,
3-{4-[4-(2,4-dimethoxypyrimidin-5-yl)-7*H*-pyrrolo[2,3-*d*]pyrimidin-6-yl]phenyl}oxetan-3-amine,
4-{4-[4-(2,4-dimethoxypyrimidin-5-y1)-7*H*-pyrrolo[2,3-*d*]pyrimidin-6-yl]phenyl}-*N*-methyltetrahydro-2*H-*pyran-4-amine,
*N*-{4-[4-(2,4-dimethoxypyrimidin-5-yl)-7*H*-pyrrolo[2,3-*d*]pyrimidin-6-yl]benzyl}cyclopropanamine,
1-{4-[4-(2,4-dimethoxypyrimidin-5-yl)-7*H*-pyrrolo[2,3-*d*]pyrimidin-6-yl]phenyl}cyclopropan-1-amine,
1-{4-[4-(2,4-dimethoxypyrimidin-5-yl)-7*H*-pyrrolo[2,3-*d*]pyrimidin-6-yl]phenyl}-*N*-methylmethanamine,
1-{4-[4-(2,4-dimethoxypyrimidin-5-yl)-7*H*-pyrrolo[2,3-*d*]pyrimidin-6-yl]phenyl}-*N*-methylpropan-1-amine,
2-amino-2-(4-(4-(2,4-dimethoxypyrimidin-5-yl)-7*H*-pyrrolo[2,3-d]pyrimidin-6-yl)phenyl)ethan-1-ol,
4-(2,4-dimethoxypyrimidin-5-yl)-6-[(2-methyl-4-pyrrolidin-2-yl)phenyl]-7*H*-pyrrolo[2,3-*d*]pyrimidine,
1-{4-[4-(2,4-dimethoxypyrimidin-5-yl)-7*H*-pyrrolo[2,3-d]pyrimidin-6-yl]-3-fluorophenyl}-N-methylmethanamine,
{5-[4-(2,4-dimethoxypyrimidin-5-yl)-7*H*-pyrrolo[2,3-d]pyrimidin-6-yl]-6-methylpyridin-2-yl}methanamine,
(*R*)-1-{4-[4-(2,4-dimethoxypyrimidin-5-yl)-7*H*-pyrrolo[2,3-*d*]pyrimidin-6-yl]phenyl}-*N*-ethylethan-1-amine,
(*S*)-1-{4-[4-(2,4-dimethoxypyrimidin-5-yl)-7*H*-pyrrolo[2,3-*d*]pyrimidin-6-yl]phenyl}-*N*-ethylethan-1-amine,
6-[4-(2,4-dimethoxypyrimidin-5-yl)-7*H*-pyrrolo[2,3-*d*]pyrimidin-6-yl]-1,2,3,4-tetrahydronaphthalen-1-amine,
1-{4-[4-(2,4-dimethoxypyrimidin-5-yl)-7*H*-pyrrolo[2,3-*d*]pyrimidin-6-yl]phenyl}cyclobutan-1-amine,
1-{4-[4-(2,4-dimethoxypyrimidin-5-yl)-7*H*-pyrrolo[2,3-*d*]pyrimidin-6-yl]phenyl}cyclopentan-1-amine,
2-{4-[4-(2,4-dimethoxypyrimidin-5-yl)-7*H*-pyrrolo[2,3-*d*]pyrimidin-6-yl]phenyl}propan-2-amine,
1-{4-[4-(2,4-dimethoxypyrimidin-5-yl)-7*H*-pyrrolo[2,3-*d*]pyrimidin-6-yl]phenyl}-*N*-ethylcyclopropan-1-amine,
1-{4-[4-(2,4-dimethoxypyrimidin-5-yl)-7*H*-pyrrolo[2,3-*d*]pyrimidin-6-yl]phenyl}-*N*-isopropylcyclopropan-1-amine,
1-{4-[4-(2,4-dimethoxypyrimidin-5-yl)-7*H*-pyrrolo[2,3-*d*]pyrimidin-6-yl]phenyl}-*N*-methylcyclopropan-1-amine,
*N*-buty1-1-{4-[4-(2,4-dimethoxypyrimidin-5-yl)-7*H*-pyrrolo[2,3-*d*]pyrimidin-6-yl]phenyl}cyclopropan-1-amine,
cyclopropyl{4-[4-(2,4-dimethoxypyrimidin-5-yl)-7*H*-pyrrolo[2,3-*d*]pyrimidin-6-yl]phenyl}methanamine,
cyclopentyl{4-[4-(2,4-dimethoxypyrimidin-5-yl)-7*H*-pyrrolo[2,3-*d*]pyrimidin-6-yl]phenyl}methanamine,
cyclohexyl{4-[4-(2,4-dimethoxypyrimidin-5-yl)-7*H*-pyrrolo[2,3-*d*]pyrimidin-6-yl]phenyl}methanamine,
2-cyclobutyl-1-{4-[4-(2,4-dimethoxypyrimidin-5-yl)-7*H*-pyrrolo[2,3-*d*]pyrimidin-6-yl]phenyl}ethan-1-amine,
1-{4-[4-(2,4-dimethoxypyrimidin-5-yl)-7*H*-pyrrolo[2,3-*d*]pyrimidin-6-yl]phenyl}-2,2,2-trifluorethan-1-amine,
4-{4-[4-(2,6-dimethoxypyridin-3-yl)-7*H*-pyrrolo[2,3-*d*]pyrimidin-6-yl]phenyl}tetrahydro-2*H*-pyran-4-amine,
4-{4-[4-(2-ethoxypyridin-3-yl)-7*H*-pyrrolo[2,3-*d*]pyrimidin-6-yl]phenyl}tetrahydro-2*H*-pyran-4-amine,
4-{4-[4-(4-(methoxy)pyrimidin-5-yl)-7*H*-pyrrolo[2,3-*d*]pyrimidin-6-yl]phenyl}tetrahydro-2*H*-pyran-4-amine,
4-{4-[4-(5-fluoro-2-methoxypyridin-3-yl)-7*H*-pyrrolo[2,3-*d*]pyrimidin-6-yl]phenyl}tetrahydro-2*H*-pyran-4-amine,
4-{4-[4-(6-fluoro-2-methoxypyridin-3-yl)-7*H*-pyrrolo[2,3-*d*]pyrimidin-6-yl]phenyl}tetrahydro-2*H*-pyran-4-amine,
4-{4-[4-(6-chloro-2-methoxypyridin-3-yl)-7*H*-pyrrolo[2,3-*d*]pyrimidin-6-yl]phenyl}tetrahydro-2*H*-pyran-4-amine,
4-{4-[4-(2-*iso*propoxypyridin-3-yl)-7*H*-pyrrolo[2,3-*d*]pyrimidin-6-yl[phenyl}tetrahydro-2*H*-pyran-4-amine,
4-{4-[4-(5-chloro-2-methoxypyridin-3-yl)-7*H*-pyrrolo[2,3-*d*]pyrimidin-6-yl]phenyl}tetrahydro-2*H*-pyran-4-amine,
4-{4-[4-(2,4-dimethoxypyridin-3-yl)-7*H*-pyrrolo[2,3-*d*]pyrimidin-6-yl]phenyl}tetrahydro-2*H*-pyran-4-amine,
1-{4-[4-(2,4-dimethoxypyridin-3-yl)-7*H*-pyrrolo[2,3-*d*]pyrimidin-6-yl]phenyl}cyclohexan-1-amine,
4-{4-[4-(2-methoxypyridin-3-yl)-7*H*-pyrrolo[2,3-*d*]pyrimidin-6-yl]phenyl}tetrahydro-2*H*-pyran-4-amine,
4-{4-[4-(2-methoxy-6-methylpyridin-3-yl)-7*H*-pyrrolo[2,3-*d*]pyrimidin-6-yl]phenyl}tetrahydro-2*H*-pyran-4-amine,
4-{4-[4-(2-cyclopropoxypyridin-3-yl)-7*H*-pyrrolo[2,3-*d*]pyrimidin-6-yl]phenyl}tetrahydro-2*H*-pyran-4-amine,
1-{4-[4-(4,6-dimethoxypyrimidin-5-yl)-7*H*-pyrrolo[2,3-*d*]pyrimidin-6-yl]phenyl}cyclohexan-1-amine,
1-{4-[4-(2-methoxypyridin-3-yl)-7*H*-pyrrolo[2,3-*d*]pyrimidin-6-yl]phenyl}cyclohexan-1-amine,
4-{4-[4-(3-methoxypyridin-4-yl)-7*H*-pyrrolo[2,3-*d*]pyrimidin-6-yl]phenyl}tetrahydro-2*H*-pyran-4-amine,
4-{4-[4-(5-fluoro-2-methoxypyridin-3-yl)-7*H*-pyrrolo[2,3-*d*]pyrimidin-6-yl]phenyl}tetrahydro-2*H*-pyran-4-amine,
1-{4-[4-(2-methoxy-6-methylpyridin-3-yl)-7*H*-pyrrolo[2,3-*d*]pyrimidin-6-yl]phenyl}cyclohexan-1-amine,
4-{4-[4-(4,6-dimethoxypyrimidin-5-yl)-7*H*-pyrrolo[2,3-*d*]pyrimidin-6-yl]phenyl}tetrahydro-2*H*-pyran-4-amine,
4-{4-[4-(4-methoxy-2-methylpyrimidin-5-yl)-7*H*-pyrrolo[2,3-*d*]pyrimidin-6-yl]phenyl}tetrahydro-2*H-*pyran-4-amine,
1-{4-[4-(4-methoxy-2-methylpyrimidin-5-yl)-7*H*-pyrrolo[2,3-*d*]pyrimidin-6-yl]phenyl}cyclohexan-1-amine,
4-{3-fluoro-4-[4-(4-methoxypyrimidin-5-yl)-7*H*-pyrrolo[2,3-*d*]pyrimidin-6-yl]phenyl}tetrahydro-2*H*-pyran-4-amine,
1-{3-fluoro-4-[4-(4-methoxypyrimidin-5-yl)-7*H*-pyrrolo[2,3-*d*]pyrimidin-6-yl]phenyl}cyclohexan-1-amine,
1-{4-[4-(2,6-dimethoxypyridin-3-yl)-7*H*-pyrrolo[2,3-*d*]pyrimidin-6-yl]phenyl}cyclohexan-1-amine,
1-{4-[4-(2-methoxy-6-trfluoromethylpyridin-3-yl)-7*H*-pyrrolo[2,3-*d*]pyrimidin-6-yl]phenyl}cyclohexan-1-amine,
1-{4-[4-(4-methoxypyrimidin-5-yl)-7*H*-pyrrolo[2,3-*d*]pyrimidin-6-yl]-phenyl}cyclohexan-1-amine,
2-{4-[4-(2,6-dimethoxypyridin-3-yl)-7*H*-pyrrolo[2,3-*d*]pyrimidin-6-yl]phenyl}propan-2-amine,
3-{4-[4-(2,6-dimethoxypyridin-3-yl)-7*H*-pyrrolo[2,3-*d*]pyrimidin-6-yl]phenyl}pentan-3-amine,
4-{3-fluoro-4-[4-(2-methoxypyridin-3-yl)-7*H*-pyrrolo[2,3-*d*]pyrimidin-6-yl]phenyl}tetrahydro-2*H*-pyran-4-amine,
4-{5-[4-(4-methoxypyrimidin-5-yl)-7*H*-pyrrolo[2,3-*d*]pyrimidin-6-yl]pyridin-2-yl}tetrahydro-2*H*-pyran-4-amine, and
4-{2-fluoro-4-[4-(4-methoxypyrimidin-5-yl)-7*H*-pyrrolo[2,3-*d*]pyrimidin-6-yl]phenyl}tetrahydro-2*H*-pyran-4-amine.

A further aspect of the present invention are the 4,6-disubstituted-7*H*-pyrrolo[2,3-*d*]pyrimidines of general formula I for use as medicaments.

Yet further aspect of the present invention are the 4,6-disubstituted-7*H*-pyrrolo[2,3-*d*]pyrimidines of general formula I for use in inhibition of pathological cell proliferation of tumor/non-tumor/cancer origin and for treatment of tumor/non-tumor/cancer disease associated with cell hyperproliferation related to overexpression of antiapoptotic factors MCL-1 and cMYC, such as cancer (both leukemia and solid tumors). Examples include Acute Myelocytic Leukemia (AML), Relapsed/Refractory Chronic Lymphocytic Leukemia (CLL), Chronic Myelomonocytic Leukemia (CMML), sarcomas, solid tumors like breast (Human Epidermal Growth Factor Receptor 2 Negative Breast Cancer, Triple-negative breast cancer), lung, ovarian, melanoma and pancreatic cancers including Castration-Resistant Prostate Cancer, lymphomas (e.g. Hodgkin lymphoma; diffuse large B-cell lymphoma; high-grade B-cell lymphoma, cutaneous T-cell lymphoma; Extranodal Marginal Zone B-Cell Lymphoma (Mucosa-Associated Lymphoid Tissue or MALT-Lymphoma), Peripheral T-Cell Lymphomas (PTCL)).

The present invention provides 4,6-disubstituted-7*H*-pyrrolo[2,3-*d*]pyrimidines of general formulas I for use in reduction of neutrophils counts and for treatment of neutrophilic diseases. Examples of (inflammatory) neutrophilic diseases include rheumatoid arthritis, systemic lupus erythematosus or antiphospholipid syndrome, cystic fibrosis, severe asthma, Sjogren's, neutrophilic dermatoses, diabetic nephropathy, regeneration after myocardial infarction or in treatment of acute liver failure (ALF).

Compounds of general formula **I** can be formulated into pharmaceutical preparations with pharmaceutically acceptable excipients. The preparations may be in liquid form or solid form, or in other forms such as aerosol. Liquid forms include solutions, suspensions, dispersions, emulsions formulated, for example, for injection or oral administration, gels, ointments. Solid forms include, for example, capsules, tablets, coated tablets, powders, suppositories, and other forms, known in the art. Another object of the present invention is thus a pharmaceutical composition comprising a therapeutically effective amount of a compound of general formula I and one or more pharmaceutically acceptable excipient. Pharmaceutically acceptable excipients include solvents, solubility controlling agents, pH controlling agents, carriers, fillers, binders, glidants, disintegrants, preservatives, sorbents, viscosity controlling agents, agents affecting sensory properties such as taste, odour or colour of the product.

The term "therapeutically effective amount" of a compound of the present invention refers to an amount of the compound or drug that is effective in treating a disease or disorder in a human or mammal. In the case of cancer treatment the "effective amount" refers to the amount that inhibits or reduces proliferation of cancer cells, reduces the primary tumor/cancer size, inhibits (that is, to a certain extent slow down and preferably stop) cancer cell infiltration into peripheral organs, inhibits (that is, to a certain extent slow down and preferably stop) the formation of tumor metastases, inhibits, to a certain extent, tumor growth and/or relieves at least to some extent one or more symptoms associated with tumor or cancer. The drug can prevent growth and/or kill existing cancer cells, thus it can be cytostatic and/or cytotoxic. In the case of neutrophilic diseases, the "effective amount" refers to the amount that reduces number of neutrophils and inflammation.

The term "pharmaceutical composition" refers to the formulation of a compound and medium, generally accepted in the art, for the delivery of a biologically active compound to a mammal, e.g., a human. Such a medium includes all pharmaceutically acceptable carriers, diluents or excipients.

The term "pharmaceutically acceptable carrier, diluent or filler" as used herein includes, without limitation, any excipient, carrier, glidant, sweetener, preservative, dye, flavor enhancer, surfactant, dispersing agent, suspending agent, isotonic agent, solvent, or emulsifier that has been approved for use in humans or domestic animals.

The invention further relates to compounds of formula I for use as an active ingredient in a pharmaceutical composition which may be prepared by conventional methods known in the art, e.g., the active ingredient may be in a mixture with pharmaceutically acceptable inert organic and/or inorganic carriers and/or with auxiliaries or, where appropriate, attached to them.

The invention also relates to compounds of the formula I for use as second or other active substances having synergistic effect with other active substances in known drugs, or the administration of the compounds of the formula I together with these drugs. Examples of such drugs are BCL-2 inhibitors, such as venetoclax.

The invention also provides the pharmaceutical composition disclosed herein above for use in inhibition of pathological cell proliferation of tumor/non-tumor/cancer origin and/or for treatment of tumor/non tumor/cancer disease associated with cell hyperproliferation. Said pharmaceutical composition may be used for the treatment of diseases listed above.

The suitability of the compounds for oral use is evidenced in vitro by good Caco-2 permeability coefficients (Papp) and in vivo by compound bioavaliability (F). Oral administration is desirable for the patient as well as for the healthcare system as it reduces need for hospitalization, costs and general risks linked to parenteral application (infection, extravasation injuries). Oral administration generally increases patient compliance.

### Brief Description of Drawings

Fig. 1: Preliminary in vivo efficacy study of compound 9 in mouse subcutaneous MV-4-11 xenograft model in female NOD/SCID mice.
Fig. 2: Neutrophil apoptosis induction by the compounds of the invention vs. KB0742.

### Detailed Description of the Invention

### Standard abbreviations and acronyms:

- Ac: acetyl
- Bpin: pinacolato boron
- Boc: *tert*-butyloxycarbonyl
- COD: 1,5-cyclooctadiene
- d: doublet
- dd: doublet of doublets
- ddd: doublet of doublets of doublets
- DCM: dichloromethane
- DMF: *N,N*-dimethylformamide
- DMSO: dimethylsulfoxide
- dt: doublet of triplets
- ESI: electrospray ionization
- EtOH: ethanol
- HPFC: high-performance flash chromatography
- HPLC: high-performance liquid chromatography
- HR: high resolution
- m: multiplet
- Me: methyl
- MeCN: acetonitrile
- MS: mass spectrometry
- NaOMe: sodium methoxide
- NMR: nuclear magnetic resonance
- Pd(OAc)₂: palladium(II) acetate
- Pd(dppf)Cl₂: [1,1'-bis(diphenylphosphino)ferrocene]dichloropalladium(II)
- Ph: phenyl
- PPh₃: triphenylphosphine
- q: quartet
- s: singlet
- SEM: 2-(trimethylsilyl)ethoxymethyl chloride
- SiO₂: silica gel
- t: triplet
- td: triplet doublet
- TFA: trifluoroacetic acid
- THF: tetrahydrofuran
- TLC: thin layer chromatography
- TPPTS: triphenylphosphine-3,3',3"-trisulfonic acid trisodium salt

### Compounds numbering

Following numbering of compounds is used:

| **Compound** | **Structure** | **Systematic name** |
|---|---|---|
| **1** | 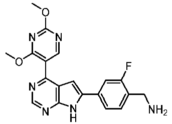 | {4-[4-(2,4-dimethoxypyrimidin-5-yl)-7*H*-pyrrolo[2,3-*d*]pyrimidin-6-yl]-2-fluorophenyl}methanamine |
| **2** | 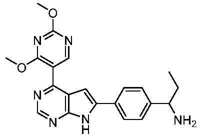 | 1-{4-[4-(2,4-dimethoxypyrimidin-5-yl)-7*H*-pyrrolo[2,3-*d*]pyrimidin-6-yl]phenyl}propan-1-amine |
| **3** | 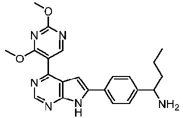 | 1-{4-[4-(2,4-dimethoxypyrimidin-5-yl)-7*H*-pyrrolo[2,3-*d*]pyrimidin-6-yl]phenyl}butan-1-amine |
| **4** | 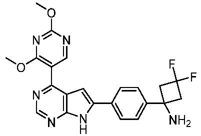 | 1-{4-[4-(2,6-dimethoxypyrimidin-5-yl)-7*H*-pyrrolo[2,3-*d*]pyrimidin-6-yl]phenyl}-3,3-difluorocyclobutan-1-amine |
| **5** | 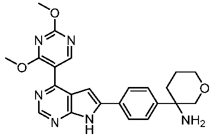 | 3 -{4-[4-(2,4-dimethoxypyrimidin-5-yl)-7*H*-pyrrolo[2,3-*d*]pyrimidin-6-yl]phenyl}tetrahydro-2H-pyran-3-amine |
| **6** | 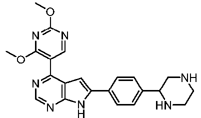 | 4-(2,4-dimethoxypyrimidin-5-yl)-6-[4-(piperazin-2-yl)phenyl]-7*H*-pyrrolo[2,3-*d*]pyrimidine |
| **7** | 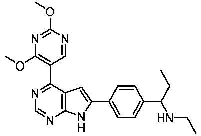 | 1-{4-[4-(2,4-dimethoxypyrimidin-5-yl)-7*H*-pyrrolo[2,3-*d*]pyrimidin-6-yl]phenyl}-*N*-ethylpropan-1-amine |
| **8** | 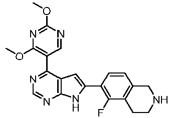 | 6-[4-(2,4-dimethoxypyrimidin-5-yl)-7*H*-pyrrolo[2,3-*d*]pyrimidin-6-yl]-5-fluoro-1,2,3,4-tetrahydroisoquinoline |
| **9** | 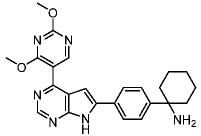 | 1-{4-[4-(2,4-dimethoxypyrimidin-5-yl)-7*H*-pyrrolo[2,3-*d*]pyrimidin-6-yl]phenyl}cyclohexan-1-amine |
| **10** | 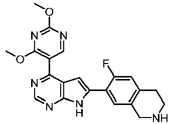 | 7-[4-(2,4-dimethoxypyrimidin-5-yl)-7*H*-pyrrolo[2,3-*d*]pyrimidin-6-yl]-6-fluoro-1,2,3, 4-tetrahydroisoquinoline |
| **11** | 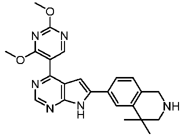 | 6-[4-(2,4-dimethoxypyrimidin-5-yl)-7*H*-pyrrolo[2,3-*d*]pyrimidin-6-yl]-4,4-dimethyl-1,2,3,4-tetrahydroisoquinoline |
| **12** | 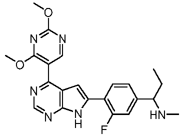 | 1-{4-[4-(2,4-dimethoxypyrimidin-5-yl)-7*H*-pyrrolo[2,3-*d*]pyrimidin-6-yl]-3-fluorophenyl}-*N*-methylpropan-1-amine |
| **13** | 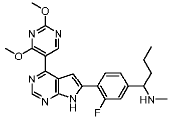 | 1-{4-[4-(2,4-dimethoxypyrimidin-5-yl)-7*H*-pyrrolo[2,3-*d*]pyrimidin-6-yl]-3-fluorophenyl}-*N*-methylbutan-1-amine |
| **14** | 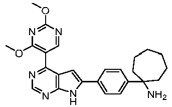 | 1-{4-[4-(2,4-dimethoxypyrimidin-5-yl)-7*H*-pyrrolo[2,3-*d*]pyrimidin-6-yl]phenyl}cycloheptan-1-amine |
| **15** | 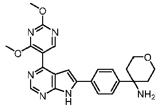 | 4-{4-[4-(2,4-dimethoxypyrimidin-5-yl)-7*H*-pyrrolo[2,3-*d*]pyrimidin-6-yl]phenyl}tetrahydro-2*H*-pyran-4-amine |
| **16** | 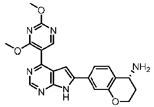 | (*R*)-7-[4-(2,4-dimethoxypyrimidin-5-yl)-7*H*-pyrrolo[2,3-*d*]pyrimidin-6-yl]chroman-4-amine |
| **17** | 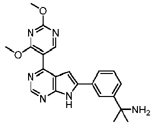 | 2-{3-[4-(2,4-dimethoxypyrimidin-5-yl)-7*H*-pyrrolo[2,3-*d*]pyrimidin-6-yl]phenyl}propan-2-amine |
| **18** | 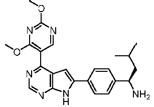 | (*R*)-1-{4-[4-(2,4-dimethoxypyrimidin-5-yl)-7*H*-pyrrolo[2,3-*d*]pyrimidin-6-yl]phenyl}-3-methylbutan-1-amine |
| **19** | 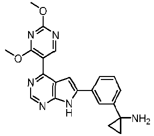 | 1-{3-[4-(2,4-dimethoxypyrimidin-5-yl)-7*H*-pyrrolo[2,3-*d*]pyrimidin-6-yl]phenyl}cyclopropan-1-amine |
| **20** | 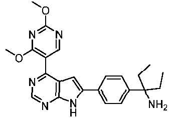 | 3-{4-[4-(2,4-dimethoxypyrimidin-5-yl)-7*H*-pyrrolo[2,3-*d*]pyrimidin-6-yl]phenyl}pentan-3-amine |
| **21** | 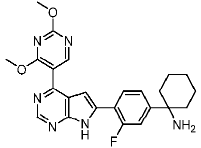 | 1-{4-[4-(2,4-dimethoxypyrimidin-5-yl)-7*H*-pyrrolo[2,3-*d*]pyrimidin-6-yl]-3-fluorophenyl}cyclohexan-1-amine |
| **22** | 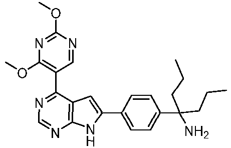 | 4-{4-[4-(2,4-dimethoxypyrimidin-5-yl)-7*H*-pyrrolo[2,3-*d*]pyrimidin-6-yl]phenyl}heptan-4-amine |
| **23** | 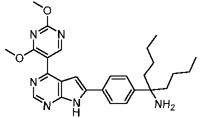 | 5-{4-[4-(2,4-dimethoxypyrimidin-5-yl)-7*H*-pyrrolo[2,3-*d*]pyrimidin-6-yl]phenyl}nonan-5-amine |
| **24** | 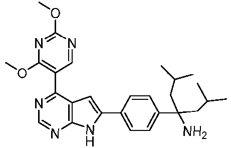 | 4-{4-[4-(2,4-dimethoxypyrimidin-5-yl)-7*H*-pyrrolo[2,3-*d*]pyrimidin-6-yl]phenyl}-2,6-dimethylheptan-4-amine |
| **25** | 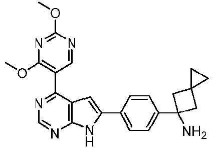 | 5-{4-[4-(2,4-dimethoxypyrimidin-5-yl)-7*H*-pyrrolo[2,3-*d*]pyrimidin-6-yl]phenyl}spiro[2.3]hexan-5-amine |
| **26** | 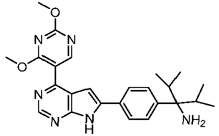 | 3-{4-[4-(2,4-dimethoxypyrimidin-5-yl)-7*H*-pyrrolo[2,3-*d*]pyrimidin-6-yl]phenyl}-2,4-dimethylpentan-3-amine |
| **27** | 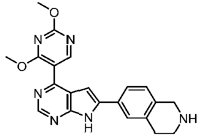 | 6-[4-(2,4-dimethoxypyrimidin-5-yl)-7*H*-pyrrolo[2,3-*d*]pyrimidin-6-yl]-1,2,3,4-tetrahydroisoquinoline |
| **28** | 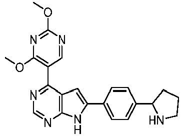 | 4-(2,4-dimethoxypyrimidin-5-yl)-6-[(4-pyrrolidin-2-yl)phenyl]-7*H*-pyrrolo[2,3-*d*]pyrimidine |
| **29** | 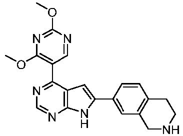 | 7-[4-(2,4-dimethoxypyrimidin-5-yl)-7*H*-pyrrolo[2,3-*d*]pyrimidin-6-yl]-1,2,3,4-tetrahydroisoquinoline |
| **30** | 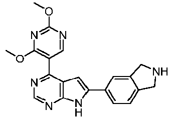 | 4-(2,4-dimethoxypyrimidin-5-yl)-6-(isoindolin-5-yl)-7*H-*pyrrolo[2,3-*d*]pyrimidine |
| **31** | 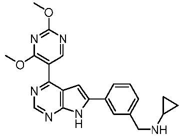 | *N*-{3-[4-(2,4-dimethoxypyrimidin-5-yl)-7*H*-pyrrolo[2,3-*d*]pyrimidin-6-yl]benzyl}cyclopropanamine |
| **32** | 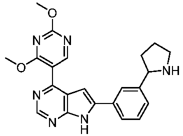 | 4-(2,4-dimethoxypyrimidin-5-yl)-6-[(3-pyrrolidin-2-yl)phenyl]-7*H*-pyrrolo[2,3-*d*]pyrimidine |
| **33** | 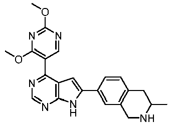 | 7-[4-(2,4-dimethoxypyrimidin-5-yl)-7*H*-pyrrolo[2,3-*d*]pyrimidin-6-yl]-3-methyl-1,2,3,4-tetrahydroisoquinoline |
| **34** | 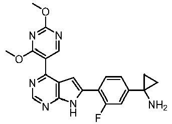 | 1-{4-[4-(2,4-dimethoxypyrimidin-5-yl)-7*H*-pyrrolo[2,3-*d*]pyrimidin-6-yl]-3-fluorophenyl}-cyclopropan-1-amine |
| **35** | 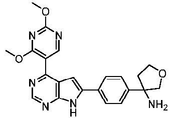 | 3-{4-[4-(2,4-dimethoxypyrimidin-5-yl)-7*H*-pyrrolo[2,3-*d*]pyrimidin-6-yl]phenyl}tetrahydrofuran-3-amine |
| **36** | 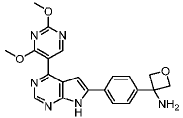 | 3-{4-[4-(2,4-dimethoxypyrimidin-5-yl)-7*H*-pyrrolo[2,3-*d*]pyrimidin-6-yl]phenyl}oxetan-3-amine |
| **37** | 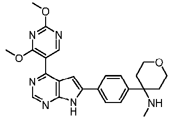 | 4-{4-[4-(2,4-dimethoxypyrimidin-5-yl)-7*H*-pyrrolo[2,3-*d*]pyrimidin-6-yl]phenyl}-*N*-methyltetrahydro-2*H*-pyran-4-amine |
| **38** | 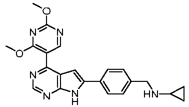 | *N*-{4-[4-(2,4-dimethoxypyrimidin-5-yl)-7*H*-pyrrolo[2,3-*d*]pyrimidin-6-yl]benzyl}cyclopropanamine |
| **39** | 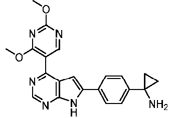 | 1-{4-[4-(2,4-dimethoxypyrimidin-5-yl)-7*H*-pyrrolo[2,3-*d*]pyrimidin-6-yl]phenyl}cyclopropan-1-amine |
| **40** | 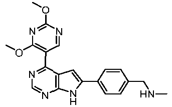 | 1-{4-[4-(2,4-dimethoxypyrimidin-5-yl)-7*H*-pyrrolo[2,3-*d*]pyrimidin-6-yl]phenyl}-*N*-methylmethanamine |
| **41** | 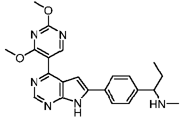 | 1-{4-[4-(2,4-dimethoxypyrimidin-5-yl)-7*H*-pyrrolo[2,3-*d*]pyrimidin-6-yl]phenyl}-*N*-methylpropan-1-amine |
| **42** | 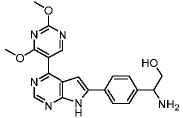 | 2-amino-2-(4-(4-(2,4-dimethoxypyrimidin-5-yl)-7*H-*pyrrolo[2,3-*d*]pyrimidin-6-yl)phenyl)ethan-1-ol |
| **43** | 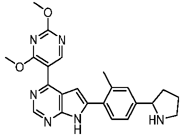 | 4-(2,4-dimethoxypyrimidin-5-yl)-6-[(2-methyl-4-pyrrolidin-2-yl)phenyl]-7*H*-pyrrolo[2,3-d]pyrimidine |
| **44** | 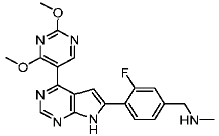 | 1-{4-[4-(2,4-dimethoxypyrimidin-5-yl)-7*H*-pyrrolo[2,3-*d*]pyrimidin-6-yl]-3-fluorophenyl}-*N*-methylmethanamine |
| **45** | 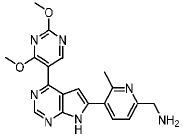 | {5-[4-(2,4-dimethoxypyrimidin-5-yl)-7*H*-pyrrolo[2,3-*d*]pyrimidin-6-yl]-6-methylpyridin-2-yl}methanamine |
| **46** | 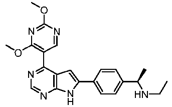 | (*R*)-1-{4-[4-(2,4-dimethoxypyrimidin-5-yl)-7*H*-pyrrolo[2,3-*d*]pyrimidin-6-yl]phenyl}-*N*-ethylethan-1-amine |
| **47** | 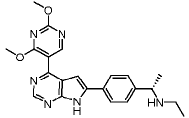 | (*S*)-1-{4-[4-(2,4-dimethoxypyrimidin-5-yl)-7*H*-pyrrolo[2,3-*d*]pyrimidin-6-yl]phenyl}-*N*-ethylethan-1-amine |
| **48** | 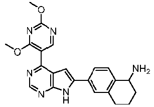 | {6-[4-(2,4-dimethoxypyrimidin-5-yl)-7*H*-pyrrolo[2,3-*d*]pyrimidin-6-yl]}-1,2,3,4-tetrahydronaphthalen-1-amine |
| **49** | 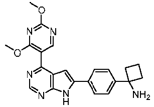 | 1-{4-[4-(2,4-dimethoxypyrimidin-5-yl)-7*H*-pyrrolo[2,3-*d*]pyrimidin-6-yl]phenyl}cyclobutan-1-amine |
| **50** | 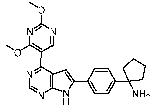 | 1-{4-[4-(2,4-dimethoxypyrimidin-5-yl)-7*H*-pyrrolo[2,3-*d*]pyrimidin-6-yl]phenyl}cyclopentan-1-amine |
| **51** | 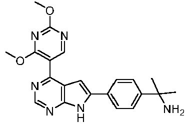 | 2-{4-[4-(2,4-dimethoxypyrimidin-5-yl)-7*H*-pyrrolo[2,3-*d*]pyrimidin-6-yl]phenyl}propan-2-amine |
| **52** | 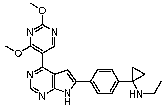 | 1-{4-[4-(2,4-dimethoxypyrimidin-5-yl)-7*H*-pyrrolo[2,3-*d*]pyrimidin-6-yl]phenyl}-*N*-ethylcyclopropan-1-amine |
| **53** | 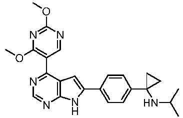 | 1-{4-[4-(2,4-dimethoxypyrimidin-5-yl)-7*H*-pyrrolo[2,3-*d*lpyrimidin-6-ylphenyl}-*N*-*iso*propylcyclopropan-1-amine |
| **54** | 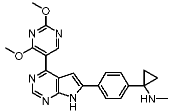 | 1-{4-[4-(2,4-dimethoxypyrimidin-5-yl)-7*H*-pyrrolo[2,3-*d*]pynmidin-6-yl]phenyl}-*N*-methylcyclopropan-1-amine |
| **55** | 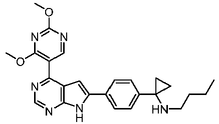 | *N*-butyl-1-{4-[4-(2,4-dimethoxypyrimidin-5-yl)-7*H-*pyrrolo[2,3-*d*]pyrimidin-6-yl]phenyl}cyclopropan-1-amine |
| **56** | 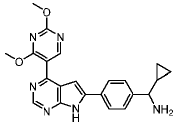 | cyclopropyl{4-[4-(2,4-dimethoxypyrimidin-5-yl)-7*H-*pyrrolo[2,3-*d*]pyrimidin-6-yl]phenyl}methanamine |
| **57** | 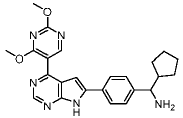 | cyclopentyl{4-[4-(2,4-dimethoxypyrimidin-5-yl)-7*H-*pyrrolo[2,3-*d*]pyrimidin-6-yl]phenyl}methanamine |
| **58** | 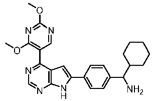 | cyclohexyl{4-[4-(2,4-dimethoxypyrimidin-5-yl)-7*H-*pyrrolo[2,3-*d*]pyrimidin-6-yl]phenyl}methanamine |
| **59** | 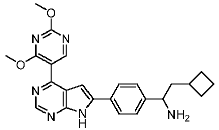 | 2-cyclobutyl-1-{4-[4-(2,4-dimethoxypyrimidin-5-yl)-7*H-*pyrrolo[2,3-*d*]pyrimidin-6-yl]phenyl}ethan-1-amine |
| **60** | 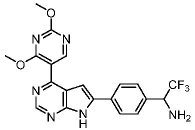 | 1-{4-[4-(2,4-dimethoxypyrimidin-5-yl)-7*H*-pyrrolo[2,3-*d*]pyrimidin-6-yl]phenyl}-2,2,2-trifluorethan-1-amine |
| **61** | 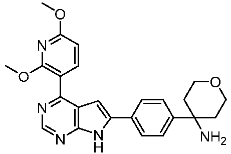 | 4-{4-[4-(2,6-dimethoxypyridin-3-yl)-7*H*-pyrrolo[2,3-*d*]pyrimidin-6-yl]phenyl}tetrahydro-2*H*-pyran-4-amine |
| **62** | 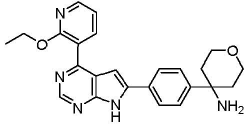 | 4-{4-[4-(2-ethoxypyridin-3-yl)-7*H*-pyrrolo[2,3-*d*]pyrimidin-6-yl]phenyl}tetrahydro-2*H*-pyran-4-amine |
| **63** | 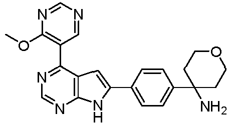 | 4-{4-[4-(4-(methoxy)pyrimidin-5-yl)-7*H*-pyrrolo[2,3-*d*]pyrimidin-6-yl]phenyl}tetrahydro-2*H*-pyran-4-amine |
| **64** | 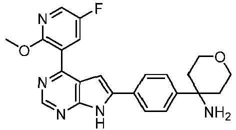 | 4-{4-[4-(5-fluoro-2-methoxypyridin-3-yl)-7*H*-pyrrolo[2,3-*d*]pyrimidin-6-yl]phenyl}tetrahydro-2*H*-pyran-4-amine |
| **65** | 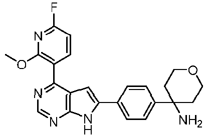 | 4-{4-[4-(6-fluoro-2-methoxypyridin-3-yl)-7*H*-pyrrolo[2,3-*d*]pyrimidin-6-yl]phenyl}tetrahydro-2*H*-pyran-4-amine |
| **66** | 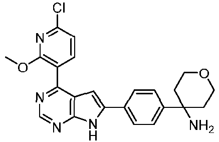 | 4-{4-[4-(6-chloro-2-methoxypyridin-3-yl)-7*H*-pyrrolo[2,3-*d*]pyrimidin-6-yl]phenyl}tetrahydro-2*H*-pyran-4-amine |
| **67** | 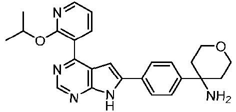 | 4-{4-[4-(2-isopropoxypyridin-3-yl)-7*H*-pyrrolo[2,3-*d*]pyrimidin-6-yl]phenyl}tetrahydro-2*H*-pyran-4-amine |
| **68** | 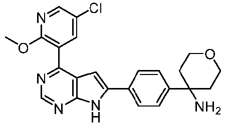 | 4-{4-[4-(5-chloro-2-methoxypyridin-3-yl)-7*H*-pyrrolo[2,3-*d*]pyrimidin-6-yl]phenyl}tetrahydro-2*H*-pyran-4-amine |
| **69** | 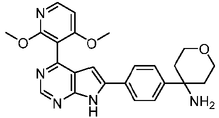 | 4-{4-[4-(2,4-dimethoxypyridin-3-yl)-7*H*-pyrrolo[2,3-*d*]pyrimidin-6-yl]phenyl}tetrahydro-2*H*-pyran-4-amine |
| **70** | 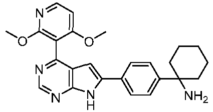 | 1-{4-[4-(2,4-dimethoxypyridin-3-yl)-7*H*-pyrrolo[2,3-*d*]pyrimidin-6-yl]phenyl}cyclohexan-1-amine |
| **71** | 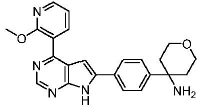 | 4-{4-[4-(2-methoxypyridin-3-yl)-7*H*-pyrrolo[2,3-*d*]pyrimidin-6-yl]phenyl}tetrahydro-2*H*-pyran-4-amine |
| **72** | 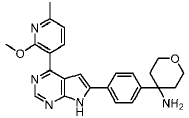 | 4-{4-[4-(2-methoxy-6-methylpyridin-3-yl)-7*H*-pyrrolo[2,3-*d*]pyrimidin-6-yl]phenyl}tetrahydro-2*H*-pyran-4-amine |
| **73** | 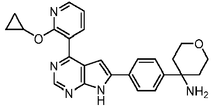 | 4-{4-[4-(2-cyclopropoxypyridin-3-yl)-7*H*-pyrrolo[2,3-*d*]pyrimidin-6-yl]phenyl}tetrahydro-2*H*-pyran-4-amine |
| **74** | 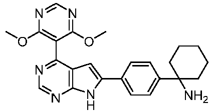 | 1-{4-[4-(4,6-dimethoxypyrimidin-5-yl)-7*H*-pyrrolo[2,3-*d*]pyrimidin-6-yl]phenyl}cyclohexan-1-amine |
| **75** | 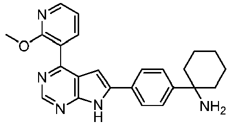 | 1-{4-[4-(2-methoxypyridin-3-yl)-7*H-*pyrrolo[2,3-*d*]pyrimidin-6-yl]phenyl}cyclohexan-1-amine |
| **76** | 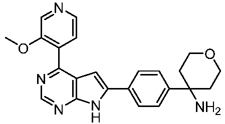 | 4-{4-[4-(3-methoxypyridin-4-yl)-7*H*-pyrrolo[2,3-*d*]pyrimidin-6-yl]phenyl}tetrahydro-2*H*-pyran-4-amine |
| **77** | 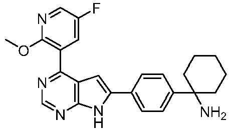 | 1-{4-[4-(5-fluoro-2-methoxypyridin-3-yl)-7*H*-pyrrolo[2,3-*d*]pyrimidin-6-yl]phenyl}cyclohexan-1-amine |
| **78** | 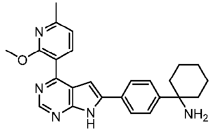 | 1-{4-[4-(2-methoxy-6-methylpyridin-3-yl)-7*H*-pyrrolo[2,3-*d*]pyrimidin-6-yl]phenyl}cyclohexan-1-amine |
| **79** | 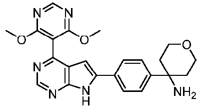 | 4-{4-[4-(4,6-dimethoxypyrimidin-5-yl)-7*H*-pyrrolo[2,3-*d*]pyrimidin-6-yl]phenyl}tetrahydro-2*H*-pyran-4-amine |
| **80** | 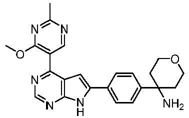 | 4-{4-[4-(4-methoxy-2-methylpyrimidin-5-yl)-7*H-*pyrrolo[2,3-*d*]pyrimidin-6-yl]phenyl}tetrahydro-2*H*-pyran-4-amine |
| **81** | 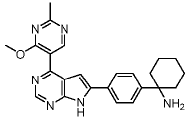 | 1-{4-[4-(4-methoxy-2-methylpyrimidin-5-yl)-7*H-*pyrrolo[2,3-*d*]pyrimidin-6-yl]phenyl}cyclohexan-1-amine |
| **82** | 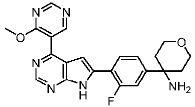 | 4-{3-fluoro-4-[4-(4-methoxypyrimidin-5-yl)-7*H-*pyrrolo[2,3-*d*]pyrimidin-6-yl]phenyl}tetrahydro-2*H*-pyran-4-amine |
| **83** | 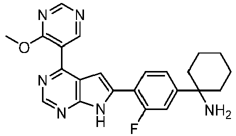 | 1-{3-fluoro-4-[4-(4-methoxypyrimidin-5-yl)-7*H-*pyrrolo[2,3-*d*]pyrimidin-6-yl]phenyl}cyclohexan-1-amine |
| **84** | 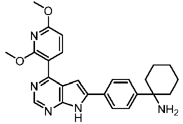 | 1-{4-[4-(2,6-dimethoxypyridin-3-yl)-7*H*-pyrrolo[2,3-*d*]pyrimidin-6-yl]phenyl}cyclohexan-1-amine |
| **85** | 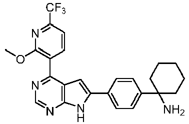 | 1-{4-[4-(2-methoxy-6-trifluoromethylpyridin-3-yl)-7*H-*pyrrolo[2,3-*d*]pyrimidin-6-yl]phenyl}cyclohexan-1-amine |
| **86** | 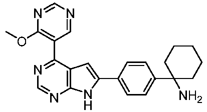 | 1-{4-[4-(4-methoxypyrimidin-5-yl)-7*H*-pyrrolo[2,3-*d*]pyrimidin-6-yl]-phenyl}cyclohexan-1-amine |
| **87** | 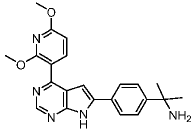 | 2-{4-[4-(2,6-dimethoxypyridin-3-yl)-7*H*-pyrrolo[2,3-*d*]pyrimidin-6-yl]phenyl}-propan-2-amine |
| **88** | 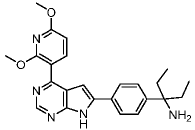 | 3-{4-[4-(2,6-dimethoxypyridin-3-yl)-7*H*-pyrrolo[2,3-*d*]pyrimidin-6-yl]phenyl}pentan-3-amine |
| **89** | 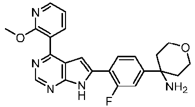 | 4-{3-fluoro-4-[4-(2-methoxypyridin-3-yl)-7*H*-pyrrolo[2,3-*d*]pyrimidin-6-yl]phenyl}tetrahydro-2H-pyran-4-amine |
| **90** | 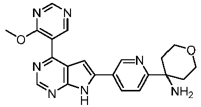 | 4-{5-[4-(4-methoxypyrimidin-5-yl)-7*H*-pyrrolo[2,3-*d*]pyrimidin-6-yl]pyridin-2-yl}tetrahydro-2H-pyran-4-amine |
| **91** | 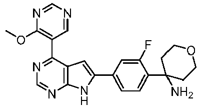 | 4-{2-fluoro-4-[4-(4-methoxypyrimidin-5-yl)-7*H-*pyrrolo[2,3-*d*]pyrimidin-6-yl]phenyl}tetrahydro-2*H*-pyran-4-amine |

### Synthesis of compounds

All the title compounds were prepared by one of the following synthetic approaches starting from simple commercially available materials. The first approach starts from 6-chloro-8-iodo-7-deazapurine **(I),** and it is based on two consecutive Suzuki couplings. The first reaction is performed in dioxane, catalyzed by Pd(dppf)Cl₂ and employs K₃PO₄ as a base and introduces the first aryl group into position 8 with high chemoselectivity. The second Suzuki coupling is then performed under aqueous conditions employing water-soluble TPPTS ligand in combination with Pd(OAc)₂ in a mixture of acetonitrile and water (Scheme 1).

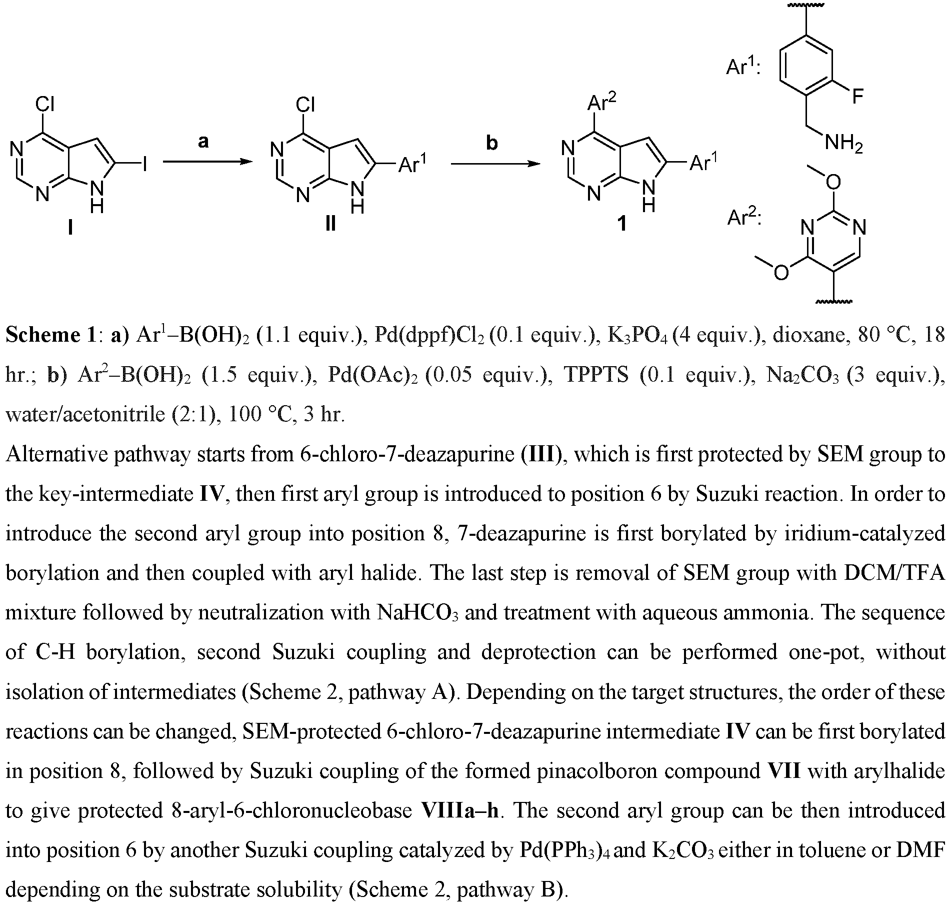

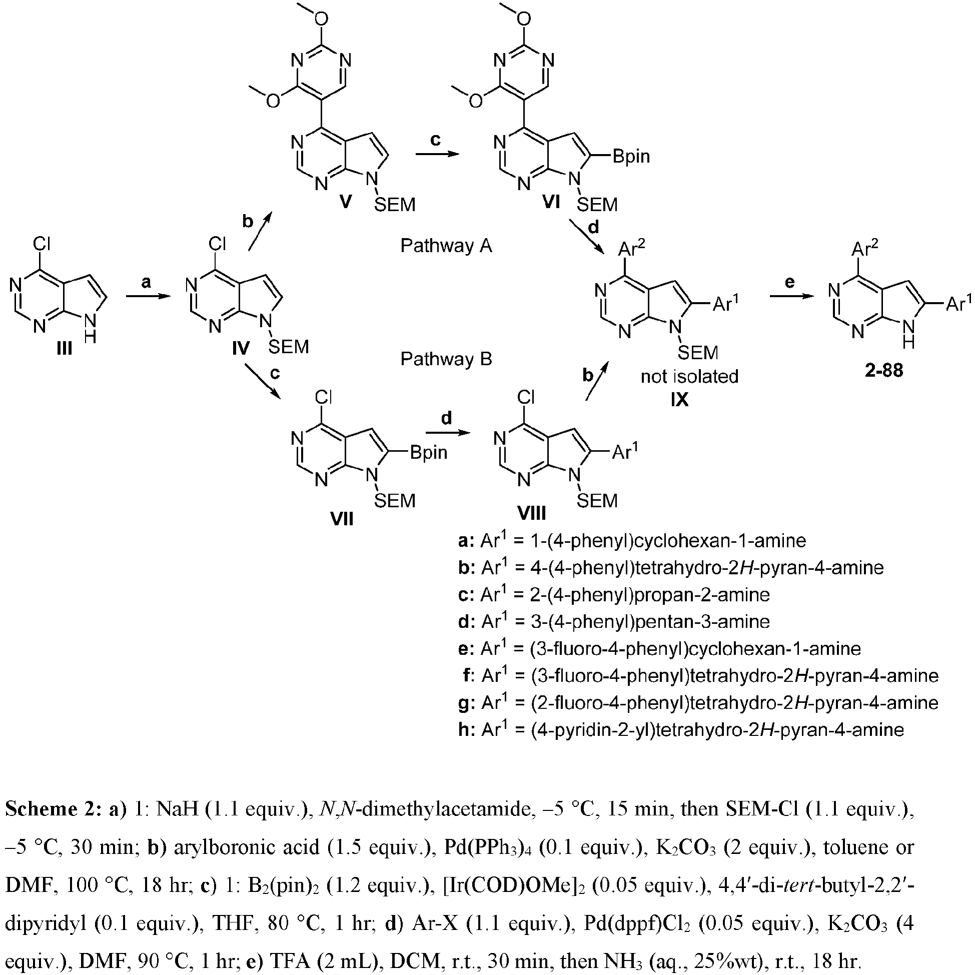

### Examples

### General Experimental Part

Reactions were monitored by thin layer chromatography (TLC) on TLC Silica gel 60F254 (Merck) and detected by UV (254 nm) and by TLC-MS analysis using an Advion Expression Compact Mass Spectrometer with electrospray ionization (ESI). NMR spectra were measured on a Bruker Avance 500 MHz spectrometer (500.0 MHz for ¹H, 125.7 MHz for ¹³C) or a Bruker Avance 600 MHz spectrometer (600.1 MHz for ¹H and 150.9 MHz for ¹³C) in DMSO-*d6*, CDC13 (referenced to the residual solvent signal). Chemical shifts are given in ppm (δ-scale) and coupling constants (J) in Hz. Complete assignment of all NMR signals was performed using a combination of H,H-COSY, H,H-ROESY, H,C-HSQC, and H,C-HMBC experiments. Low resolution mass spectra were measured on LCQ Fleet (Thermo Fisher Scientific) using electrospray ionization (ESI). High resolution mass spectra were measured on LTQ Orbitrap XL (Thermo Fisher Scientific). High performance flash chromatography (HPFC) was performed with an ISCO Combiflash Rf system on RediSep Rf Gold Silica Gel Disposable columns. The purity of all final compounds (>95%) was determined by analytical HPLC and by clean NMR spectra.

### Suzuki coupling in position 8 of 6-chloro-8-iodo-7-deazapurine (I)

### General procedure A:

8-Iodo-6-chloro-7-deazapurine **(I)** (769 mg, 2.75 mmol, 1.0 equiv.) was combined with aryl boronic acid (3.025 mmol, 1.1 equiv.), Pd(dppf)Cl₂ (224 mg, 0.31 mmol) and K₃PO₄ (2.34 g, 11.0 mmol, 4 equiv.) in dioxane (20 mL) and stirred under argon atmosphere at 80 °C for 18 hours. The reaction was then cooled to ambient temperature a filtered through a celite. The filtrate was then concentrated and the crude product was purified by HPFC (SiO₂, DCM/MeOH, 0 → 10%).

### Suzuki coupling in position 6 of 6-chloro-8-aryl-7-deazapurine

### General procedure B:

6-Chloro-8-aryl-7-deazapurine **(II)** (0.5 mmol), Pd(OAc)₂ (0.025 mmol, 0.05 equiv.), arylboronic acid (0.75 mmol, 1.5 equiv.), TPPTS (0.060 mmol, 0.12 equiv.) and Na₂CO₃ (1.5 mmol, 3 equiv.) were dissolved in water-acetonitrile mixture (1:1, 3 mL) and the mixture was heated to 100 °C for 1-16 h. The solvent was evaporated and the crude product was purified by HPFC (SiO₂, DCM/MeOH, 0 - 10%).

### Suzuki coupling in position 6 of 6-chloro-9-SEM-7-deazapurine or 6-chloro-9-SEM-8-aryl-7-deazapurine

### General procedure C:

6-Chloro-9-SEM-7-deazapurine **(IV)** (142 mg, 0.5 mmol), Pd(PPh₃)₄ (57.8 mg, 0.05 mmol, 0.1 equiv.), arylboronic acid (0.75 mmol, 1.5 equiv.), K₂CO₃ (138 mg, 1.0 mmol, 2 equiv.) were dissolved in toluene (10 mL) or DMF (5 mL) or DMF/H₂O (3:1, 4 mL) and the mixture was heated to 100 °C for 18 h. The solvent was evaporated (in case of using DMF, the mixture was co-evaporated several times with toluene) and the crude product was purified by HPFC (SiO₂, cHex/EtOAc, 0 → 80%).

### General procedure D

6-Chloro-9-SEM-7-deazapurine **(IV)** or 6-chloro-9-SEM-8-aryl-7-deazapurine **(VIII)** (1.0 equiv.), arylboronic acid/ester (1.3 equiv.), Pd(OAc)₂ (0.05 equiv.), Cs₂CO₃ (4.0 equiv.), dppf (0.1 equiv.) and CuCl (1.0 equiv.) were dissolved in DMF and the mixture was heated to 100 °C for 3 h. The solvent was co-evaporated several times with toluene and the crude product was purified by HPFC (SiO₂, cHex/EtOAc, 0 → 100% or DCM/MeOH, 0 → 10%).

### C-H borylation in position 8

### General procedure E:

6-(2,4-Dimethoxypyrimidin-5-yl)-9-SEM-7-deazapurine **(V)** (1.0 equiv.), bispinacolatodiboron (1.2 equiv.), [Ir(COD)OMe]₂ (0.05 equiv.), 4,4'-di-*tert*-butyl-2,2'-bipyridine (0.1 equiv.) was dissolved in anhydrous THF (10 mL) under argon atmosphere. The solution was heated at 80 C for 1 h. The solvent was removed under reduced pressure. The crude product was purified by HPFC (SiO₂, DCM/MeOH, 0 → 3%).

### Suzuki coupling in position 8 of 6-aryl-8-Bpin-9-SEM-7-deazapurine

### General procedure F:

6-(2,4-Dimethoxypyrimidin-5-yl)-8-Bpin-9-SEM-7-deazapurine **(VI)** (0.5 mmol, 1.0 equiv.), aryl halide (1.1-2.25 equiv.), Pd(dppf)Cl₂ (18.3 mg, 0.025 mmol, 0.05 equiv.), K₂CO₃ (276 mg, 2.0 mmol, 4 equiv.) were dissolved in DMF (5 mL) and the mixture was heated to 90 °C for 1 h. The solvent was co-evaporated several times with toluene and the crude product was purified by HPFC (SiO₂, cHex/EtOAc, 0 → 80%).

### General procedure G:

6-(2,4-Dimethoxypyrimidin-5-yl)-8-Bpin-9-SEM-7-deazapurine **(VI)** (1.0 equiv.), aryl halide (1.4 equiv.), Pd(dppf)Cl₂ (0.1 equiv.), K₃PO₄ (2.5 equiv.) were dissolved in DMF (5 mL) or in dioxane (5 mL) or in dioxane/H₂O (7:5, 6 mL) and the mixture was heated to 90 °C for 1 h. The solvent was co-evaporated several times with toluene and the crude product was purified by HPFC (SiO₂, DCM/MeOH from 0 → 10%).

### SEM/Boc deprotection

### General procedure H:

A SEM-protected intermediate **(IX)** (0.68 mmol) was dissolved in a mixture of DCM (2 mL) and trifluoroacetic acid (7 mL) and the mixture was stirred at 25 °C for 1 h. Solvent was 10 × co-evaporated with MeOH. Saturated solution of NaHCO₃ was added to reach pH 7 or higher, solvent was evaporated, and the residue was stirred overnight with aq. ammonia (25% w/w, 34 mL). Next day, solvent was evaporated, and crude mixture was purified by HPFC on reverse phase (C18, H₂O/MeOH 5 → 100%) or by HPFC (SiO₂, DCM/MeOH 0 → 20%) followed by HPLC (XBridge^{®} Prep C18 5µm OBD - 19×250 mm - 20 mL/min, 5 min at 10%, gradient over 30 min, 5 min 100%).

### [4-(4-Chloro-7H-pyrrolo[2,3-d]pyrimidin-6-yl)-2-fluorophenyl]methanamine (II)

GP A starting from 4-chloro-6-iodo-7*H*-pyrrolo[2,3-*d*]pyrimidine (750 mg, 2.69 mmol) and [4-(aminomethyl)-3-fluorophenyl]boronic acid hydrochloride (600 mg, 2.96 mmol), HPFC (DCM/MeOH 0 → 15%) gave compound **II** (160 mg, 22%) as a yellow powder, which was used directly for next step. HR MS (ESI) for C₁₃H₁₁N₄ClF[M + H]⁺: calcd 277.0650; found 277.0653.

### {4-[4-(2,4-Dimethoxypyrimidin-5-yl)-7H-pyrrolo[2,3-d]pyrimidin-6-yl]-2-fluorophenyl}methanamine (1)

GP B staring from II (160 mg, 0.58 mmol) and (2,4-dimethoxypyrimidin-5-yl)boronic acid (159 mg, 0.87 mmol). HPFC (DCM/MeOH 0 → 30%) gave compound 1 (111 mg, 50%) as a white powder. ¹H NMR (500.0 MHz, DMSO-*d*₆): 3.78 (bs, 2H, CH₂N); 4.01 (s, 3H, CH₃O-4'); 4.02 (s, 3H, CH₃O-2'); 7.08 (s, 1H, H-5); 7.59 (t, 1H, *J*_{H,F} = *J*_{5,6} = 8.1, H-5-C₆H₃F); 7.77-7.83 (m, 2H, H-2,6-C₆H₃F); 8.67 (s, 1H, H-6'); 8.82 (s, 1H, H-2). ¹⁹F NMR (470.4 MHz, DMSO-d₆): -115.37 (dd, 1H, *J*_{F,H} = 11.1, 8.1). HR MS (ESI) for C₁₉H₁₈O₂N₆F [M + H]⁺: calcd 381.1469; found 381.1466.

### 4-Chloro-7-[(2-(trimethylsilyl)ethoxy)methyl]-7H-pyrrolo[2,3-d]pyrimidine (IV)

Solution of 6-chloro-7-deazapurine **(III)** (10 g, 65.2 mmol, 1.0 equiv.) in DMAc (30 mL, 2.0 M) was cooled to -5 °C in an ice/brine bath. NaH (4.5 g, 110 mmol, 1.7 equiv., 60 wt% in mineral oil) was added in portions as a solid. The solution darkened over 30 minutes. Then SEM-Cl (12.7 ml, 71.7 mmol, 1.1 equiv.) was added dropwise within 30 minutes. The reaction was stirred for 30 min at 22 °C. The mixture was treated with water (150 mL) and diluted with EtOAc (150 mL), layers were separated and the aqueous layer was extracted with EtOAc (3 × 100 mL). The combined organic layers were dried over MgSO₄. HPFC (SiO₂, cHex/EtOcA 0 → 10%) gave protected nucleobase **(IV)** (17.7 g, 96%) as a pale yellow oil which solidified in freezer.

*R_{f}=* 0.65 (SiO₂; cHex/Ea 7:3). ¹H NMR (400 MHz, CDCl₃): -0.06 (s, 9H, CH₃Si); 0.91 (m, 2H, OCH₂CH₂Si); 3.52 (m, 2H, OCH₂CH₂Si); 5.65 (s, 2H, NCH₂O); 6.67 (d, *J_{5,6} =* 3.7 Hz, 1H, 5-H); 7.39 *(d, J_{5,6} =* 3.6 Hz, 1H, 6-H); 8.66 (s, 1H, 2-H). HR MS (ESI) for C₁₂H₁₉ClN₃OSi⁺ ([M + H]⁺): calcd 284.0986; found: 284.0978.

### 4-(2,4-Dimethoxypyrimidin-5-yl)-7-[(2-(trimethylsilyl)ethoxy)methyl]-7H-pyrrolo[2,3-d]pyrimidine (V)

General procedure C starting from SEM-protected 6-chloro-7-deazapurine **(IV)** (15.0 g, 52.8 mmol), in DMF. HPFC (SiO₂, cHex/EtOAc 0 → 70%) gave V (20.3 g, 97%) as a pale yellow solid.

*R_{f}=* 0.14 (SiO₂; cHex/Ea 7:3). ¹H NMR (400 MHz, CDCl₃): -0.06 (s, 9H, CH₃Si); 0.92 (m, 2H, OCH₂CH₂Si); 3.57 (m, 2H, OCH₂CH₂Si); 4.06 (s, 3H, CH₃O-4'); 4.08 (s, 3H, CH₃O-2'); 5.67 (s, 2H, NCH₂O); 6.53 (d, *J_{5,6} =* 3.7 Hz, 1H, H-5); 7.36 (d, *J_{5,6} =* 3.7 Hz, 1H, H-6); 8.70 (s, 1H, H-6'); 8.95 (s, 1H, H-2). HR MS (ESI) for C₁₈H₂₆N₅O₃SI⁺ ([M + H]⁺): calcd 388.1805; found: 388.1796.

### 4-(2,4-Dimethoxypyrimidin-5-yl)-6-(4,4,5,5-tetramethyl-1,3,2-dioxaborolan-2-yl)-7-[2-(trimethylsilyl)ethoxymethyl]-7H-pyrrolo[2,3-d]pyrimidine (VI)

General procedure **E** starting from intermediate **V** (19.5 g, 50.3 mmol). HPFC (SiO₂, cHex/EtOAc 0 → 60%) gave borylated key-intermediate **VI** (16.8 g, 65%) as a pale orange solid.

*R_{f}=* 0.33 (SiO₂; cHex/Ea 1:1). ¹H NMR (400 MHz, CDCl₃): -0.07 (s, 9H, CH₃Si); 0.89 (m, 2H, OCH₂C**H**₂Si); 1.36 (s, 12H, (C**H₃**)₂C); 3.57 (m, 2H, OCH₂C**H₂**Si); 4.06 (s, 3H, CH₃O-4'), 4.08 (s, 3H, CH₃O-2'), 5.92 (s, 2H, NCH₂O), 7.11 (s, 1H, H-5), 8.65 (s, 1H, H-6'), 8.99 (s, 1H, H-2). HR MS (ESI) for C₂₄H₃₇BN₅O₅Si⁺ ([M + H]⁺: calcd 514.2657; found: 514.2647.

### 1-{4-[4-(2,4-Dimethoxypyrimidin-5-yl)-7H-pyrrolo[2,3-d]pyrimidin-6-yl]phenyl}propan-1-amine (2)

GP F starting from **VI** (300 mg, 0.58 mmol) and *tert-butyl* (1-(4-bromophenyl)propyl)carbamate (198 mg, 0.63 mmol). Deprotection by GP H (HPFC, SiO₂, DCM/MeOH 0 → 10%) gave compound 2 (89 mg, 39%) as a yellow foam.

¹H NMR (500.0 MHz, DMSO-*d*₆): 0.80 (t, 3H, *J*_{vic} = 7.4, CH₃CH₂CHN); 1.46-1.66 (m, 2H, CH₃CH₂CHN); 3.72 (t, 1H, *J*_{vic} = 6.7, CH₃CH₂CHN); 4.01 (s, 3H, CH₃O-4'); 4.02 (s, 3H, CH₃O-2'); 6.94 (s, 1H, H-5); 7.40-7.44 (m, 2H, CH-m-phenylene); 7.89-7.93 (m, 2H, CH-o-phenylene); 8.67 (s, 1H, H-6'); 8.79 (s, 1H, H-2). HR MS (ESI) for C₂₁H₂₃O₂N₆ [M + H]⁺: calcd 391.1877; found 391.1876.

### 1-{4-[4-(2,4-Dimethoxypyrimidin-5-yl)-7H-pyrrolo[2,3-d]pyrimidin-6-yl]phenyl}butan-1-amine (3)

GP F starting from **VI** (625 mg, 1.2 mmol) and *tert-butyl* (1-(4-bromophenyl)butyl)carbamate (599 mg, 1.8 mmol). Deprotection by GP H (HPFC, C-18, H₂O/MeOH 5 → 100%) gave compound 3 (115 mg, 23%) as a yellow solid.

¹H NMR (500 MHz, DMSO-d₆): 0.85 (t, 1H, *J_{CH3,CH2}* = 7.4 Hz, CH₃CH₂CH₂); 1.11 - 1.36 (m, 2H, CH₃CH₂CH₂); 1.45 - 1.60 (m, 2H, CH₃CH₂CH₂); 3.79 (t, 1H, *J_{CH,CH2} =* 6.8 Hz, CHNH₂); 4.01 (s, 3H, CH₃O-4'); 4.02 (s, 3H, CH₃O-2'); 6.93 (s, 1H, H-5); 7.42 (m, 2H, H-m-Ph); 7.90 (m, 2H, H-*o*-Ph); 8.67 (s, 1H, H-6'); 8.79 (s, 1H, H-2). HR MS (ESI) for C₂₂H₂₅N₆O₂⁺ ([M + H]⁺): calcd 405.2039; found 405.2030.

### 1-{4-[4-(2,6-Dimethoxypyrimidin-5-yl)-7H-pyrrolo[2,3-d]pyrimidin-6-yl]phenyl}-3,3-difluorocyclobutan-1-amine (4)

GP G starting from **VI** (300 mg, 0.58 mmol) and *tert-butyl* (1-(4-bromophenyl)-3,3-difluorocyclobutyl)carbamate (296 mg, 0.81 mmol). Deprotection by GP H (HPFC, SiO₂, DCM/MeOH 0 → 10%) gave compound **4** (181 mg, 71%) as a yellow solid.

¹H NMR (500 MHz, DMSO-d₆): 2.80 (btd, 2H, *J_{CH2,F}* = *J_{gem}* = 13_{.}8 Hz, *J_{CH2,CH2}* = 12.0 Hz, CCH₂-a); 3.08 (td, 2H, *J_{CH2,F}* = *J_{gem}* = 14.1 Hz, *J_{CH2,CH2}* = 10.4 Hz, CCH₂-b); 4.01 (s, 3H, CH₃O-4'); 4.02 (s, 3H, CH₃O-2'); 6.99 (s, 1H, H-5); 7.57 (m, 2H, H-m-Ph); 7.97 (m, 2H, H-*o*-Ph); 8.68 (s, 1H, H-6'); 8.81 (s, 1H, H-2); 12.67 (bs, 1H, NH). ¹⁹F NMR (470.4 MHz, DMSO-d₆): -85.36 (dpent, 1F, *J_{F,F} =* 193.3 Hz, *J_{F,CH2} =* 12.8 Hz, CF₂); -82.41 (dpent, 1F, *J_{F,F} =* 193.3 Hz, *J_{F,CH2} =* 11.8 Hz, CF₂).

HR MS (ESI) for C₂₂H₂₁N₆O₂⁺ (M+H)⁺: calcd 439.16886; found 439.16900.

### 3-{4-[4-(2,4-Dimethoxypyrimidin-5-yl)-7H-pyrrolo[2,3-d]pyrimidin-6-yl]phenyl}tetrahydro-2H-pyran-3-amine (5)

GP G starting from **VI** (300 mg, 0.58 mmol) and 3-(4-bromophenyl)tetrahydro-2*H*-pyran-3-amine (210 mg, 0.82 mmol). Deprotection by GP H (HPFC, SiO₂, DCM/MeOH 0 → 10% followed by HPLC) gave compound 5 (103 mg, 41%) as a yellow solid.

¹H NMR (500 MHz, DMSO-d₆): 1.47 (m, 1H, H-5a-C₅H₈O); 1.77-1.94 (m, 2H, H-4a,5b-C₅H₈O); 2.14 (m, 1H, H-4b-C₅H₈O); 3.49-3.63 (m, 2H, H-2a,6a-C₅H₈O); 3.70-3.84 (m, 2H, H-2b,6b-C₅H₈O); 4.01 (s, 3H, CH₃O-4'); 4.02 (s, 3H, CH₃O-2'); 7.00 (s, 1H, H-5); 7.69 (m, 2H, H-m-Ph); 7.98 (m, 2H, H-*o-*Ph); 8.68 (s, 1H, H-6'); 8.81 (s, 1H, H-2); 12.70 (bs, 1H, NH). HRMS (ESI) for C₂₃H₂₅N₆O₃⁺ (M+H)⁺: calcd 433.19827; found 433.19792.

### 4-(2,4-Dimethoxypyrimidin-5-yl)-6-[4-(piperazin-2-yl)phenyl]-7H-pyrrolo[2,3-d]pyrimidine (6)

GP F starting from **VI** (300 mg, 0.58 mmol) and 2-(4-bromophenyl)piperazine (212 mg, 0.88 mmol). Deprotection by GP H (HPFC, SiO₂, DCM/MeOH 0 → 10%) gave compound **6** (50 mg, 21%) as a yellow solid.

¹H NMR (600.1 MHz, DMSO-d6): 2.39 (m, 1H, H-3a-piperazinyl); 2.60 (m, 1H, H-5a-piperazinyl); 2.73 (m, 1H, H-6a-piperazinyl); 2.77 (m, 1H, H-5b-piperazinyl); 2.85 (m, 1H, H-3b-piperazinyl); 2.89 (m, 1H, H-6b-piperazinyl); 3.64 (dd, 1H, J_{2,3} = 10.0 and 2.7 Hz, H-2-piperazinyl); 4.01 (s, 3H, CH₃O-4'); 4.02 (s, 3H, CH₃O-2'); 6.93 (s, 1H, H-5); 7.45 (m, 2H, H-m-Ph); 7.91 (m, 2H, H-*o*-Ph); 8.67 (s, 1H, H-6'); 8.78 (s, 1H, H-2). HR MS (ESI) for C₂₂H₂₄O₂N₇ [M + H]⁺: calcd 418.19860; found 418.19876.

### 1-{4-[4-(2,4-Dimethoxypyrimidin-5-yl)-7H-pyrrolo[2,3-d]pyrimidin-6-yl]phenyl}-N-ethylpropan-1-amine (7)

GP F starting from **VI** (300 mg, 0.58 mmol) and 1-(4-bromophenyl)-*N*-ethylpropan-1-amine (170 mg, 0.70 mmol). Deprotection by GP H (HPFC, SiO₂, DCM/MeOH 0 → 10%) gave compound 7 (21 mg, 9%) as a yellow solid.

¹H NMR (600.1 MHz, DMSO-d₆): 0.76 (t, 3H, *J_{CH3,CH2}* = 7.4 Hz, CH₃CH₂CH); 0.98 (t, 3H, *J_{CH3,CH2}* = 7.1 Hz, CH₃CH₂NH); 1.54 (dpent, 1H, *J_{gem}* = 13.6 Hz, *J_{CH2,CH3}* = *J_{CH2,CH}* = 7.4 Hz, CH₃CH₂CH-a); 1.70 (bdpent, 1H, *J_{gem}* = 13.6 Hz, *J_{CH2,CH3}* = *J_{CH2,CH}* = 6.9 Hz, CH_{3C}H₂CH-b); 2.29 - 2.38 (m, 2H, CH₃CH₂NH); 3.51 (m, 1H, CH₃CH₂CH); 4.01 (s, 3H, CH₃O-4'); 4.02 (s, 3H, CH₃O-2'); 6.95 (s, 1H, H-5); 7.40 (m, 2H, H-m-Ph); 7.92 (m, 2H, H-o-Ph); 8.68 (s, 1H, H-6'); 8.80 (s, 1H, H-2); 12.61 (bs, 1H, NH). HR MS (ESI) for C₂₃H₂₇O₂N₆ [M + H]⁺: calcd 419.21900; found 419.21881.

### 6-[4-(2,4-Dimethoxypyrimidin-5-yl)-7H-pyrrolo[2,3-d]pyrimidin-6-yl]-5-fluoro-1,2,3,4-tetrahydroisoquinoline (8)

GP F starting from **VI** (200 mg, 0.39 mmol) and 6-bromo-5-fluoro-1,2,3,4-tetrahydroisoquinoline hydrochloride (156 mg, 0.59 mmol). Deprotection by GP H (HPFC, SiO₂, DCM/MeOH 0 → 10%) gave compound **8** (55 mg, 35%) as a yellow solid.

¹H NMR (500 MHz, DMSO-d₆): 2.68 (bt, 2H, *J_{4,3}* = 5.9 Hz, H-4-C₉H₉NF); 2.98 (t, 2H, *J_{3,4} =* 5.9 Hz, H-3-C₉H₉NF); 3.88 (s, 2H, H-1-C₉H₉NF); 4.00 (s, 3H, CH₃O-4'); 4.01 (s, 3H, CH₃O-2'); 6.83 (d, 1H, *J_{5,F} =* 2.8 Hz, H-5); 7.03 (d, 1H, *J_{8,7} =* 8.1 Hz, H-8-C₉H₉NF); 7.73 (t, 1H, *J_{7,8}* = *J_{7,F}* = 7.9 Hz, H-7-C₉H₉NF); 8.69 (s, 1H, H-6'); 8.83 (s, 1H, H-2); 12.58 (bs, 1H, NH). ¹⁹F NMR (470.4 MHz, DMSO-d₆): -119.92 (d, 1F, *J_{F,7} =7.7* Hz, F-5-C₉H₉NF). HR MS (ESI) for C₂₁H₂₀O₂N₆F [M + H]⁺: calcd 407.16263; found 407.16293; for C₂₁H₁₉O₂N₆FNa [M + Na]⁺: calcd 429.114457; found 429.14485.

### 1-{4-[4-(2,4-Dimethoxypyrimidin-5-yl)-7H-pyrrolo[2,3-d]pyrimidin-6-yl]phenyl}cyclohexan-1-amine (9)

GP F starting from **VI** (1.43 g, 2.79 mmol) and *tert-butyl* (1-(4-iodophenyl)cyclohexyl)carbamate (1.34 g, 3.35 mmol). Deprotection by GP H (HPFC, SiO₂, DCM/MeOH 0 → 10%) gave compound 9 (569 mg, 47%) as a yellow powder.

¹H NMR (500 MHz, DMSO-d₆): 1.27 (m, 1H, H-4a-cyclohexyl); 1.41-1.50 (m, 2H, H-3a,5a-cyclohexyl); 1.50-1.64 (m, 3H, H-2a,6a, 4b-cyclohexyl); 1.66-1.87 (m, 4H, H-2b,3b,5b,6b-cyclohexyl); 4.01 (s, 3H, CH₃O-4'); 4.02 (s, 3H, CH₃O-2'); 6.93 (s, 1H, H-5); 7.64 (m, 2H, H-m-Ph); 7.90 (m, 2H, H-*o*-Ph); 8.68 (s, 1H, H-6'); 8.80 (s, 1H, H-2). HR MS (APCI) for C₂₄H₂₇O₂N₆ [M + H]⁺: calcd 431.21900; found 431.21891.

### 7-[4-(2,4-Dimethoxypyrimidin-5-yl)-7H-pyrrolo[2,3-d]pyrimidin-6-yl]-6-fluoro-1,2,3,4-tetrahydroisoquinoline (10)

GP F starting from **VI** (200 mg, 0.39 mmol) and 7-bromo-6-fluoro-1,2,3,4-tetrahydroisoquinoline hydrochloride (156 mg, 0.58 mmol). Deprotection by GP H (HPFC, SiO₂, DCM/MeOH 0 → 10%) gave compound **10** (18 mg, 11%) as a yellow solid.

¹H NMR (500 MHz, DMSO-d₆): 2.92 (t, 2H, *J_{4,3}* = 6.1 Hz, H-4-C₉H₉NF); 3.23 (t, 2H, *J_{3,4} =* 6.1 Hz, H-3-C₉H₉NF); 4.00 (s, 3H, CH₃O-4'); 4.01 (s, 3H, CH₃O-2'); 4.14 (s, 2H, H-1-C₉H₉NF); 6.85 (d, 1H, *J_{5,F}* = 2.7 Hz, H-5); 7.23 (d, 1H, *J_{5,F} =* 12.0 Hz, H-5-C₉H₉NF); 7.77 (d, 1H, *J_{8,F} =* 7.7 Hz, H-8-C₉H₉NF); 8.70 (s, 1H, H-6'); 8.85 (s, 1H, H-2); 12.62 (vbs, 1H, NH). ¹⁹F NMR (470.4 MHz, DMSO-d₆): -116.76 (bs, 1F, F-6-C₉H₉NF). HR MS (ESI) for C₂₁H₂₀O₂N₆F [M + H]⁺: calcd 407.16263; found 407.16239.

### 6-[4-(2,4-Dimethoxypyrimidin-5-yl)-7H-pyrrolo[2,3-d]pyrimidin-6-yl]-4,4-dimethyl-1,2,3,4-tetrahydroisoquinoline (11)

GP F starting from **VI** (200 mg, 0.39 mmol) and 6-bromo-4,4-dimethyl-1,2,3,4-tetrahydroisoquinoline (144 mg, 0.58 mmol). Deprotection by GP H (HPFC, SiO₂, DCM/MeOH 0 → 10%) gave compound **11** (82 mg, 51%) as a yellow solid.

¹H NMR(500 MHz, DMSO-d₆): 1.29 (s, 6H, CH₃ -4''); 2.72 (s, 2H, H-3"); 3.86 (s, 2H, H-1''); 4.00 (s, 3H, CH₃O-4'); 4.02 (s, 3H, CH₃O-2'); 6.97 (s, 1H, H-5); 7.07 (d, 1H, *J_{8",7"} =* 8.0 Hz, H-8"); 7.68 (dd, 1H, *J_{7",8"} =* 8.0 Hz, *J_{7",5"} =* 1.9 Hz, H-7"); 7.98 (d, 1H, *J_{5",7"} =* 1.9 Hz, H-5"); 8.67 (s, 1H, H-6'); 8.79 (s, 1H, H-2); 12.63 (bs, 1H, NH-7). HR MS (ESI) for C₂₃H₂₅O₂N₆ [M + H]⁺: calcd 417.20335; found 417.20328.

### 1-{4-[4-(2,4-Dimethoxypyrimidin-5-yl)-7H-pyrrolo[2,3-d]pyrimidin-6-yl]-3-fluorophenyl}-N-methylpropan-1-amine (12)

GP F starting from **VI** (200 mg, 0.39 mmol) and 1-(4-bromo-3-fluorophenyl)-N-methylpropan-1-amine hydrochloride (166 mg, 0.58 mmol). Deprotection by GP H (HPFC, C-18, H₂O/MeOH 5 → 100%) gave compound **12** (87 mg, 53%) as a yellow solid.

"HNMR (500 MHz, DMSO-d₆): 0.77 (t, 3H, *J_{CH3,CH2}* = 7.4 Hz, CH₃CH₂CH); 1.52 and 1.67 (2×m, 2×1H, CH₃CH₂CH); 2.11 (s, 3H, CH₃NH); 3.37 (m, 1H, CH₃CH₂CH); 4.00 (s, 3H, CH₃O-4'); 4.01 (s, 3H, CH₃O-2'); 6.83 (d, 1H, *J_{8,F} =* 3.0 Hz, H-5); 7.25 (bdd, 1H, *J_{5,6} =* 8.0 Hz, *J_{5,3} =* 1.5 Hz, H-5-Ph); 7.28 (bdd, 1H, *J_{3,F} =* 12.8 Hz, *J_{3,5} =* 1.4 Hz, H-3-Ph); 7.97 (t, 1H, *J_{6,5} = J_{6,F} =* 8.0 Hz, H-6-Ph); 8.69 (s, 1H, H-6'); 8.81 (s, 1H, H-2). ¹⁹F NMR (470.4 MHz, DMSO-d₆): -113.96 (bt, 1F, *J_{F,3} = J_{F,6} =* 9.3 Hz, F-2-Ph). HR MS (ESI) for C₂₂H₂₄O₂N₆F [M + H]⁺: calcd 423.19393; found 423.19405; for C₂₂H₂₃O₂N₆FNa [M + Na]⁺: calcd 445.17587; found 445.17594.

### 1-{4-[4-(2,4-Dimethoxypyrimidin-5-yl)-7H-pyrrolo[2,3-d]pyrimidin-6-yl]-3-fluorophenyl}-N-methylbutan-1-amine (13)

GP F starting from **VI** (300 mg, 0.58 mmol) and 1-(4-bromo-3-fluorophenyl)-*N*-methylbutan-1-amine hydrochloride (261 mg, 0.88 mmol). Deprotection by GP H (HPFC, SiO₂, DCM/MeOH 0 → 10%) gave compound **13** (63 mg, 25%) as a yellow solid.

¹H NMR (500 MHz, DMSO-d₆): 0.83 (t, 3H, *J_{CH3,CH2}* = 7.4 Hz, CH₃CH₂CH₂CH); 1.09 - 1.32 (m, 2H, CH₃C**H₂**CH₂CH); 1.48 and 1.62 (2×m, 2×1H, CH₃CH₂C**H₂**CH); 2.10 (s, 3H, C**H₃**NH); 3.45 (t, 1H, *J_{CH,CH2}* = 6.9 Hz, CH₃CH₂CH₂C**H**); 4.00 (s, 3H, CH₃O-4'); 4.01 (s, 3H, CH₃O-2'); 6.83 (d, 1H, *J_{8,F} =* 3.0 Hz, H-5); 7.25 (bdd, 1H, *J_{5,6}=* 8.0 Hz, *J_{5,3} =* 1.4 Hz, H-5-Ph); 7.28 (bd, 1H, *J_{3,F} =* 12.8 Hz, H-3-Ph); 7.97 (t, 1H, *J_{6,5} = J_{6,F} =* 8.0 Hz, H-6-Ph); 8.69 (s, 1H, H-6'); 8.81 (s, 1H, H-2). ¹⁹F NMR (470.4 MHz, DMSO-d₆): -113.96 (bt, 1F, *J_{F,3} = J_{F,6} =* 9.6 Hz, F-2-Ph). HR MS (ESI) for C₂₃H₂₆O₂N₆F [M + H]⁺: calcd 437.20958; found 437.20970; for C₂₃H₂₅O₂N₆FNa [M +Na]⁺: calcd 459.19152; found 459.19174.

### 1-{4-[4-(2,4-Dimethoxypyrimidin-5-yl)-7H-pyrrolo[2,3-d]pyrimidin-6-yl]phenyl}cycloheptan-1-amine (14)

GP F starting from **VI** (200 mg, 0.39 mmol) and 1-(4-bromophenyl)cycloheptan-1-amine hydrochloride (178 mg, 0.58 mmol). Deprotection by GP H (HPFC, SiO₂, DCM/MeOH 0 → 10% followed by C-18, H₂O/MeOH 5 → 100%) gave compound **14** (80 mg, 46%) as a yellow solid.

¹H NMR (500 MHz, DMSO-d₆): 1.47 (m, 2H, H-3a,6a-cycloheptyl); 1.51-1.63 (m, 4H, H-4,5-cycloheptyl); 1.65 (m, 2H, H-2a,7a-cycloheptyl); 1.74 (m, 2H, H-3b,6b-cycloheptyl); 1.98 (m, 2H, H-2b,7b-cycloheptyl); 4.02 (s, 3H, CH₃O-4'); 4.03 (s, 3H, CH₃O-2'); 6.93 (s, 1H, H-5); 7.63 (m, 2H, H-*m*-Ph); 7.90 (m, 2H, H-*o*-Ph); 8.68 (s, 1H, H-6'); 8.80 (s, 1H, H-2). HR MS (ESI) for C₂₅H₂₉O₂N₆ [M + H]⁺: calcd 445.23465; found 445.23459; for C₂₅H₂₈O₂N₆Na [M + Na]⁺: calcd 467.21660; found 467.21653.

### 4-{4-[4-(2,4-Dimethoxypyrimidin-5-yl)-7H-pyrrolo[2,3-d]pyrimidin-6-yl]phenyl}tetrahydro-2H-pyran-4-amine (15)

GP F starting from **VI** (300 mg, 0.58 mmol) and 4-(4-iodophenyl)tetrahydro-2*H*-pyran-4-amine (267 mg, 0.88 mmol). Deprotection by GP H (HPFC, SiO₂, DCM/MeOH 0 → 10% followed by C-18, H₂O/MeOH 5 → 100%) gave compound **15** (131 mg, 52%) as a yellow solid.

¹H NMR (500 MHz, DMSO-d₆): 1.54 (dm, 2H, *J_{gem} =* 13.4 Hz, H-3a,5a-tetrahydropyranyl); 1.98 (ddd, 2H, *J_{gem}* = 13.4 Hz, *J_{3b,2b}* = 11.4 Hz, *J_{3b,2},* = 4.7 Hz, H-3b,5b-tetrahydropyranyl); 3.64 (bddd, 2H, *J_{gem} =* 11.2 Hz, *J_{2a,3b}* = 4.3 Hz, *J_{2a,3a}* = 3.0 Hz, H-2a,6a-tetrahydropyranyl); 3.88 (td, 2H, *J_{gem} = J_{2b,3b} =* 11.22 Hz, *J_{2b,3a}* = 2.3 Hz, H-2b,6b-tetrahydropyranyl); 4.01 (s, 3H, CH₃O-4'); 4.02 (s, 3H, CH₃O-2'); 6.96 (s, 1H, H-5); 7.62 (m, 2H, H-m-Ph); 7.93 (m, 2H, H-*o*-Ph); 8.68 (s, 1H, H-6'); 8.80 (s, 1H, H-2); 12.64 (vbs, 1H, NH). HR MS (ESI) for C₂₃H₂₅O₃N₆ [M + H]⁺: calcd 433.19827; found 433.19831.

### (R)-7-[4-(2,4-Dimethoxypyrimidin-5-yl)-7H-pyrrolo[2,3-d]pyrimidin-6-yl]chroman-4-amine (16)

GP F starting from **VI** (400 mg, 0.78 mmol) and *tert*-butyl (*R*)-(7-bromochroman-4-yl)carbamate (308 mg, 0.94 mmol). Deprotection by GP H (HPFC, SiO₂, DCM/MeOH 0 → 10% followed by C-18, H₂O/MeOH 5 → 100%) gave compound **16** (50 mg, 16%) as a yellow solid.

¹H NMR (500 MHz, DMSO-d₆): 1.77 (dtd, 1H, *J_{gem}* = 13.4 Hz, *J_{3a,2a}* = *J_{3a,4}* = 6.7 Hz, *J_{3a,2b}* = 2.9 Hz, H-3a-chromanyl); 2.03 (dddd, 1H, *J_{gem} =* 13.4 Hz, *J_{3b,2b}* = 8.2 Hz, *J_{3b,4} =* 5.0 Hz, *J_{3b,2a}* = 3.2 Hz, H-3b-chromanyl); 3.96 (t, 1H, *J_{4,3}a = J_{4,3b}* = 5.6 Hz, H-4-chromanyl); 4.00 (s, 3H, CH₃O-4'); 4.02 (s, 3H, CH₃O-2'); 4.18 (ddd, 1H, *J_{gem} =* 10.9 Hz, *J_{2a,3b}* = 7.0 Hz, *J_{2a,3b}* = 3.17 Hz, H-2a-chromanyl); 4.30 (ddd, 1H, *J_{gem} =* 10.9 Hz, *J_{2b,3b}* = 8.2 Hz, *J_{2b,3a}* = 2.9 Hz, H-2b-chromanyl); 6.81 (s, 1H, H-5); 6.82 (d, 1H, *J_{8,7}* = 8.5 Hz, H-8-chromanyl); 7.70 (dd, 1H, *J_{7,8} =* 8.5 Hz, *J_{7,5} =* 2.3 Hz, H-7-chromanyl); 8.01 (d, 1H, *J_{5,7}* = 2.2 Hz, H-5-chromanyl); 8.65 (s, 1H, H-6'); 8.76 (s, 1H, H-2); 12.47 (vbs, 1H, NH). HR MS (ESI) for C₂₁H₂₁O₃N₆ [M + H]⁺: calcd 405.16697; found 405.16692; for C₂₁H₂₀O₃N₆Na [M + Na]⁺: calcd 427.14891; found 427.14883.

### 2-{3-[4-(2,4-Dimethoxypyrimidin-5-yl)-7H-pyrrolo[2,3-d]pyrimidin-6-yl]phenyl}propan-2-amine (17)

GP F starting from **VI** (300 mg, 0.58 mmol) and 2-(3-bromophenyl)propan-2-amine (188 mg, 0.88 mmol). Deprotection by GP H (HPFC, SiO₂, DCM/MeOH 0 → 10% followed by C-18, H₂O/MeOH 5 → 100%) gave compound **17** (140 mg, 62%) as a yellow solid.

¹H NMR (500 MHz, DMSO-d₆): 1.42 (s, 2×3H, CH₃C); 4.01 (s, 3H, CH₃O-4'); 4.02 (s, 3H, CH₃O-2'); 7.00 (s, 1H, H-5); 7.39 (t, 1H, *J_{5,4} = J_{5,6} =* 7.8 Hz, H-5-Ph); 7.75 (dm, 1H, *J_{4,5} =* 7.8 Hz, H-4-Ph); 7.78 (dm, 1H, *J_{6,5} =* 7.8 Hz, H-6-Ph); 7.12 (t, 1H, *J_{2,4} = J_{2,6} =* 1.8 Hz, H-2-Ph); 8.68 (s, 1H, H-6'); 8.81 (s, 1H, H-2). HR MS (ESI) for C₂₁H₂₃O₂N₆ [M + H]⁺: calcd 391.18770; found 391.18762; for C₂₁H₂₂O₂N₆Na [M + Na]⁺: calcd 413.16965; found 413.16941.

### (R)-1-{4-[4-(2,4-Dimethoxypyrimidin-5-yl)-7H-pyrrolo[2,3-d]pyrimidin-6-yl]phenyl}-3-methylbutan-1-amine (18)

GP F starting from **VI** (300 mg, 0.58 mmol) and (R)-1-(4-bromophenyl)-3-methylbutan-1-amine hydrochloride (244 mg, 0.88 mmol). Deprotection by GP H (HPFC, SiO₂, DCM/MeOH 0 → 10% followed by C-18, H₂O/MeOH 5 → 100%) gave compound **18** (140 mg, 57%) as a yellow solid.

¹H NMR (500 MHz, DMSO-d₆): 0.86 and 0.89 (2×d, 2×3H, *J_{CH3,CH} =* 6.4 Hz, ((CH₃)₂CHCH₂); 1.32 - 1.58 (m, 3H, ((CH₃)₂CHCH₂); 3.85 (t, 1H, *J_{CH,CH2} =* 7.1 Hz, CHNH₂); 4.01 (s, 3H, CH₃O-4'); 4.02 (s, 3H, CH₃O-2'); 6.94 (s, 1H, H-5); 7.43 (m, 2H, H-*m*-Ph); 7.90 (m, 2H, H-*o*-Ph); 8.67 (s, 1H, H-6'); 8.80 (s, 1H, H-2). HR MS (ESI) for C₂₃H₂₇O₂N₆ [M + H]⁺: calcd 419.21900; found 419.21880.

### 1-{3-[4-(2,4-Dimethoxypyrimidin-5-yl)-7H-pyrrolo[2,3-d]pyrimidin-6-yl]phenyl}cyclopropan-1-amine (19)

GP F starting from **VI** (300 mg, 0.58 mmol) and 1-(3-bromophenyl)cyclopropan-1-amine (186 mg, 0.88 mmol). Deprotection by GP H (HPFC, SiO₂, DCM/MeOH 0 → 10% followed by C-18, H₂O/MeOH 5 → 100%) gave compound **19** (56 mg, 25%) as a yellow solid.

¹H NMR (500 MHz, DMSO-d₆): 0.98-1.06 (m, 4H, CH₂-cyclopropyl); 4.01 (s, 3H, CH₃O-4'); 4.02 (s, 3H, CH₃O-2'); 7.00 (s, 1H, H-5); 7.34 (dt, 1H, *J_{4,5} =* 7.9 Hz, *J_{4,6} = J_{4,2} =* 1.6 Hz, H-4-Ph); 7.38 (t, 1H, *J_{5,4} = J_{5,6} =* 7.6 Hz, H-5-Ph); 7.74 (dt, 1H, *J_{6,5} =* 7.4 Hz, *J_{6,4} = J_{6,2} =* 1.5 Hz, H-6-Ph); 7.86 (bt, 1H, *J_{2,4} = J_{2,6} =* 1.7 Hz, H-2-Ph); 8.67 (s, 1H, H-6'); 8.81 (s, 1H, H-2); 12.64 (bs, 1H, NH). HR MS (ESI) for C₂₁H₂₁O₂N₆ [M + H]⁺: calcd 389.17205; found 389.17194; for C₂₁H₂₀O₂N₆Na [M + Na]⁺: calcd 411.15400; found 411.15383.

### 3-{4-[4-(2,4-Dimethoxypyrimidin-5-yl)-7H-pyrrolo[2,3-d]pyrimidin-6-yl]phenyl}pentan-3-amine (20)

GP F starting from **VI** (300 mg, 0.58 mmol) and *tert-butyl* (3-(4-bromophenyl)pentan-3-yl)carbamate (240 mg, 0.70 mmol). Deprotection by GP H (HPFC, SiO₂, DCM/MeOH 0 → 10% followed by C-18, H₂O/MeOH 5 → 100%) gave compound **20** (109 mg, 45%) as a yellow solid.

¹H NMR (500 MHz, DMSO-d₆): 0.65 (t, 6H, *J_{CH3,CH2} =* 7.3 Hz, (CH₃CH₂C); 1.55-1.65 (m, 2H, CH₃CH₂C-a); 1.72-1.82 (m, 2H, CH₃CH₂C-b); 4.01 (s, 3H, CH₃O-4'); 4.02 (s, 3H, CH₃O-2'); 6.94 (s, 1H, H-5); 7.52 (m, 2H, H-*m*-Ph); 7.91 (m, 2H, H-*o*-Ph); 8.68 (s, 1H, H-6'); 8.80 (s, 1H, H-2); 12.62 (vbs, 1H, NH). HR MS (ESI) for C₂₃H₂₇O₂N₆ [M + H]⁺: calcd 419.21900; found 419.21875; for C₂₃H₂₆O₂N₆Na [M + Na]⁺: calcd 441.20095; found 441.20059.

### 1-{4-[4-(2,4-Dimethoxypyrimidin-5-yl)-7H-pyrrolo[2,3-d]pyrimidin-6-yl]-3-fluorophenyl}cyclohexan-1-amine (21)

GP F starting from **VI** (300 mg, 0.58 mmol) and *tert-butyl* (1-(4-bromo-3-fluorophenyl)cyclohexyl)carbamate (260 mg, 0.70 mmol). Deprotection by GP H (HPFC, SiO₂, DCM/MeOH 0 → 10% followed by C-18, H₂O/MeOH 5 → 100%) gave compound 21 (130 mg, 50%) as a yellow solid.

¹H NMR (500 MHz, DMSO-d₆): 1.27 (m, 1H, H-4a-cyclohexyl); 1.40-1.50 (m, 2H, H-3a,5a-cyclohexyl); 1.50-1.58 (m, 2H, H-2a,6a); 1.60 (m, 1H, H-4b-cyclohexyl); 1.67-1.84 (m, 4H, H-2b,3b,5b,6b-cyclohexyl); 4.00 (s, 3H, CH₃O-4'); 4.01 (s, 3H, CH₃O-2'); 6.84 (d, 1H, *J_{5,F} =* 2.7 Hz, H-5); 7.50 (dd, 1H, *J_{5,6} =* 8.2 Hz, *J_{5,3} =* 1.8 Hz, H-5-Ph); 7.53 (dd, 1H, *J_{3,F} =* 13.8 Hz, *J_{3,5} =* 1.8 Hz, H-3-Ph); 7.92 (t, 1H, *J_{6,5} = J_{6,F} =* 8.3 Hz, H-6-Ph); 8.70 (s, 1H, H-6'); 8.84 (s, 1H, H-2). ¹⁹F NMR (470.4 MHz, DMSO-d₆): -113.71 (ddd, 1F, *J_{F,3} =* 13.8 Hz, *J_{F,6} =* 8.3 Hz, *J_{F,5}* = 2.7 Hz, F-2-Ph). HR MS (ESI) for C₂₄H₂₆O₂N₆F [M + H]⁺: calcd 449.20958; found 449.20952; for C₂₄H₂₅O₂N₆FNa [M + Na]⁺: calcd 471.19152; found 471.19147.

### 4-{4-[4-(2,4-Dimethoxypyrimidin-5-yl)-7H-pyrrolo[2,3-d]pyrimidin-6-yl]phenyl}heptan-4-amine (22)

GP F starting from **VI** (300 mg, 0.58 mmol) and *tert-butyl* (4-(4-bromophenyl)heptan-4-yl)carbamate (432 mg, 1.16 mmol). Deprotection by GP H (HPFC, SiO₂, DCM/MeOH 0 → 10% followed by C-18, H₂O/MeOH 5 → 100%) gave compound **22** (111 mg, 43%) as a yellow solid.

¹H NMR (500 MHz, DMSO-d₆): 0.78 (t, 6H, *J_{CH3,CH2} =* 7.3 Hz, (CH₃CH₂CH₂C); 0.83 - 0.95 (m, 2H, CH₃CH₂CH₂-a); 1.19-1.32 (m, 2H, CH₃CH₂CH₂-b); 1.51-1.61 (m, 2H, CH₃CH₂CH₂C-a); 1.67-1.77 (m, 2H, CH₃CH₂CH₂-b); 4.02 (s, 3H, CH₃O-4'); 4.03 (s, 3H, CH₃O-2'); 6.94 (s, 1H, H-5); 7.53 (m, 2H, H-m-Ph); 7.90 (m, 2H, H-*o*-Ph); 8.68 (s, 1H, H-6'); 8.80 (s, 1H, H-2); 12.62 (vbs, 1H, NH). HR MS (ESI) for C₂₅H₃₁O₂N₆ [M + H]⁺: calcd 447.25030; found 447.25022; for C₂₅H₃₀O₂N₆Na [M + Na]⁺: calcd 469.23225; found 469.23205.

### 5-{4-[4-(2,4-Dimethoxypyrimidin-5-yl)-7H-pyrrolo[2,3-d]pyrimidin-6-yl]phenyl}nonan-5-amine (23)

GP F starting from VI (300 mg, 0.58 mmol) and *tert-butyl* (5-(4-bromophenyl)nonan-5-yl)carbamate (465 mg, 1.16 mmol). Deprotection by GP H (HPFC, SiO₂, DCM/MeOH 0 → 10% followed by C-18, H₂O/MeOH 5 → 100%) gave compound **23** (99 mg, 36%) as a yellow solid.

¹H NMR (500 MHz, DMSO-d₆): 0.78 (t, 6H, *J_{CH3,CH2} =* 7.2 Hz, (CH₃CH₂CH₂CH₂C); 0.80-0.91 (m, 2H, CH₃CH₂CH₂CH₂-a); 1.11-1.26 (m, 6H, CH₃CH₂CH₂CH₂-b, CH₃CH₂CH₂CH₂); 1.52-1.61 and 1.69-1.79 (2×m, 2×2H, CH₃CH₂CH₂CH₂C); 4.01 (s, 3H, CH₃O-4'); 4.02 (s, 3H, CH₃O-2'); 6.94 (s, 1H, H-5); 7.51 (m, 2H, H-*m*-Ph); 7.89 (m, 2H, H-*o*-Ph); 8.67 (s, 1H, H-6'); 8.79 (s, 1H, H-2); 12.61 (vbs, 1H, NH). HR MS (ESI) for C₂₇H₃₅O₂N₆ [M + H]⁺: calcd 475.28160; found 475.28149; for C₂₇H₃₄O₂N₆Na [M + Na]⁺: calcd 497.26355; found 497.26344.

### 4-{4-[4-(2,4-Dimethoxypyrimidin-5-yl)-7H-pyrrolo[2,3-d]pyrimidin-6-yl]phenyl}-2,6-dimethylheptan-4-amine (24)

GP F starting from **VI** (300 mg, 0.58 mmol) and *tert*-butyl (4-(4-bromophenyl)2,6-dimethylheptan-4-yl)carbamate (465 mg, 1.16 mmol). Deprotection by GP H (HPFC, SiO₂, DCM/MeOH 0 → 10% followed by C-18, H₂O/MeOH 5 → 100%) gave compound **24** (84 mg, 30%) as a yellow solid.

¹H NMR (500 MHz, DMSO-d₆): 0.49 and 0.85 (2×d, 2×6H, *J_{CH3,CH} =* 6.3 Hz, (CH₃)₂CHCH₂C); 1 44-1.57 (m, 4H, (CH₃)₂CHCH₂, (CH₃)₂CHCH₂-a); 1.69-1.58 (m, 2H, (CH₃)₂CHCH₂-b); 4.01 (s, 3H, CH₃O-4'); 4.02 (s, 3H, CH₃O-2'); 6.95 (s, 1H, H-5); 7.55 (m, 2H, H-m-Ph); 7.90 (m, 2H, H-*o*-Ph); 8.67 (s, 1H, H-6'); 8.79 (s, 1H, H-2); 12.61 (vbs, 1H, NH). HR MS (ESI) for C₂₇H₃₅O₂N₆ [M + H]⁺: calcd 475.28160; found 475.28152; for C₂₇H₃₄₎O₂N₆Na[M +Na]⁺: calcd 497.26355; found 497.26349.

### 5-{4-[4-(2,4-Dimethoxypyrimidin-5-yl)-7H-pyrrolo[2,3-d]pyrimidin-6-yl]phenyl}spiro[2.3]hexan-5-amine (25)

GP F starting from **VI** (360 mg, 0.7 mmol) and *tert*-butyl (5-(4-bromophenyl)spiro[2.3]hexan-5-yl)carbamate (370 mg, 1.05 mmol). Deprotection by GP H (HPFC, SiO₂, DCM/MeOH 0 → 10% followed by C-18, H₂O/MeOH 5 → 100%) gave compound **25** (140 mg, 47%) as a white powder.

¹H NMR (500 MHz, DMSO-d₆): 0.43-0.48 and 0.50-0.55 (2×m, 2×2H, H-4",5''); 2.33-2.38 and 2.44-2.49 (2×m, 2×2H, H-2",6"); 4.01 (s, 3H, CH₃O-4'); 4.02 (s, 3H, CH₃O-2'); 6.95 (s, 1H, H-5); 7.66 (m, 2H, H-m-Ph); 7.94 (m, 2H, H-o-Ph); 8.68 (s, 1H, H-6'); 8.80 (s, 1H, H-2); 12.62 (bs, 1H, NH). HR MS (ESI) for C₂₄H₂₅O₂N₆ [M + H]⁺: calcd 429.20335; found 429.20328; for C₂₄H₂₄O₂N₆Na [M + Na]⁺: calcd 451.18530; found 451.18520.

### 3-{4-[4-(2,4-Dimethoxypyrimidin-5-yl)-7H-pyrrolo[2,3-d]pyrimidin-6-yl]phenyl}-2,4-dimethylpentan-3-amine (26)

GP F starting from **VI** (239 mg, 0.47 mmol) and 3-(4-bromophenyl)-2,4-dimethylpentan-3-amine (189 mg, 0.71 mmol). Deprotection by GP H (HPFC, SiO₂, DCM/MeOH 0 → 10% followed by C-18, H₂O/MeOH 5 → 100%) gave compound **26** (66 mg, 32%) as a yellowish powder.

¹H NMR (500 MHz, DMSO-d₆): 0.68 and 0.76 (2×d, 2×3H, *J_{CH3,CH} =* 6.7 Hz, CH₃-*i*Pr); 2.20 (sept, 2H, CH-iPr); 4.01 (s, 3H, CH₃O-4'); 4.02 (s, 3H, CH₃O-2'); 6.95 (s, 1H, H-5); 7.58 (m, 2H, H-m-Ph); 7.90 (m, 2H, H-o-Ph); 8.68 (s, 1H, H-6'); 8.80 (s, 1H, H-2); 12.561 (bs, 1H, NH). HR MS (ESI) for C₂₅H₃₁O₂N₆ [M + H]⁺: calcd 447.25030; found 447.25052.

### 6-[4-(2,4-Dimethoxypyrimidin-5-yl)-7H-pyrrolo[2,3-d]pyrimidin-6-yl]-1,2,3,4-tetrahydroisoquinoline (27)

GP F starting from **VI** (129 mg, 0.25 mmol) and 6-bromo-1,2,3,4-tetrahydroisoquinoline (58 mg, 0.275 mmol). Deprotection by GP H (HPFC, C-18, H₂O/MeOH 5 → 100%) gave compound **27** (35 mg, 36%) as a white powder.

¹H NMR (500 MHz, DMSO-d₆): 2.74 (t, 2H, *J_{4,3}* = 5.8 Hz, H-4-C₉H₁₀N); 2.96 (t, 2H, *J_{3,4} =* 5.9 Hz, H-3-C₉H₁₀N); 3.87 (s, 2H, H-1-C₉H₁₀N); 4.01 (s, 3H, CH₃O-4'); 4.03 (s, 3H, CH₃O-2'); 6.91 (s, 1H, H-5); 7.11 (bd, 1H, *J_{8,7}* = 8.7 Hz, H-8-C₉H₁₀N); 7.70 (m, 1H, H-7-C₉H₁₀N); 7.71 (s, 1H, H-5-C₉H₁₀N); 8.66 (s, 1H, H-6'); 8.78 (s, 1H, H-2); 12.58 (bs, 1H, NH-7). HRMS (ESI) calculated for C₂₁H₂₁O₂N₆: 389.17205; found: 389.17220.

### 4-(2,4-Dimethoxypyrimidin-5-yl)-6-[(4-pyrrolidin-2-yl)phenyl]-7H-pyrrolo[2,3-d]pyrimidine (28)

GP F starting from **VI** (130 mg, 0.25 mmol) and 2-(4-bromophenyl)pyrrolidine (62 mg, 0.275 mmol). Deprotection by GP H (HPFC, C-18, H₂O/MeOH 5 → 100%) gave compound **28** (37 mg, 37%) as a white powder.

¹H NMR (500 MHz, DMSO-d₆): 1.51 (ddt, 1H, *J_{gem}* = 12.1 Hz, *J_{3a,4}* = 9.1 Hz, *J_{3a,4}* = *J_{3a,2}* = 7.9 Hz, H-3a-pyrrolidinyl); 1.66-1.86 (m, 2H, H-4-pyrrolidinyl); 2.15 (dddd, 1H, *J_{gem} =* 12.1 Hz, *J_{3b,4} =* 8.2 Hz, *J_{3b,2} =* 7.2 Hz, *J_{3b,4} =* 4.9 Hz, H-3b-pyrrolidinyl); 2.91 (ddd, 1H, *J_{gem}* = 9.9 Hz, *J_{5a,4} =* 8.2 and 6.5 Hz, H-5a-pyrrolidinyl); 3.04 (ddd, 1H, *J_{gem}* = 9.9 Hz, *J_{5b,4} =* 7.6 and 5.4 Hz, H-5b-pyrrolidinyl); 3.17 (m, 1H, NH-pyrrolidinyl); 4.02 (s, 3H, CH₃O-4'); 4.01 (s, 3H, CH₃O-2'); 4.09 (t, 1H, *J_{2,3a} = J_{2,3b} =* 7.7 Hz, H-2-pyrrolidinyl); 6.94 (s, 1H, H-5);7.46 (m, 2H, H-m-Ph); 7.91 (m, 2H, H-o-Ph); 8.67 (s, 1H, H-6'); 8.80 (s, 1H, H-2); 12.63 (vbs, 1H, NH). HRMS (ESI) calculated for C₂₂H₂₃O₂N₆: 403.18770; found: 403.18761.

### 7-[4-(2,4-Dimethoxypyrimidin-5-yl)-7H-pyrrolo[2,3-d]pyrimidin-6-yl]-1,2,3,4-tetrahydroisoquinoline (29)

GP F starting from **VI** (130 mg, 0.25 mmol) and 7-bromo-1,2,3,4-tetrahydroisoquinoline (52 mg, 0.26 mmol). Deprotection by GP H (HPFC, C-18, H₂O/MeOH 5 → 100%) gave compound **29** (25 mg, 25%) as a white solid.

¹H NMR (500 MHz, DMSO-d₆): 3.03 (t, 2H, *J_{4,3}* = 6.3 Hz, H-4-C₉H₁₀N); 3.43 (t, 2H, *J_{3,4} =* 6.3 Hz, H-3-C₉H₁₀N); 4.01 (s, 3H, CH₃O-4'); 4.02 (s, 3H, CH₃O-2'); 4.33 (s, 2H, H-1-C₉H₁₀N); 6.98 (d, 1H, *J_{5,NH}* = 2.0 Hz, H-5); 7.36 (d, 1H, *J_{5,6}* = 8.1 Hz, H-5-C₉H₁₀N); 7.86 (bd, 1H, *J_{8,6}* = 1.9 Hz, H-8-C₉H₁₀N); 7.88 (dd, 1H, *J_{6,5} =* 8.0 Hz, *J_{6,8} =* 2.0 Hz, H-6-C₉H₁₀N); 8.67 (s, 1H, H-6'); 8.82 (s, 1H, H-2); 9.04 (bs, 2H, NH-2-C₉H₁₀N); 12.69 (d, 1H, *J_{NH,5}* = 2.0 Hz, NH-7). HRMS (ESI) calculated for C₂₁H₂₁O₂N₆: 389.17205; found: 389.17198.

### 4-(2,4-Dimethoxypyrimidin-5-yl)-6-(isoindolin-5-yl)-7H-pyrrolo[2,3-d]pyrimidine (30)

GP F starting from **VI** (130 mg, 0.25 mmol) and 5-bromo-isoindoline (55 mg, 0.26 mmol). Deprotection by GP H (HPFC, C-18, H₂O/MeOH 5 → 100%) gave compound **30** (30 mg, 32%) as a white solid.

¹H NMR (500 MHz, DMSO-d₆): 4.01 (s, 3H, CH₃O-4'); 4.02 (s, 3H, CH₃O-2'); 4.53-4.61 (m, 4H, H-1,3-C₈H₈N); 7.03 (d, 1H, *J_{5,NH}* = 2.1 Hz, H-5); 7.53 (d, 1H, *J_{7,6}* = 8.0 Hz, H-7-C₈H₈N); 7.98 (dd, 1H, *J_{6,7}* = 8.0 Hz, *J_{6,4} =* 1.7 Hz, H-6-C₈H₈N); 8.01 (bs, 1H, H-4-C₈H₈N); 8.68 (s, 1H, H-6'); 8.83 (s, 1H, H-2); 9.45 (bs, 2H, NH-2-C₈H₈N); 12.74 (bd, 1H, *J_{NH,5} =* 1.9 Hz, NH). HRMS (ESI) calculated for C₂₀H₁₉O₂N₆: 375.15640; found: 375.15630.

### N-{3-[4-(2,4-dimethoxypyrimidin-5-yl)-7H-pyrrolo[2,3-d]pyrimidin-6-yl]benzyl}cyclopropanamine (31)

GP F starting from **VI** (130 mg, 0.25 mmol) and N-(3-bromobenzyl)cyclopropanamine (62 mg, 0.275 mmol). Deprotection by GP H (HPFC, C-18, H₂O/MeOH 5 → 100%) gave compound **31** (30 mg, 30%) as a white solid.

¹H NMR (500 MHz, DMSO-d₆): 0.28 and 0.36 (2×m, 2×2H, CH₂-cyclopropyl); 2.08 (m, 1H, CH-cyclopropyl); 3.79 (s, 2H, CH₂NH); 4.02 (s, 3H, CH₃O-4'); 4.03 (s, 3H, CH₃O-2'); 6.99 (s, 1H, H-5); 7.35 (dm, 1H, *J_{4,5} =* 7.6 Hz, H-4-Ph); 7.42 (t, 1H, *J_{5a,4} = J₅*,*₆ =* 7.6 Hz, H-5-Ph); 7.83 (bdt, 1H, *J_{6,5} =* 7.8 *Hz, J_{6,4} = J_{6,2} =* 1.5 Hz, H-6-Ph); 7.95 (bt, 1H, *J_{2,6} = J_{2,4} =* 1.6 Hz, H-2-Ph); 8.68 (s, 1H, H-6'); 8.81 (s, 1H, H-2); 12.65 (bs, 1H, NH-7). HRMS (ESI) calculated for C₂₂H₂₃O₂N₆: 403.18770; found: 403.18732.

### 4-(2,4-Dimethoxypyrimidin-5-yl)-6-[(3-pyrrolidin-2-yl)phenyl]-7H-pyrrolo[2,3-d]pyrimidine (32)

GP F starting from VI (130 mg, 0.25 mmol) and 2-(3-bromophenyl)pyrrolidine (62 mg, 0.275 mmol). Deprotection by GP H (HPFC, C-18, H₂O/MeOH 5 → 100%) gave compound **32** (25 mg, 25%) as a white solid.

¹H NMR (500 MHz, DMSO-d₆): 1.55 (ddt, 1H, *J_{gem} =* 12.2 Hz, *J_{3a,4} =* 9.2 Hz, *J_{3a,4} = J_{3a,2} =* 7.7 Hz, H-3a-pyrrolidinyl); 1.69-1.86 (m, 2H, H-4-pyrrolidinyl); 2.15 (dddd, 1H, *J_{gem} =* 12.2 Hz, *J_{3b,4} =* 8.2 Hz, *J_{3b,2} =* 7.3 Hz, *J_{3b,4} =* 4.8 Hz, H-3b-pyrrolidinyl); 2.89 (ddd, 1H, *J_{gem}* = 9.9 Hz, *J_{5a,4} =* 8.2 and 6.6 Hz, H-5a-pyrrolidinyl); 3.06 (ddd, 1H, *J_{gem}* = 9.9 Hz, *J_{5b,4} =* 7.7 and 5.2 Hz, H-5b-pyrrolidinyl); 4.01 (s, 3H, CH₃O-4'); 4.02 (s, 3H, CH₃O-2'); 4.06 (t, 1H, *J_{2,3a} = J_{2,3b} =* 7.6 Hz, H-2-pyrrolidinyl); 6.95 (s, 1H, H-5); 7.37 - 7.42 (m, 2H, H-4,5-Ph); 7.80 (m, 1H, H-6-Ph); 7.95 (bs, 1H, H-2-Ph); 8.67 (s, 1H, H-6'); 8.80 (s, 1H, H-2). HRMS (ESI) calculated for C₂₂H₂₃O₂N₆: 403.18770; found: 403.18764.

### 7-[4-(2,4-Dimethoxypyrimidin-5-yl)-7H-pyrrolo[2,3-d]pyrimidin-6-yl]-3-methyl-1,2,3,4-tetrahydroisoquinoline (33)

GP F starting from **VI** (130 mg, 0.25 mmol) and 7-bromo-3-methyl-1,2,3,4-tetrahydroisoquinoline hydrobromide (84 mg, 0.275 mmol). Deprotection by GP H (HPFC, C-18, H₂O/MeOH 5 → 100%) gave compound **33** (20 mg, 20%) as a white solid.

¹H NMR (500 MHz, DMSO-d₆): 1.14 (d, 3H, *J_{CH3,3}* = 6.3 Hz, CH₃-C₁₀H₁₂N); 2.40 (dd, 1H, *J_{gem}* = 16.4 *Hz, J_{4a,3}* = 10.4 Hz, H-4a-C₁₀H₁₂N); 2.72 (dd, 1H, *J_{gem}* = 16.4 Hz, *J_{4b,3}* = 3.7 Hz, H-4b-C₁₀H₁₂N); 2.86 (dqd, 1H, *J_{3,4a}* = 10.4 Hz, *J_{3,CH3}* = 6.3 Hz, *J_{4b,3} =* 3.8 Hz, H-3-C₁₀H₁₀N); 3.94 (s, 2H, H-1-C₁₀H₁₂N); 4.00 (s, 3H, CH₃O-4'); 4.01 (s, 3H, CH₃O-2'); 6.90 (s, 1H, H-5); 7.15 (d, 1H, *J_{5,6} =* 8.0 Hz, H-5-C₁₀H₁₂N); 7.67 (d, 1H, *J_{8,6} =* 1.9 Hz, H-8-C₁₀H₁₂N); 7.70 (dd, 1H, *J_{6,5}* = 7.9 Hz, *J_{6,8} =* 2.0 Hz, H-6-C₁₀H₁₂N); 8.66 (s, 1H, H-6'); 8.78 (s, 1H, H-2); 12.55 (bs, 1H, NH). HRMS (ESI) calculated for C₂₂H₂₃O₂N₆: 403.18770; found: 403.18765.

### 1-{4-[4-(2,4-Dimethoxypyrimidin-5-yl)-7H-pyrrolo[2,3-d]pyrimidin-6-yl]-3-fluorophenyl}-cyclopropan-1-amine (34)

GP F starting from **VI** (200 mg, 0.39 mmol) and *tert-butyl* (1-(4-bromo-3-fluorophenyl)cyclopropyl)carbamate (161 mg, 0.49 mmol). Deprotection by GP H (HPFC, C-18, H₂O/MeOH 5 → 100%) gave compound **34** (29 mg, 18%) as a white solid.

¹H NMR (500 MHz, DMSO-d₆): 1.02-1.05 (m, 4H, q-2H, r-2H); 4.01 (s, 6H, 2'-O-CH₃, 4'-O-CH₃); 6.82-6.83 (d, 1H, o-H); 7.19-7.21 (m, 1H, o-H); 7.31-7.35 (m, 1H, 5-H); 7.87-7.91 (t, 1H, m-H); 8.70 (s, 1H, 2-H); 8.84 (s, 1H, 6'-H); 12.60 (s, 1H, N₇H). HRMS (ESI) calculated for C₂₁H₂₀O₂N₆F: 407.16263; found: 407.16270.

### 3-{4-[4-(2,4-Dimethoxypyrimidin-5-yl)-7H-pyrrolo[2,3-d]pyrimidin-6-yl]phenyl}tetrahydrofuran-3-amine (35)

GP F starting from **VI** (380 mg, 0.749 mmol) and *tert*-butyl 3-(4-bromophenyl)tetrahydrofuran-3-yl)carbamate (380 mg, 1.11 mmol). Deprotection by GP H (HPFC, C-18, H₂O/MeOH 5 → 100%) gave compound **35** (50 mg, 19%) as a white solid.

¹H NMR (500 MHz, DMSO-d₆): 2.05 (dddd, 1H, *J_{gem}* = 12.2 Hz, *J_{4a,5b}* = 7.0 Hz, *J_{4a,5a}* = 3.7 Hz, *J_{4a,2a}* = 0.9 Hz, H-4a-tetrahydrofuryl); 2.24 (dt, 1H, *J_{gem} =* 12.2 Hz, *J_{4b,5b} = J_{4b,5a} =* 8.7 Hz, H-4b-tetrahydrofuryl); 3.72 (dd, 1H, *J_{gem} =* 8.3 Hz, *J_{2a,4a}* = 0.9 Hz, H-2a-tetrahydrofuryl); 3.72 (d, 1H, *J_{gem} =* 8.3 Hz, H-2b-tetrahydrofuryl); 3.94 (td, 1H, *J_{gem}* = *J_{5a,4b} = J_{4b,5b} =* 8.3 Hz, *J_{5a,4a} =* 3.7 Hz, H-5a-tetrahydrofuryl); 4.01 (s, 3H, CH₃O-4'); 4.02 (s, 3H, CH₃O-2'); 4.04 (td, 1H, *J_{gem} = J_{5b,4b}* = 8.3 Hz, *J_{5b,4a}* = 7.1 Hz, H-5b-tetrahydrofuryl); 6.97 (s, 1H, H-5); 7.60 (m, 2H, H-*m*-Ph); 7.94 (m, 2H, H-*o*-Ph); 8.68 (s, 1H, H-6'); 8.80 (s, 1H, H-2); 12.65 (bs, 1H, NH).HRMS (ESI) calculated for C₂₂H₂₂O₃N₆: 418.1753; found: 418.1759.

### 3-{4-[4-(2,4-Dimethoxypyrimidin-5-yl)-7H-pyrrolo[2,3-d]pyrimidin-6-yl]phenyl}oxetan-3-amine (36)

GP F starting from **VI** (288 mg, 0.56 mmol) and *tert-butyl* 3-(4-bromophenyl)oxetan-3-yl)carbamate (276 mg, 0.84 mmol). Deprotection by GP H (HPFC, C-18, H₂O/MeOH 5 → 100%) gave compound **36** (49 mg, 25%) as a white solid.

¹H NMR (500 MHz, DMSO-d₆): 4.01 (s, 3H, CH₃O-4'); 4.02 (s, 3H, CH₃O-2'); 4.65 - 4.75 (m, 4H, CCH₂O); 7.00 (s, 1H, H-5); 7.68 (m, 2H, H-*m*-Ph); 8.00 (m, 2H, H-*o*-Ph); 8.68 (s, 1H, H-6'); 8.81 (s, 1H, H-2); 12.68 (bs, 1H, NH). HRMS (ESI) calculated for C₂₁H₂₁O₃N₆: 405.16697; found: 405.16704.

### 4-{4-[4-(2,4-Dimethoxypyrimidin-5-yl)-7H-pyrrolo[2,3-d]pyrimidin-6-yl]phenyl}-N-methyltetrahydro-2H-pyran-4-amine (37)

GP F starting from **VI** (219 mg, 0.43 mmol) and *tert*-butyl 4-(4-bromophenyl)tetrahydro-2*H*-pyran-4-yl)(methyl)carbamate (292 mg, 0.79 mmol). Deprotection by GP H (HPFC, C-18, H₂O/MeOH 5 → 100%) gave compound **36** (32 mg, 19%) as a white solid.

¹H NMR (500 MHz, DMSO-d₆): 1.22 (m, 2H, H-3a,5a-tetrahydropyranyl); 1.80 (m, 2H, H-3b,5b-tetrahydropyranyl); 1.87 (s, 3H, *N*-CH₃); 3.55-3.58 (m, 2H, H-2a,6a-tetrahydropyranyl); 3.80-3.85 (m, 2H, H-2b,6b-tetrahydropyranyl); 4.01 (s, 3H, CH₃O-4'); 4.02 (s, 3H, CH₃O-2'); 6.96 (s, 1H, H-5); 7.52 (m, 2H, H-*m*-Ph); 7.93 (m, 2H, H-*o*-Ph); 8.67 (s, 1H, H-6'); 8.80 (s, 1H, H-2); 12.64 (vbs, 1H, NH). HRMS (ESI) calculated for C₂₄H₂₇O₃N₆: 447.21392; found: 447.21372.

### N-{4-[4-(2,4-Dimethoxypyrimidin-5-yl)-7H-pyrrolo[2,3-d]pyrimidin-6-yl]benzyl}cyclopropanamine (38)

GP F starting from **VI** (250 mg, 0.49 mmol) and *N*-(4-bromobenzyl)cyclopropanamine hydrochloride (190 mg, 0.72 mmol). Deprotection by GP H (HPFC, C-18, H₂O/MeOH 5 → 100%) gave compound **38** (26 mg, 13%) as a white solid.

¹H NMR (500.0 MHz, DMSO-*d*₆): 0.24-0.37 (m, 4H, CH₂-cyclopropyl); 2.04 (m, 1H , CH-cyclopropyl); 3.75 (s, 2H, CH₂N); 4.01 (s, 3H, CH₃O-4'); 4.02 (s, 3H, CH₃O-2'); 6.95 (s, 1H, H-5); 7.39 - 7.45 (m, 2H, CH-*m*-phenylene); 7.88-7.94 (m, 2H, CH-*o*-phenylene); 8.67 (s, 1H, H-6'); 8.80 (s, 1H, H-2); 12.63 (bs, 1H, H-7). HR MS (ESI) for C₂₂H₃₂O₂N₆ [M + H]⁺: calcd 403.1877; found 403.1873.

### 1-{4-[4-(2,4-Dimethoxypyrimidin-5-yl)-7H-pyrrolo[2,3-d]pyrimidin-6-yl]phenyl}-cyclopropan-1-amine (39)

GP F starting from **VI** (300 mg, 0.58 mmol) and *tert*-butyl (1-(4-bromophenyl)cyclopropyl)carbamate (362 mg, 1.16 mmol). Deprotection by GP H (HPFC, C-18, H₂O/MeOH 5 → 100%) gave compound **39** (97 mg, 43%) as a yellowish solid.

¹H NMR (500 MHz, DMSO-d₆): 0.98 - 1.13 (m, 4H, H-cyclopropyl); 4.01 (s, 3H, CH₃O-4'); 4.02 (s, 3H, CH₃O-2'); 6.96 (s, 1H, H-5); 7.41 (m, 2H, H-*m*-Ph); 7.91 (m, 2H, H-*o*-Ph); 8.67 (s, 1H, H-6'); 8.80 (s, 1H, H-2); 12.63 (bs, 1H, NH). HR MS (ESI) for C₂₁H₂₁O₂N₆ [M + H]⁺: calcd 389.17205; found 389.17184.

### 1-{4-[4-(2,4-Dimethoxypyrimidin-5-yl)-7H-pyrrolo[2,3-d]pyrimidin-6-yl]phenyl}-N-methylmethanammonium trifluoroacetate (40)

GP F starting from **VI** (249 mg, 0.49 mmol) and 1-(4-bromophenyl)-*N*-methylmethanamine (175 mg, 0.88 mmol). Deprotection by GP H (without neutralization step using NaHCO₃) (HPFC, C-18, H₂O/MeOH 5 → 100%) gave compound **40** (126 mg, 52%) as a yellowish powder.

¹H NMR (500.0 MHz, DMSO-*d*₆): 2.53 (s, 3H, CH₃N); 4.01 (s, 3H, CH₃O-4'); 4.02 (s, 3H, CH₃O-2'); 4.08 (bs, 2H, CH₂N); 7.06 (s, 1H, H-5); 7.52-7.57 (m, 2H, CH-*m*-phenylene); 8.01-8.07 (m, 2H, CH*o*-phenylene); 8.14 (vbs, 1H, NH); 8.68 (s, 1H, H-6'); 8.83 (s, 1H, H-2); 12.71 (bs, 1H, H-7). HR MS (ESI) for C₂₀H₂₁O₂N₆ [M + H]⁺: calcd 377.1721; found 377.1728.

### 1-{4-[4-(2,4-Dimethoxypyrimidin-5-yl)-7H-pyrrolo[2,3-d]pyrimidin-6-yl]phenyl}-N-methylpropan-1-amine (41)

GP F starting from **VI** (300 mg, 0.58 mmol) and 1-(4-bromophenyl)-*N*-methylpropanamine (175 mg, 0.76 mmol). Deprotection by GP H (HPFC, C-18, H₂O/MeOH 5 → 100%) gave compound **41** (129 mg, 55%) as a yellow solid.

¹H NMR (500.0 MHz, DMSO-d₆): 0.76 (t, 3H, *J*_{vic} = 7.4, CH₃CH₂CH); 1.52 (dp, 1H, *J_{gem}* = 13.4, *J*_{vic} = 7.4, CH₃CHₐH_{b}CH); 1.68 (dqd, 1H, *J_{gem}* = 13.4, *J*_{vic} = 7.4, 6.0, CH₃CHₐH_{b}CH); 2.11 (s, 3H, CH₃N); 3.35 (dd, 1H, *J*_{vic} = 7.4, 6.0, CH₃CH₂CH); 4.01 (s, 3H, CH₃O-4'); 4.02 (s, 3H, CH₃O-2'); 6.95 (s, 1H, H-5); 7.37-7.40 (m, 2H, CH-*m*-phenylene); 7.91-7.94 (m, 2H, CH-*o*-phenylene); 8.68 (s, 1H, H-6'); 8.80 (s, 1H, H-2); 12.61 (bs, 1H, H-7). HR MS (ESI) for C₂₀H₂₄O₂N₆ [M + H]⁺: calcd 405.2014; found 405.2020.

### 2-Amino-2-(4-(4-(2,4-dimethoxypyrimidin-5-yl)-7H-pyrrolo[2,3-d]pyrimidin-6-yl)phenyl)ethan-1-ol (42)

GP F starting from **VI** (300 mg, 0.58 mmol) and 2-amino-2-(4-bromophenyl)ethan-1-ol (188 mg, 0.87 mmol). Deprotection by GP H (HPFC, C-18, H₂O/MeOH 5 → 100%) gave compound **42** (92 mg, 40%) as a yellow solid.

¹H NMR (500.2 MHz, DMSO-d₆): 3.33 (dd, 1H, *J_{gem}* = 10.4, *J*_{vic} = 7.8, CHₐH_{b}OH); 3.48 (dd, 1H, *J_{gem}* = 10.4, *J*_{vic} = 4.9, CHₐH_{b}OH); 3.90 (dd, 1H, *J*_{vic} = 7.8, 4.9, CHNH₂); 4.01 (s, 3H, CH₃O-4'); 4.02 (s, 3H, CH₃O-2'); 4.79 (bs, 1H, OH); 6.94 (s, 1H, H-5); 7.44-7.48 (m, 2H, CH-*m*-phenylene); 7.89-7.92 (m, 2H, CH-*o*-phenylene); 8.67 (s, 1H, H-6'); 8.80 (s, 1H, H-2). HR MS (ESI) for C₂₀H₂₁O₃N₆ [M + H]⁺: calcd 393.1669; found 393.1667.

### 4-(2,4-Dimethoxypyrimidin-5-yl)-6-[(2-methyl-4-pyrrolidin-2-yl)phenyl]-7H-pyrrolo|[2,3-d]pyrimidine (43)

GP F starting from **VI** (300 mg, 0.58 mmol) and 2-(4-bromo-3-methylphenyl)pyrrolidine (209 mg, 0.87 mmol). Deprotection by GP H (HPFC, C-18, H₂O/MeOH 5 → 100%) gave compound **43** (37 mg, 15%) as a yellow solid.

¹H NMR (500.0 MHz, DMSO-*d*₆): 1.49 (ddt, 1H, *J_{gem}* = 12.1, *J_{3b,4}* = 9.2, 7.6, *J_{3b,2}* = 7.6, H-3b-pyrrolidine); 1.68 - 1.83 (m, 2H, H-4-pyrrolidine); 2.13 (dddd, 1H, *J_{gem}* = 12.1, *J_{3a,4}* = 8.3, 4.9, *J*_{3a,2} = 7.2, H-3a-pyrrolidine); 2.46 (s, 3H, CH₃); 2.89 (ddd, 1H, *J_{gem}* = 9.9, *J*_{5b,4} = 8.2, 6.6, H-5b-pyrrolidine); 3.03 (ddd, 1H, *J_{gem}* = 9.9, *J*_{5a,4} = 7.6, 5.3, H-5a-pyrrolidine); 4.00 (s, 3H, CH₃O-4'); 4.01 (s, 3H, CH₃O-2'); 4.04 (dd, 1H, *J*_{2,3} = 7.6, 7.2, H-2-pyrrolidine); 6.57 (s, 1H, H-5); 7.30 (dd, 1H, *J*_{5,6} = 7.9, *J*_{5,3} = 1.9, H-5-C₆H₃Me); 7.35 (d, 1H, *J_{3,5}* = 1.9, H-3-C₆H₃Me); 7.50 (d, 1H, *J_{6,5}* = 7.9, H-6-C₆H₃Me); 8.70 (s, 1H, H-6'); 8.81 (s, 1H, H-2); 12.36 (bs, 1H, H-7). HR MS (ESI) for C₂₃H₂₅O₂N₆ [M + H]⁺: calcd 417.2035; found 417.2031.

### 1-{4-[4-(2,4-Dimethoxypyrimidin-5-yl)-7H-pyrrolo[2,3-d]pyrimidin-6-yl]-3-fluorophenyl}-N-methylmethanamine (44)

GP F starting from **VI** (300 mg, 0.58 mmol) and [(4-bromo-3-fluorophenyl)methyl](methyl)amine (190 mg, 0.87 mmol). Deprotection by GP H (HPFC, C-18, H₂O/MeOH 5 → 100% followed by SiO₂, DCM/MeOH 0 → 10%) gave compound **44** (68 mg, 30%) as a yellow solid.

¹H NMR (500.2 MHz, DMSO-*d*₆): 2.27 (s, 3H, CH₃N); 3.70 (s, 2H, CH₂N); 4.00 (s, 3H, CH₃O-4'); 4.02 (s, 3H, CH₃O-2'); 6.86 (d, 1H, *J*_{H,F} = 2.9, H-5); 7.29 (dd, 1H, *J_{5,6}* = 8.1, *J_{5,3}* = 1.6, H-5-C₆H₃F); 7.33 (dd, 1H, *J*_{H,F} = 12.7, *J*_{3,5} = 1.6, H-3-C₆H₃F); 7.93 (t, 1H, *J*_{H,F} = *J_{6,5}* = 8.1, H-6-C₆H₃F); 8.70 (s, 1H, H-6'); 8.85 (s, 1H, H-2). ¹⁹F NMR (470.4 MHz, DMSO-*d*₆): -109.93 (ddd, 1H, *J*_{F,H} = 12.7, 8.1, 2.9, F). HR MS (ESI) for C₂₀H₂₀O₂N₆F [M + H]⁺: calcd 395.1626; found 395.1622.

### {5-[4-(2,4-Dimethoxypyrimidin-5-yl)-7H-pyrrolo[2,3-d]pyrimidin-6-yl]-6-methylpyridin-2-yl}methanamine (45)

GP F starting from **VI** (300 mg, 0.58 mmol) and *tert*-butyl ((5-bromo-6-methylpyridin-2-yl)methyl)carbamate (295 mg, 0.98 mmol). Deprotection by GP H (HPFC, C-18, H₂O/MeOH 5 → 100% followed by SiO₂, DCM/MeOH 0 → 10%) gave compound **45** (26 mg, 11%) as a white solid. ¹H NMR (500.2 MHz, DMSO-d₆): 2.66 (s, 3H, CH₃); 3.85 (s, 2H, CH₂N); 4.00 (s, 3H, CH₃O-4'); 4.01 (s, 3H, CH₃O-2'); 6.70 (s, 1H, H-5); 7.42 (d, 1H, *J_{5a,4}* = 8.0, H-5-py); 7.94 (d, 1H, *J_{4,5}* = 8.0, H-4-py); 8.71 (s, 1H, H-6'); 8.84 (s, 1H, H-2). HR MS (ESI) for C₁₉H₂₀O₂N₇ [M + H]⁺: calcd 378.1673; found 378.1671.

### (R)-1-{4- [4-(2,4-Dimethoxypyrimidin-5-yl)-7H-pyrrolo[2,3-d]pyrimidin-6-yl]phenyl}-N-ethylethan-1-amine (46)

GP F starting from **VI** (350 mg, 0.68 mmol) and (*R*)-1-(4-bromophenyl)-*N*-ethylethan-1-amine hydrochloride (270 mg, 1.18 mmol). Deprotection by GP H (HPFC, SiO₂, DCM/MeOH 0 → 10%) gave compound **46** (24 mg, 26%) as a white solid.

¹H NMR (500.0 MHz, DMSO-d₆): 1.05 (t, 3H, *J =* 7.1, N-CH₂-C*H₃*); 1.31 (s, 1H, N*H*); 1.37 (d, 3H, *J* = 6.7, N-CH-C*H₃*); 2.60 (m, 1H, C*H*-NH); 3.67 (q, 2H, *J =* 6.7, C*H₂*-NH₂); 4.09 (s, 3H, O-CH₃); 4.10 (s, 3H, O-CH₃); 7.03 (s, 1H, H-5); 7.49 (m, 2H, H-o-Ph); 8.00 (m, 2H, , H-*m*-Ph); 8.76 (s, 1H, H-6'); 8.88 (s, 1H, H-2); 12.71 (br, 1H, NH). HR MS (ESI) for C₂₂H₂₅N₆O₂⁺ ([M + H]⁺): calcd 405.20335; found 405.20296).

### (S)-1-{4-[4-(2,4-Dimethoxypyrimidin-5-yl)-7H-pyrrolo[2,3-d]pyrimidin-6-yl]phenyl}-N-ethylethan-1-amine (47)

GP F starting from **VI** (350 mg, 0.68 mmol) and (*S*)-1-(4-bromophenyl)-*N*-ethylethan-1-amine hydrochloride (218 mg, 0.82 mmol). Deprotection by GP H (HPFC, SiO₂, DCM/MeOH 0 → 10%) gave compound **47** (30 mg, 30%) as a white solid.

¹H NMR (500.0 MHz, DMSO-d₆): 1.05 (t, 3H, *J =* 7.1, N-CH₂-C*H₃*); 1.31 (s, 1H, N*H*); 1.37 (d, 3H, *J* = 6.7, N-CH-C*H₃*); 2.60 (m, 1H, CH-NH); 3.67 (q, 2H, *J =* 6.7, C*H₂*-NH₂); 4.09 (s, 3H, O-CH₃); 4.10 (s, 3H, O-CH₃); 7.03 (s, 1H, H-5); 7.49 (m, 2H, H-*o*-Ph); 8.00 (m, 2H, , H-*m*-Ph); 8.76 (s, 1H, H-6'); 8.88 (s, 1H, H-2); 12.71 (br, 1H, N*H*). HR MS (ESI) for C₂₂H₂₅N₆O₂⁺ ([M + H]⁺): calcd 405.20335; found 405.2038).

### 6-[4-(2,4-Dimethoxypyrimidin-5-yl)-7H-pyrrolo[2,3-d]pyrimidin-6-yl]-1,2,3,4-tetrahydronaphthalen-1-amine (48)

GP F starting from **VI** (300 mg, 0.58 mmol) and 7-bromo-1,2,3,4-tetrahydronaphthalen-1-amine hydrochloride (231 mg, 0.88 mmol). Deprotection by GP H (HPFC, SiO₂, DCM/MeOH 0 → 10%) gave compound **48** (152 mg, 65%) as a white solid.

¹H NMR (500 MHz, DMSO-d₆): 1.57 (m, 1H, H-2"a); 1.67 (m, 1H, H-3"a); 1.85-1.98 (m, 2H, H-2''b,3''b); 2.65-2.80 (m, 2H, H-4"); 3.87 (m, 1H, H-1"); 4.01 (s, 3H, CH₃O-4'); 4.02 (s, 3H, CH₃O-2'); 6.91 (s, 1H, H-5); 7.14 (d, 1H, *J_{5",6"}* = 8.0 Hz, H-5"); 7.70 (dd, 1H, *J_{6",5"} = 8.0 Hz, J_{6",8"}* = 1.9 Hz, H-6-"); 8.05 (d, 1H, *J_{8",6"} =* 1.8 Hz, H-8"); 7.66 (s, 1H, H-6'); 8.79 (s, 1H, H-2). HR MS (ESI) for C₂₂H₂₃N₆O₂⁺ ([M + H]⁺): calcd 403.18770; found 403.18725.

### 1-{4-[4-(2,4-Dimethoxypyrimidin-5-yl)-7H-pyrrolo[2,3-d]pyrimidin-6-yl]phenyl}cyclobutan-1-amine (49)

GP F starting from **VI** (400 mg, 0.78 mmol) and 1-(4-bromophenyl)cyclobutan-1-amine (264 mg, 1.17 mmol). Deprotection by GP H (HPFC, SiO₂, DCM/MeOH 0 → 10%) gave compound **49** (42 mg, 13%) as a white solid.

¹H NMR (400 MHz, DMSO-*d*₆) δ 8.82 (s, 1H, H-2), 8.69 (s, 1H, H-6'), 8.02-7.98 (m, 2H, H-m-Ph), 7.61-7.56 (m, 2H, H-o-Ph), 7.01 (s, 1H, H-5), 4.03 (s, 3H, O-CH₃), 4.02 (s, 3H, O-CH₃), 2.30 (tdd, *J =* 11.9, 6.4, 4.3 Hz, 4H, H-2b,3b,4b,5b-C₅H₈), 2.07 (dddd, *J* = 10.9, 9.0, 7.2, 4.4 Hz, 2H, H-2a,5a-C₅H₈), 1.74 (dtt, *J* = 11.1, 9.0, 6.7 Hz, 2H, H-3a,4a-C₅H₈). HR MS (ESI) for C₂₃H₂₅O₂N₆ [M + H]⁺: calcd 417.20335; found 417.20344.

### 1-{4-[4-(2,4-Dimethoxypyrimidin-5-yl)-7H-pyrrolo[2,3-d]pyrimidin-6-yl]phenyl}cyclopentan-1-amine (50)

GP F starting from **VI** (400 mg, 0.78 mmol) and 1-(4-bromophenyl)cyclopentan-1-amine hydrochloride (259 mg, 0.94 mmol). Deprotection by GP H (HPFC, SiO₂, DCM/MeOH 0 → 10%) gave compound **50** (20 mg, 6%) as a white solid.

¹H NMR (400.0 MHz, DMSO-d₆): 1.43 (s, 6H, C(C*H₃*)₂); 4.02 (s, 3H, O-CH₃); 4.03 (s, 3H, O-CH₃); 6.96 (s, 1H, H-5); 7.64 (m, 2H H-*m*-Ph); 7.92 (m, 2H, H-o-Ph); 8.68 (s, 1H, H-6'); 8.81 (s, 1H, H-2); 12.65 (bs, 1H, NH). HR MS (ESI) for C₂₁H₂₃O₂N₆ [M + H]⁺: calcd 391.18770; found 391.18746.

### 2-{4-[4-(2,4-Dimethoxypyrimidin-5-yl)-7H-pyrrolo[2,3-d]pyrimidin-6-yl]phenyl}propan-2-amine (51)

GP F starting from **VI** (255 mg, 0.50 mmol) and *tert*-butyl (2-(4-bromophenyl)propan-2-yl)carbamate (172 mg, 0.55 mmol). Deprotection by GP H (HPFC, SiO₂, DCM/MeOH 0 → 10%) gave compound **51** (23 mg, 12%) as a yellowish solid.

¹H NMR (400.0 MHz, DMSO-d₆): 1.43 (s, 6H, C(C*H₃*)₂); 4.02 (s, 3H, O-CH₃); 4.03 (s, 3H, O-CH₃); 6.96 (s, 1H, H-5); 7.64 (m, 2H H-*m*-Ph); 7.92 (m, 2H, H-*o*-Ph); 8.68 (s, 1H, H-6'); 8.81 (s, 1H, H-2); 12.65 (bs, 1H, NH). HR MS (ESI) for C₂₁H₂₃O₂N₆ [M + H]⁺: calcd 391.18770; found 391.18746.

### 1-{4-[4-(2,4-Dimethoxypyrimidin-5-yl)-7H-pyrrolo[2,3-d]pyrimidin-6-yl]phenyl}-N-ethylcyclopropan-1-amine (52)

GP F starting from **VI** (700 mg, 1.36 mmol) and *tert*-butyl (1-(4-bromophenyl)cyclopropyl)(ethyl)carbamate (696 mg, 2.04 mmol). Deprotection by GP H (HPFC, C-18, H₂O/MeOH 5 → 100%) gave compound **52** (278 mg, 49%) as a white solid.

¹H NMR (500 MHz, DMSO-d₆): 0.88-0.98 (m, 4H, CH₂-cyclopropyl); 0.98 (t, 3H, *J_{CH3,CH2} =* 7.1 Hz, CH₃CH₂); 2.46 (q, 2H, *J_{CH2,CH3} =* 7.1 Hz, CH₃CH₂); 4.01 (s, 3H, CH₃O-4'); 4.02 (s, 3H, CH₃O-2'); 6.93 (s, 1H, H-5); 7.39 (m, 2H, H-*m*-Ph); 7.89 (m, 2H, H-*o*-Ph); 8.67 (s, 1H, H-6'); 8.79 (s, 1H, H-2); 12.62 (bs, 1H, NH). HR MS (ESI) for C₂₃H₂₅N₆O₂⁺ ([M + H]⁺): calcd 417.2039; found 417.2031; for C₂₃H₂₄N₆O₂Na⁺ ([M + Na]⁺): calcd 439.1858; found: 439.1850.

### 1-{4-[4-(2,4-Dimethoxypyrimidin-5-yl)-7H-pyrrolo[2,3-d]pyrimidin-6-yl]phenyl}-N-isopropylcyclopropan-1-amine (53)

GP F starting from **VI** (300 mg, 0.58 mmol) and *tert*-butyl (1-(4-bromophenyl)cyclopropyl)(isopropyl)carbamate (311 mg, 0.88 mmol). Deprotection by GP H (HPFC, C-18, H₂O/MeOH 5 → 100%) gave compound **53** (110 mg, 44%) as a pale yellow solid.

¹H NMR (500 MHz, DMSO-d₆): 0.87-0.92 (m, 4H, CH₂-cyclopropyl); 0.91 (d, 6H, *J_{CH3,CH} =* 6.2 Hz, CH₃-*i*-Pr); 2.67 (sept, 1H, *J_{CH,CH3}* = 6.2 Hz, CH-*i*-Pr); 4.01 (s, 3H, CH₃O-4'); 4.02 (s, 3H, CH₃O-2'); 6.93 (s, 1H, H-5); 7.48 (m, 2H, H-*m*-Ph); 7.89 (m, 2H, H-*o*-Ph); 8.67 (s, 1H, H-6'); 8.79 (s, 1H, H-2); 12.61 (bs, 1H, NH). HR MS (ESI) for C₂₄H₂₇N₆O₂⁺ ([M + H]⁺): calcd 431.2196; found: 431.2191.

### 1-{4-[4-(2,4-Dimethoxypyrimidin-5-yl)-7H-pyrrolo[2,3-d]pyrimidin-6-yl]phenyl}-N-methylcyclopropan-1-amine (54)

GP F starting from **VI** (724 mg, 1.40 mmol) and *tert*-butyl (1-(4-bromophenyl)cyclopropyl)(methyl)carbamate (690 mg, 2.1 mmol). Deprotection by GP H (HPFC, C-18, H₂O/MeOH 5 → 100%) gave compound **54** (110 mg, 19%) as a pale yellow solid.

¹H NMR (500 MHz, CDCl₃): 0.89-0.97 (m, 4H, CH₂-cyclopropyl); 2.18 (s, 3H, CH₃NH); 4.01 (s, 3H, CH₃O-4'); 4.02 (s, 3H, CH₃O-2'); 6.93 (s, 1H, H-5); 7.38 (m, 2H, H-*m*-Ph); 7.90 (m, 2H, H-*o*-Ph); 8.67 (s, 1H, H-6'); 8.79 (s, 1H, H-2); 12.62 (bs, 1H, NH). HR-ESI-MS: *found:* 403.1874, ([M + H]⁺, calcd for C₂₂H₂₃N₆O₂⁺ 403.1883).

### N-butyl-1-{4-[4-(2,4-dimethoxypyrimidin-5-yl)-7H-pyrrolo[2,3-d]pyrimidin-6-yl]phenyl}cyclopropan-1-amine (55)

GP F starting from **VI** (693 mg, 1.35 mmol) and *tert*-butyl (1-(4-bromophenyl)cyclopropyl)(butyl)carbamate (746 mg, 2.03 mmol). Deprotection by GP H (HPFC, C-18, H₂O/MeOH 5 → 100%) gave compound **55** (130 mg, 22%) as a pale yellow solid.

¹H NMR (500 MHz, DMSO-d₆): 0.82 (t, 3H, C**H**₃CH₂CH₂CH₂); 0.88-0.97 (m, 4H, CH₂-cyclopropyl); 1.27 (m, 2H, CH₃CH₂CH₂CH₂); 1.34 (m, 2H, CH₃CH₂C**H**₂CH₂); 2.42 (m, 2H, CH₃CH₂CH₂CH₂); 4.01 (s, 3H, CH₃O-4'); 4.02 (s, 3H, CH₃O-2'); 6.93 (s, 1H, H-5); 7.39 (m, 2H, H-*m*-Ph); 7.89 (m, 2H, H-*o-*Ph); 8.67 (s, 1H, H-6'); 8.79 (s, 1H, H-2); 12.61 (bs, 1H, NH). HR-ESI-MS: *found:* 445.2345, ([M + H]⁺, calcd for C₂₅H₂₉N₆O₂⁺ 445.2352).

### Cyclopropyl{4-[4-(2,4-dimethoxypyrimidin-5-yl)-7H-pyrrolo[2,3-d]pyrimidin-6-yl]phenyl}methanamine (56)

GP F starting from **VI** (1.4 g, 2.73 mmol) and *tert*-butyl {(4-bromophenyl)(cyclopropyl)methyl}carbamate (1.33 g, 4.08 mmol). Deprotection by GP H (HPFC, C-18, H₂O/MeOH 5 → 100%) gave compound **56** (613 mg, 56%) as a pale yellow solid.

¹H NMR (500 MHz, CDCl₃): 0.26-0.39 (m, 3H, CH₂-cyclopropyl); 0.46 (m, 1H, CH₂-cyclopropyl); 0.98 (m, 1H, CH-cyclopropyl); 3.21 (d, 1H, J_{CH,CH} = 8.0 Hz, CHNH₂); 4.01 (s, 3H, CH₃O-4'); 4.02 (s, 3H, CH₃O-2'); 6.94 (s, 1H, H-5); 7.50 (m, 2H, H-*m*-Ph); 7.90 (m, 2H, H-*o*-Ph); 8.68 (s, 1H, H-6'); 8.80 (s, 1H, H-2). HR MS (ESI) for C₂₂H₂₃N₆O₂⁺ ([M + H]⁺): calcd for 403.1883; found 403.1876.

### Cyclopentyl{4-[4-(2,4-dimethoxypyrimidin-5-yl)-7H-pyrrolo[2,3-d]pyrimidin-6-yl]phenyl}methanamine (57)

GP F starting from **VI** (305 mg, 0.59 mmol) and *tert*-butyl ((4-bromophenyl)(cyclopentyl)methyl)carbamate (316 mg, 0.89 mmol). Deprotection by GP H (HPFC, C-18, H₂O/MeOH 5 → 100%) gave compound **57** (81 mg, 32%) as a pale yellow solid.

¹H NMR (500 MHz, DMSO-d₆): 1.11 and 1.24 (2×m, 2×1H, H-2"/5"); 1.31 - 1.65 (m, 5H, H-3''/4'', H-2"/5"); 1.78 (m, 1H, H-2''/5''); 1.99 (dpent, 1H, *J_{CH,CH} =* 8.5 Hz, *J_{CH,CH2}* = 8.2 Hz, H-1"); 3.55 (d, 1H, *J_{CH,CH} =* 8.5 Hz, CHNH₂); 4.01 (s, 3H, CH₃O-4'); 4.02 (s, 3H, CH₃O-2'); 6.93 (s, 1H, H-5); 7.42 (m, 2H, H-*m*-Ph); 7.89 (m, 2H, H-*o*-Ph); 8.687 (s, 1H, H-6'); 8.79 (s, 1H, H-2). HR MS (ESI) for C₂₄H₂₇N₆O₂⁺ ([M + H]⁺): calcd 431.2196; found 431.2190; for C₂₄H₂₆N₆O₂Na⁺ ([M + Na]⁺): calcd 453.2015; found 453.2008.

### Cyclohexyl{4-[4-(2,4-dimethoxypyrimidin-5-yl)-7H-pyrrolo[2,3-d]pyrimidin-6-yl]phenyl}methanamine (58)

GP F starting from **VI** (390 mg, 0.76 mmol) and *tert*-butyl ((4-bromophenyl)(cyclohexyl)methyl)carbamate (420 mg, 1.14 mmol). Deprotection by GP H (HPFC, C-18, H₂O/MeOH 5 → 100%) gave compound **58** (132 mg, 39%) as a pale yellow solid.

¹HNMR (500 MHz, CDCl₃): 0.77-1.25 (m, 5H, CH₂-cyclohexyl); 1.31-1.47 (m, 2H, CH₂-cyclohexyl, CH-cyclohexyl); 1.51-1.65 (m, 2H, CH2-cyclohexyl); 1.69 and 1.87 (2×m, 2×1H, CH₂-cyclohexyl); 3.55 (d, 1H, J_{CH,CH} = 6.5 Hz, CHNH₂); 4.01 (s, 3H, CH₃O-4'); 4.02 (s, 3H, CH₃O-2'); 6.94 (s, 1H, H-5); 7.37 (m, 2H, H-*m*-Ph); 7.89 (m, 2H, H-*o*-Ph); 8.67 (s, 1H, H-6'); 8.80 (s, 1H, H-2). HR MS (ESI) for C₂₅H₂₉N₆O₂⁺ ([M + H]⁺): calcd 445.2352; found: 445.2344.

### 2-Cyclobutyl-1-{4-[4-(2,4-dimethoxypyrimidin-5-yl)-7H-pyrrolo[2,3-d]pyrimidin-6-yl]phenyl}ethan-1-amine (59)

GP F starting from **VI** (380 mg, 0.74 mmol) and *tert*-butyl (1-(4-bromophenyl)-2-cyclobutylethyl)carbamate (393 mg, 1.11 mmol). Deprotection by GP H (HPFC, C-18, H₂O/MeOH 5 → 100%) gave compound **59** (73 mg, 23%) as a pale yellow solid.

¹HNMR (500 MHz, DMSO-d₆): 1.51 and 1.16 (2×m, 2×1H, CH₂-2,4-cyclobutyl); 1.66 - 1.81 (m, 4H, CH₂-cyclobutyl, CH₂-3-cyclobutyl); 1.85 and 1.99 (2×m, 2×1H, CH₂-2,4-cyclobutyl); 2.19 (sept, 1H, *J_{CH,CH2} =* 7.8 Hz, CH-1-cyclobutyl); 3.80 (t, 1H, *J_{CH,CH2} =* 7.0 Hz, CHNH₂); 4.01 (s, 3H, CH₃O-4'); 4.02 (s, 3H, CH₃O-2'); 6.96 (s, 1H, H-5); 7.43 (m, 2H, H-*m*-Ph); 7.92 (m, 2H, H-*o*-Ph); 8.68 (s, 1H, H-6'); 8.80 (s, 1H, H-2); 12.63 (bs, 1H, NH). HR MS (ESI) for C₂₄H₂₇N₆O₂⁺ ([M + H]⁺): calcd 431.2196; found: 431.21869; for C₂₄H₂₆N₆O₂Na⁺ ([M + Na]⁺): calcd 453.2015; found: 453.20082.

### 1-{4-[4-(2,4-Dimethoxypyrimidin-5-yl)-7H-pyrrolo[2,3-d]pyrimidin-6-yl]phenyl}-2,2,2-trifluorethan-1-amine (60)

GP F starting from **VI** (300 mg, 0.58 mmol) and *tert*-butyl (1-(4-bromophenyl)-2,2,2-trifluoroethyl)carbamate (310 mg, 0.88 mmol). Deprotection by GP H (HPFC, SiO₂, DCM/MeOH 0 → 10% followed by C-18, H₂O/MeOH 5 → 100%) gave compound **60** (58 mg, 23%) as a white solid.

¹HNMR (500 MHz, DMSO-d₆): 4.01 (s, 3H, CH₃O-4'); 4.02 (s, 3H, CH₃O-2'); 4.55 (bm, 1H, CHNH₂); 7.03 (d, 1H, *J_{CH,NH} =* 1.6 Hz, H-5); 7.60 (m, 2H, H-m-Ph); 7.99 (m, 2H, H-o-Ph); 8.68 (s, 1H, H-6'); 8.82 (s, 1H, H-2); 12.69 (bs, 1H, NH). ¹⁹F NMR (470.4 MHz, DMSO-d₆): -74.70 (d, 3F, *J_{F,H} =* 8.3 Hz, CF₃). HR MS (ESI) for C₂₀H₁₈F₃N₆O₂⁺ ([M + H]⁺): calcd^{:} 431.1443; found 431.14378; for C₂₀H₁₇F₃N₆O₂Na⁺ ([M + Na]⁺): calcd for 453.1263; found: 453.12558.

### 4-[4-(4-Chloro-7-{[2-(trimethylsilyl)ethoxy]methyl}-7H-pyrrolo[2,3-d]pyrimidin-6-yl)phenyl]tetrahydro-2H-pyran-4-amine (VIIIb)

GP G starting from **VII** (519 mg, 1.26 mmol) and 4-(4-bromophenyl)tetrahydro-2*H*-pyran-4-amine (230 mg, 0.9 mmol), (HPFC, SiO₂, DCM/MeOH 0 → 5% followed by C-18, H₂O/MeOH 5 → 100%) gave compound **VIIIb** (140 mg, 20%) as a yellowish oil.

¹H NMR (400 MHz, Chloroform-*d*) δ -0.03 (s, 9H, 3x -CH₃); 1.01-0.94 (m, 2H, H-5'); 1.58-1.50 (m, 2H, H-2a,6a-C₅H₈O); 1.73-1.66 (m, 2H, H-NH₂); 2.24 (ddd, *J* = 13.7, 10.8, 4.6 Hz, 2H, H-2b,6b-C₅H₈O); 3.78-3.72 (m, 2H, H-4'); 3.88-3.81 (m, 2H, H-3a,5a-C₅H₈O); 3.96 (ddd, *J* = 11.5, 10.6, 2.4 Hz, 2H, H-3b,5b-C₅H₈O); 5.64 (s, 2H, H-2'); 6.70 (s, 1H, H-5); 7.68-7.54 (m, 2H, H-*o*-Ph); 7.86-7.70 (m, 2H, H-*m*-Ph); 8.67 (s, 1H, H-2). HRMS (ESI) for C₂₃H₃₂N₄O₂ClSi⁺ (M+H)⁺: calcd 459.19776; found 459.19804.

### 4-{4-[4-(2,6-Dimethoxypyridin-3-yl)-7H-pyrrolo[2,3-d]pyrimidin-6-yl]phenyl}tetrahydro-2H-pyran-4-amine (61)

GP C starting from **VIIIb** (169 mg, 0.37 mmol) and 2,6-dimethoxypyridin-3-yl boronic acid (88 mg, 0.48 mmol) in DMF/H₂O mixture (3/1; 4.9 mL). Deprotection by GP H (HPFC, SiO₂, DCM/MeOH 0 → 10% followed by C-18, H₂O/MeOH 5 → 100%) gave compound **61** (55 mg, 35%) as a pale yellow solid.

¹H NMR (500 MHz, CDCl₃): 1.73 (bd, 2H, *J_{gem} =* 13.1 Hz, H-3a,5a-C₅H₈O); 2.26 (bddd, 2H, *J_{gem} =* 13.1 Hz, *J_{3b,2b}* = 10.7 Hz, *J_{3b,2a}* = 4.2 Hz, H-3b,5b-C₅H₈O); 3.84 (dm, 2H, *J_{gem} =* 11.5 Hz, H-2a,6a-C₅H₈O); 3.97 (tm, 2H, *J_{gem} = J_{2b,3b} =* 11.1 Hz, H-2b,6b-C₅H₈O); 4.04 (s, 3H, CH₃O-6'); 4.06 (s, 3H, CH₃O-2'); 6.53 (d, 1H, *J_{5',4'} =* 8.1 Hz, H-5'); 6.83 (s, 1H, H-5); 7.65 (m, 2H, H-*m*-Ph); 7.86 (m, 2H, H-*o*-Ph); 8.07 (d, 1H, *J_{4',5'} =* 8.1 Hz, H-4'); 8.97 (s, 1H, H-2); 12.37 (bs, 1H, NH). HRMS (ESI) for C₂₄H₂₆N₅O₃⁺ (M+H)⁺: calcd 432.20302; found 432.20273.

### 4-14-[4-(2-Ethoxypyridin-3-yl)-7H-pyrrolo[2,3-d]pyrimidin-6-yl]phenyl}tetrahydro-2H-pyran-4-amine (62)

GP C starting from **VIIIb** (158 mg, 0.34 mmol) and 2-ethoxypyridin-3-yl boronic acid (74 mg, 0.45 mmol) in DMF/H₂O mixture (3/1; 4.9 mL). Deprotection by GP H (HPFC, SiO₂, DCM/MeOH 0 → 10% followed by C-18, H₂O/MeOH 5 → 100%) gave compound **62** (62 mg, 44%) as a pale yellow solid.

¹H NMR (500 MHz, CD₃OD): 1.28 (t, 3H, *J_{CH3,CH2} =* 7.1 Hz, C**H**₃CH₂O); 1.92 (ddd, 2H, *J_{gem} =* 13.5 Hz, *J_{3a,2a}* = 6.7 Hz, *J_{3a,2b}* = 3.2 Hz, H-3a,5a-C₅H₈O); 2.37 (ddd, 2H, *J_{gem}* = 13.5 Hz, *J_{3b,2b}* = 7.7 Hz, *J_{3b,2a}* = 3.4 Hz, H-3b,5b-C₅H₈O); 3.65 (ddd, 2H, *J_{gem}* = 11.8 Hz, *J_{2a,3a}* = 6.7 Hz, *J_{2a,3b}* = 3.4 Hz, H-2a,6a-C₅H₈O); 3.93 (ddd, 2H, *J_{gem}* = 11.8 Hz, *J_{2b,3b}* = 7.7 Hz, *J_{2b,3a}* = 3.2 Hz, H-2b,6b-C₅H₈O); 4.46 (q, 2H, *J_{CH2,CH3} =* 7.1 Hz, CH₃C**H**₂O); 6.86 (s, 1H, H-5); 7.14 (dd, 1H, *J_{5',4'} =* 7.3 Hz, *J_{5',6'} =* 5.0 Hz, H-5'); 7.67 (m, 2H, H-*m*-Ph); 7.91 (m, 2H, H-*o*-Ph); 7.98 (dd, 1H, *J_{4',5'} =* 7.3 Hz, *J_{4',6'}* = 2.0 Hz, H-4'); 8.30 (dd, 1H, *J_{6',5'}* = 5.0 *Hz, J_{6',4'} =* 2.0 Hz, H-6'); 8.77 (s, 1H, H-2). HRMS (ESI) for C₂₄H₂₆N₅O₂⁺ (M+H)⁺: calcd 416.20810; found 416.20820.

### 4-14-[4-(4-(Methoxy)pyrimidin-5-yl)-7H-pyrrolo[2,3-d]pyrimidin-6-yl]phenyl}tetrahydro-2H-pyran-4-amine (63)

GP C starting from **VIIIb** (185 mg, 0.40 mmol) and 4-methoxypyrimidin-5-yl boronic acid (81 mg, 0.52 mmol) in DMF/H₂O mixture (3/1; 4.9 mL). Deprotection by GP H (HPFC, SiO₂, DCM/MeOH 0 → 10% followed by C-18, H₂O/MeOH 5 → 100%) gave compound **63** (60 mg, 37%) as a pale yellow solid.

¹H NMR (500 MHz, CD₃OD): 2.12 (bddd, 2H, *J_{gem}* = 13.7 Hz, *J_{3a,2a}* = 9.1 Hz *J_{3a,2b}* = 3.5 Hz H-3a,5a-C₅H₈O); 2.56 (bd, 2H, *J_{gem} =* 13.7 Hz, H-3b,5b-C₅H₈O); 3.57 (bddd, 2H, *J_{gem} =* 12.0 Hz, *J_{2a,3a}* = 9.1 Hz *J_{2a,3b}* = 2.4 Hz, H-2a,6a-C₅H₈O); 3.95 (bdt, 2H, *J_{gem}* = 12.0 Hz, *J_{2b,3b}* = *J_{2b,3a}* = 4.5 Hz, H-2b,6b-C₅H₈O); 4.12 (s, 3H, CH₃O); 6.98 (s, 1H, H-5); 7.74 (m, 2H, H-*m*-Ph); 8.04 (m, 2H, H-*o*-Ph); 8.84 (m, 2H, H-2,6'); 8.93 (bs, 1H, H-2)'. HRMS (ESI) for C₂₂H₂₃N₆O₂⁺ (M+H)⁺: calcd 403.18770; found 403.18764.

### 4-{4-[4-(5-Fluoro-2-methoxypyridin-3-yl)-7H-pyrrolo[2,3-d]pyrimidin-6-yl]phenyl}tetrahydro-2H-pyran-4-amine (64)

GP C starting from **VIIIb** (141 mg, 0.31 mmol) and 5-fluoro-2-methoxypyridin-3-yl boronic acid (68 mg, 0.40 mmol) in DMF/H₂O mixture (3/1; 4.9 mL). Deprotection by GP H (HPFC, SiO₂, DCM/MeOH 0 → 10% followed by C-18, H₂O/MeOH 5 → 100%) gave compound **64** (100 mg, 78%) as a pale yellow solid.

¹H NMR (500 MHz, CD₃OD): 2.18 (ddd, 2H, *J_{gem}* = 13.9 Hz, *J_{3a,2a}* = 9.8 Hz, *J_{3a,2b}* = 4.0 Hz, H-3a,5a-C₅H₈O); 2.63 (bdm, 2H, *J_{gem}* = 13.9 Hz, H-3b,5b-C₅H₈O); 3.54 (ddd, 2H, *J_{gem}* = 12.4 Hz, *J_{2a,3a}* = 9.8 Hz, *J_{2a,3b}* = 1.9 Hz, H-2a,6a-C₅H₈O); 3.95 (dt, 2H, *J_{gem}* = 12.4 Hz, *J_{2b,3b}* = *J_{2b,3a}* = 4.2 Hz, H-2b,6b-C₅H₈O); 3.98 (s, 3H, CH₃O); 6.95 (s, 1H, H-5); 7.76 (m, 2H, H-*m*-Ph); 7.88 (dd, 1H, *J_{4',F} =* 8.1 Hz, *J_{4',6'} =* 3.0 Hz, H-4'); 8.05 (m, 2H, H-o-Ph); 8.24 (d, 1H, *J_{6',4'} =* 3.0 Hz, H-6'); 8.82 (s, 1H, H-2). ¹⁹ F NMR (470.4 MHz, CD₃OD): -136.79 (d, 1F, *J_{F,4'} =* 8.1 Hz, F-5'). HRMS (ESI) for C₂₃H₂₃FN₅O₂⁺ (M+H)⁺: calcd 420.18303; found 420.18292.

### 4-{4-[4-(6-Fluoro-2-methoxypyridin-3-yl)-7H-pyrrolo[2,3-d]pyrimidin-6-yl]phenyl}tetrahydro-2H-pyran-4-amine (65)

GP C starting from **VIIIb** (245 mg, 0.53 mmol) and 6-fluoro-2-methoxypyridin-3-yl boronic acid (117 mg, 0.69 mmol) in DMF/H₂O mixture (3/1; 4.9 mL). Deprotection by GP H (HPFC, SiO₂, DCM/MeOH 0 → 10% followed by C-18, H₂O/MeOH 5 → 100%) gave compound **65** (57 mg, 25%) as a pale yellow solid.

¹H NMR (500 MHz, DMSO-d₆): 1.65 (dm, 2H, *J_{gem}* = 13.4 Hz, H-3a,5a-C₅H₈O); 2.07 (ddd, 2H, *J_{gem}* = 13.4 Hz, *J_{3b,2b}* = 10.2 Hz, *J_{3b,2a}* = 4.3 Hz, H-3b,5b-C₅H₈O); 3.60 (dt, 2H, *J_{gem}* = 11.2 Hz, *J_{2a,3a}* = *J_{2a,3b}* = 4.1 Hz, H-2a,6a-C₅H₈O); 3.87 (btd, 2H, *J_{gem}* = *J_{2b,3b}* = 10.8 Hz, *J_{2b,3a}* = 2.5 Hz, H-2b,6b-C₅H₈O); 3.93 (s, 3H, CH₃O); 7.939 (dd, 1H, *J_{5',4'} =* 8.0 Hz, *J_{5',F} =* 2.7 Hz, H-5'); 6.944 (s, 1H, H-5); 7.64 (m, 2H, H-*m*-Ph); 7.96 (m, 2H, H-*o*-Ph); 8.23 (dd, 1H, *J_{4',5'} = J_{4',F} =* 8.2 Hz, H-4'); 8.82 (s, 1H, H-2); 12.67 (bs, 1H, NH). ¹⁹ F NMR (470.4 MHz, CD₃OD): -65.11 (dd, 1F, *J_{F,4'} =* 8.1 Hz, *J_{F,5'} =* 2.7 Hz, Hz F-5'). HRMS (ESI) for C₂₃H₂₃FN₅O₂⁺ (M+H)⁺: calcd 420.18303; found 420.18284.

### 4-{4-|4-(6-Chloro-2-methoxypyridin-3-yl)-7H-pyrrolo[2,3-d]pyrimidin-6-yl]phenyl}tetrahydro-2H-pyran-4-amine (66)

GP C starting from **VIIIb** (195 mg, 0.42 mmol) and 6-chloro-2-methoxypyridin-3-yl boronic acid (103 mg, 0.55 mmol) in DMF (4.2 mL). Deprotection by GP H (HPFC, SiO₂, DCM/MeOH 0 → 10% followed by C-18, H₂O/MeOH 5 → 100%) gave compound **66** (20 mg, 11%) as a pale yellow solid.

¹H NMR (500 MHz, DMSO-d₆): 1.62 (bdm, 2H, *J_{gem}* = 12.7 Hz, H-3a,5a-C₅H₈O); 2.05 (m, 2H, H-3b,5b-C₅H₈O); 3.61 (dt, 2H, *J_{gem} =* 11.3 Hz, *J_{2a,3a} = J_{2a,3b}* = 4.0 Hz, H-2a,6a-C₅H₈O); 3.87 (td, 2H, *J_{gem}* = *J_{2b,3b}* = 11.0 Hz, *J_{2b,3a}* = 2.4 Hz, H-2b,6b-C₅H₈O); 3.94 (s, 3H, CH₃O); 6.95 (s, 1H, H-5); 7.31 (d, 1H, *J_{5',4'} =* 7.7 Hz, H-5'); 7.64 (m, 2H, H-*m*-Ph); 7.96 (m, 2H, H-*o*-Ph); 8.09 (dd, 1H, *J_{4',5'} =* 7.7 Hz, H-4'); 8.82 (s, 1H, H-2); 12.69 (bs, 1H, NH). HRMS (ESI) for C₂₃H₂₃ClN₅O₂⁺ (M+H)⁺: calcd 436.15348; found 436.15369.

### 4-{4-[4-(2-Isopropoxypyridin-3-yl)-7H-pyrrolo[2,3-d]pyrimidin-6-yl]phenyl}tetrahydro-2H-pyran-4-amine (67)

GP C starting from **VIIIb** (195 mg, 0.42 mmol) and 2-*iso*propoxypyridin-3-yl boronic acid (100 mg, 0.55 mmol) in DMF (4.2 mL). Deprotection by GP H (HPFC, SiO₂, DCM/MeOH 0 → 10% followed by C-18, H₂O/MeOH 5 → 100%) gave compound **67** (22 mg, 12%) as a pale yellow solid.

¹H NMR (500 MHz, DMSO-d₆): 1.25 (d, 6H, *J_{CH3,CH}* = 6.2 Hz, (C**H**₃)₂CH); 1.65 (dm, 2H, *J_{gem}* = 13.3 Hz, H-3a,5a-C₅H₈O); 2.07 (ddd, 2H, *J_{gem} =* 13.3 Hz, *J_{3b,2b}* = 10.4 Hz, *J_{3b,2a}* = 4.2 Hz, H-3b,5b-C₅H₈O); 3.59 (dt, 2H, *J_{gem}* = 11.4 Hz, *J_{2a,3a}* = *J_{2a,3b}* = 4.0 Hz, H-2a,6a-C₅H₈O); 3.87 (btd, 2H, *J_{gem}* = *J_{2b,3b}* = 10.9 Hz, *J_{2b,3a}* = 2.4 Hz, H-2b,6b-C₅H₈O); 5.48 (sept, 1H, *J_{CH,CH3}* = 6.2 Hz, (CH₃)₂C**H**); 6.96 (s, 1H, H-5); 7.15 (dd, 1H, *J_{5',4'}* = 7.4 Hz, *J_{5',6'}* = 4.9 Hz, H-5'); 7.65 (m, 2H, H-*m*-Ph); 7.96 (m, 2H, H-*o*-Ph); 8.02 (dd, 1H, *J_{4',5'} =* 7.4 Hz, *J_{4',6'}* = 2.0 Hz, H-4'); 8.34 (dd, 1H, *J_{6',5'}* = 4.9 Hz, *J_{6',4'}* = 2.0 Hz, H-6'); 8.82 (s, 1H, H-2); 12.63 (bs, 1H, NH). HRMS (ESI) for C₂₅H₂₈N₅O₂⁺ (M+H)⁺: calcd 430.22375; found 430.22391.

### 4-{4-[4-(5-Chloro-2-methoxypyridin-3-yl)-7H-pyrrolo[2,3-d]pyrimidin-6-yl]phenyl}tetrahydro-2H-pyran-4-amine (68)

GP C starting from **VIIIb** (165 mg, 0.36 mmol) and 5-chloro-2-methoxypyridin-3-yl boronic acid (88 mg, 0.47 mmol) in DMF (3.6 mL). Deprotection by GP H (HPFC, SiO₂, DCM/MeOH 0 → 10% followed by C-18, H₂O/MeOH 5 → 100%) gave compound **68** (35 mg, 22%) as a pale yellow solid.

¹H NMR (500 MHz, DMSO-d₆): 1.66 (bdm, 2H, *J_{gem}* = 13.9 Hz, H-3a,5a-C₅H₈O); 2.08 (bddd, 2H, *J_{gem}* = 13.3 Hz, *J_{3b,2b}* = 10.1 Hz, *J_{3b,2a}* = 4.2 Hz, H-3b,5b-C₅H₈O); 3.59 (bdt, 2H, *J_{gem}* = 11.4 Hz, *J_{2a,3a}* = *J_{2a,3b}* = 4.0 Hz, H-2a,6a-C₅H₈O); 3.87 (bt, 2H, *J_{gem} = J_{2b,3b} =* 11.0 Hz, H-2b,6b-C₅H₈O); 3.94 (s, 3H, CH₃O); 6.95 (s, 1H, H-5); 7.65 (m, 2H, H-*m*-Ph); 7.98 (m, 2H, H-*o*-Ph); 8.09 (d, 1H, *J_{4',6'}* = 2.7 Hz, H-4'); 8.43 (d, 1H, *J_{6',4'}* = 2.7 Hz, H-6'); 8.84 (s, 1H, H-2); 12.73 (bs, 1H, NH). HRMS (ESI) for C₂₃H₂₃ClN₅O₂⁺ (M+H)⁺: calcd 436.15348; found 436.15323.

### 4-{4-[4-(2,4-Dimethoxypyridin-3-yl)-7H-pyrrolo[2,3-d]pyrimidin-6-yl]phenyl}tetrahydro-2H-pyran-4-amine (69)

GP C starting from **VIIIb** (150 mg, 0.33 mmol) and 2,4-dimethoxypyridin-3-yl boronic acid (78 mg, 0.43 mmol) in DMF (3.6 mL). Deprotection by GP H (HPFC, SiO₂, DCM/MeOH 0 → 10% followed by C-18, H₂O/MeOH 5 → 100%) gave compound **69** (48 mg, 34%) as a pale yellow solid.

¹H NMR (500 MHz, CD₃OD): 2.16 (ddd, 2H, *J_{gem}* = 13.8 *Hz, J_{3a,2a}* = 9.8 Hz, *J_{3a,2b}* = 4.0 Hz, H-3a,5a-C₅H₈O); 2.62 (bdm, 2H, *J_{gem}* = 13.8 Hz, H-3b,5b-C₅H₈O); 3.54 (bt, 2H, *J_{gem}* = *J_{2a,3a}* = 10.9 Hz, H-2a,6a-C₅H₈O); 3.83 (s, 3H, CH₃O-4'); 3.85 (s, 3H, CH₃O-2'); 3.94 (bdt, 2H, *J_{gem}* = 12.5 Hz, *J_{2b,3b}* = *J_{2b,3a}* = 4.2 Hz, H-2b,6b-C₅H₈O); 6.73 (s, 1H, H-5); 6.95 (d, 1H, *J_{5',6'}* = 6.1 Hz, H-5'); 7.73 (m, 2H, H-*m*-Ph); 8.01 (m, 2H, H-*o*-Ph); 8.24 (d, 1H, *J_{6',5'}* = 6.1 Hz, H-6'); 8.76 (s, 1H, H-2). HRMS (ESI) for C₂₄H₂₆N₅O₃⁺ (M+H)⁺: calcd 432.20302; found 432.20310.

### Tert-butyl {1-[4-(4-(chloro)-7-{[2-(trimethylsilyl)ethoxy]methyl}-7H-pyrrolo[2,3-d]pyrimidin-6-yl)phenyl]cyclohexyl}carbamate (VIIIa)

GP G starting from **VII** (1.0 g, 2.44 mmol) and *tert*-butyl 1-(4-bromophenyl)cyclohexylcarbamate (1.62 g, 4.6 mmol), (HPFC, SiO₂, cHex/EtOAc 0 → 90%) gave compound **VIIIa** (1.0 g, 74%) as a yellowish solid.

¹H NMR (401 MHz, Chloroform-*d*): -0.03 (s, 9H); 1.02-0.90 (m, 2H, H-5'); 1.34-1.06 (m, 2H, H-4a,4b cyclohexyl); 1.41 (s, 9H, CH₃-*t*Bu); 1.74-1.55 (m, 4H, H-2b,3b,5b,6b- cyclohexyl); 1.85-1.74 (m, 2H H-3a,5a- cyclohexyl); 2.25 (m, 2H H-2a,6a- cyclohexyl); 3.81-3.65 (m, 2H, H-4'); 4.93 (s, 1H, H-NH); 5.61 (s, 2H H-2'); 6.67 (s, 1H, H-5); 7.53 (d, *J =* 8.5 Hz, 2H, H-o-Ph); 7.74 (d, *J =* 8.5 Hz, 2H, H-*m*-Ph); 8.64 (s, 1H, H-2). HRMS (ESI) for C₂₉H₄₂N₄O₂ClSi⁺ (M+H)⁺: calcd 557.27092; found 557.27107.

### 1-{4-[4-(2,4-Dimethoxypyridin-3-yl)-7H-pyrrolo[2,3-d]pyrimidin-6-yl]phenyl}cyclohexan-1-amine (70)

GP C starting from **VIIIa** (150 mg, 0.27 mmol) and 2,4-dimethoxypyridin-3-yl boronic acid (64 mg, 0.35 mmol) in DMF (3.6 mL). Deprotection by GP H (HPFC, SiO₂, DCM/MeOH 0 → 10% followed by C-18, H₂O/MeOH 5 → 100%) gave compound **70** (24 mg, 21%) as a pale yellow solid.

¹H NMR (500 MHz, DMSO-d₆): 1.31-1.44 (m, 3H, H-4a,3a,5a-cyclohexyl); 1.49 (m, 1H, H-4b-cyclohexyl); 1.64-1.79 (m, 4H, H-2a,6a,3b,5b-cyclohexyl); 2.00-2.13 (m, 2H, H-2b,6b-cyclohexyl); 3.75 (bs, 6H, CH₃O-2',4'); 6.68 (s, 1H, H-5); 6.99 (d, 1H, *J_{5',6'} =* 6.0 Hz, H-5'); 7.63 (m, 2H, H-*m*-Ph); 7.95 (m, 2H, H-*o*-Ph); 8.24 (d, 1H, *J_{6',5'} =* 6.0 Hz, H-6'); 8.77 (s, 1H, H-2); 12.63 (bs, 1H, NH). HRMS (ESI) for C₂₅H₂₈N₅O₂⁺ (M+H)⁺: calcd 430.2165; found 430.2241.

### 4-{4-[4-(2-Methoxypyridin-3-yl)-7H-pyrrolo[2,3-d]pyrimidin-6-yl]phenyl}tetrahydro-2H-pyran-4-amine (71)

GP C starting from **VIIIb** (137 mg, 0.30 mmol) and 2-methoxypyridin-3-yl boronic acid (59 mg, 0.39 mmol) in DMF (5.0 mL). Deprotection by GP H (HPFC, SiO₂, DCM/MeOH 0 → 10% followed by C-18, H₂O/MeOH 5 → 100%) gave compound 71 (54 mg, 45%) as a pale yellow solid.

¹H NMR (500 MHz, DMSO-d₆): 1.64 (dm, 2H, *J_{gem} =* 13.4 Hz, H-3a,5a-C₅H₈O); 2.06 (ddd, 2H, *J_{gem} =* 13.4 Hz, *J_{3b,2b}* = 10.3 Hz, *J_{3b,2a}* = 4.3 Hz, H-3b,5b-C₅H₈O); 3.60 (dt, 2H, *J_{gem}* = 11.4 Hz, *J_{2a,3a}* = *J_{2a,3b}* = 4.1 Hz, H-2a,6a-C₅H₈O); 3.87 (btd, 2H, *J_{gem} = J_{2b,3b} =* 10.8 Hz, *J_{2b,3a}* = 2.5 Hz, H-2b,6b-C₅H₈O); 3.93 (s, 3H, CH₃O); 6.90 (s, 1H, H-5); 7.21 (dd, 1H, *J_{5',4'} =* 7.3 Hz, *J_{5',6'} =* 5.0 Hz, H-5'); 7.63 (m, 2H, H-*m-*Ph); 7.96 (m, 2H, H-o-Ph); 8.03 (dd, 1H, *J_{4',5'} =* 7.3 Hz, *J_{4',6'}* = 2.0 Hz, H-4'); 8.37 (dd, 1H, *J_{6',5'} =* 5.0 *Hz, J_{6',4'}* = 2.0 Hz, H-6'); 8.82 (s, 1H, H-2); 12.66 (bs, 1H, NH). HRMS (ESI) for C₂₃H₂₄N₅O₂⁺ (M+H)⁺: calcd 402.19245; found 402.19240.

### 4-{4-[4-(2-Methoxy-6-methylpyridin-3-yl)-7H-pyrrolo[2,3-d]pyrimidin-6-yl]phenyl}tetrahydro-2H-pyran-4-amine (72)

GP C starting from **VIIIb** (158 mg, 0.34 mmol) and 2-methoxy-6-methylpyridin-3-yl boronic acid (75 mg, 0.45 mmol) in DMF/H₂O mixture (3/1; 4.4 mL). Deprotection by GP H (HPFC, SiO₂, DCM/MeOH 0 → 10% followed by C-18, H₂O/MeOH 5 → 100%) gave compound **72** (136 mg, 95%) as a pale yellow solid.

¹H NMR (500 MHz, CD₃OD): 2.17 (ddd, 2H, *J_{gem}* = 13.8 Hz, *J_{3a,2a}* = 9.9 Hz, *J_{3a,2b}* = 4.1 Hz, H-3a,5a-C₅H₈O); 2.56 (s, 3H, CH₃); 2.63 (dm, 2H, *J_{gem}* = 13.8 Hz, H-3b,5b-C₅H₈O); 3.55 (td, 2H, *J_{gem}* = *J_{2a,3a}* = 11.4 Hz, *J_{2a,3b}* = 1.9 Hz, H-2a,6a-C₅H₈O); 3.95 (bdt, 2H, *J_{gem}* = 12.4 Hz, *J_{2b,3b} = J_{2b,3a}* = 4.1 Hz, H-2b,6b-C₅H₈O); 3.98 (s, 3H, CH₃O-4'); 6.92 (s, 1H, H-5); 7.03 (d, 1H, *J_{5',4'} =* 7.5 Hz, H-5'); 7.75 (m, 2H, H-m-Ph); 7.89 (d, 1H, *J_{4',5'} =* 7.5 Hz, H-4'); 8.03 (m, 2H, H-o-Ph); 8.78 (s, 1H, H-2). HRMS (ESI) for C₂₄H₂₅O₂N₅Na⁺ (M+Na)⁺: calcd 438.4970; found 438.19005.

### 4-{4-[4-(2-Cyclopropoxypyridin-3-yl)-7H-pyrrolo[2,3-d]pyrimidin-6-yl]phenyl}tetrahydro-2H-pyran-4-amine (73)

GP C starting from **VIIIb** (130 mg, 0.28 mmol) and 2-cyclopropoxypyridin-3-yl boronic acid (66 mg, 0.37 mmol) in DMF/H₂O mixture (3/1; 3.8 mL). Deprotection by GP H (HPFC, SiO₂, DCM/MeOH 0 → 10% followed by C-18, H₂O/MeOH 5 → 100%) gave compound **73** (34 mg, 28%) as a pale yellow solid.

¹H NMR (500 MHz, CD₃OD): 0.63-0.68 and 0.74-0.80 (2×m, 2×2H, CH₂-cyclopropyl); 2.13 (bddd, 2H, *J_{gem}* = 13.9 Hz, *J_{3a,2a}* = 9.2 Hz, *J_{3a,2b} =* 3.9 Hz, H-3a,5a-C₅H₈O); 2.59 (dm, 2H, *J_{gem}* = 13.9 Hz, H-3b,5b-C₅H₈O); 3.57 (ddd, 2H, *J_{gem}* = 12.1 Hz, *J_{2a,3a}* = 9.2 Hz, *J_{2a,3b}* = 2.4 Hz, H-2a,6a-C₅H₈O); 3.95 (dt, 2H, *J_{gem}* = 12.2 Hz, *J_{2b,3b}* = *J_{2b,3a}* = 4.4 Hz, H-2b,6b-C₅H₈O); 4.39 (tt, 1H, *J_{CH,CH2}* = 6.2 and 3.0 Hz, CH-cyclopropyl); 6.86 (s, 1H, H-5); 7.23 (dd, 1H, *J_{5',4'}* = 7.4 Hz, *J_{5',6'} =* 5.0 Hz, H-5'); 7.75 (m, 2H, H-*m-*Ph); 8.01 (m, 2H, H-*o*-Ph); 8.02 (dd, 1H, *J_{4',5'} =* 7.4 Hz, *J_{4',6'} =* 1.9 Hz, H-4'); 8.38 (dd, 1H, *J_{6',5'}* = 5.0 *Hz, J_{6',4'} =* 1.9 Hz, H-6'); 8.79 (s, 1H, H-2). HRMS (ESI) for C₂₅H₂₅O₂N₅Na⁺ (M+Na)⁺: calcd 450.5080; found 450.19012.

### 1-{4-[4-(4,6-Dimethoxypyrimidin-5-yl)-7H-pyrrolo[2,3-d]pyrimidin-6-yl]phenyl}cyclohexan-1-amine (74)

GP C starting from **VIIIa** (100 mg, 0.18 mmol) and 4,6-dimethoxypyrimidin-5-yl boronic acid (43 mg, 0.23 mmol) in DMF/H₂O mixture (3/1; 2.4 mL). Deprotection by GP H (HPFC, SiO₂, DCM/MeOH 0 → 10% followed by C-18, H₂O/MeOH 5 → 100%) gave compound **74** (31 mg, 40%) as a pale yellow solid.

¹H NMR (500 MHz, DMSO-d₆): 1.32 (m, 1H, H-4a-cyclohexyl); 1.39-1.47 (m, 2H, H-3a,5a-cyclohexyl); 1.54 (m, 1H, H-4b-cyclohexyl); 1.57-1.65 (m, 2H, H-2a,6a-cyclohexyl); 1.67-1.77 (m, 2H, H-3b,5b-cyclohexyl); 1.87-1.97 (m, 2H, H-2b,6b-cyclohexyl); 3.87 (s, 6H, CH₃O); 6.77 (s, 1H, H-5); 7.63 (m, 2H, H-m-Ph); 7.92 (m, 2H, H-o-Ph); 8.67 (s, 1H, H-2'); 8.78 (s, 1H, H-2); 12.64 (bs, 1H, NH). HRMS (ESI) for C₂₄H₂₇O₂N₆⁺ (M+H)⁺: calcd 431.5195; found 431.21909.

### 1-{4-[4-(2-Methoxypyridin-3-yl)-7H-pyrrolo[2,3-d]pyrimidin-6-yl]phenyl}cyclohexan-1-amine (75)

GP C starting from **VIIIa** (240 mg, 0.43 mmol) and 2-methoxypyridin-3-yl boronic acid (86 mg, 0.56 mmol) in DMF (7.2 mL). Deprotection by GP H (HPFC, SiO₂, DCM/MeOH 0 → 10% followed by C-18, H₂O/MeOH 5 → 100%) gave compound **75** (78 mg, 45%) as a pale yellow solid.

¹H NMR (500 MHz, CD₃OD): 1.46 - 1.59 (m, 3H, H-4a,3a,5a-cyclohexyl); 1.63 (m, 1H, H-4b-cyclohexyl); 1.73-1.83 (m, 2H, H-3b,5b-cyclohexyl); 1.92-2.01 (m, 2H, H-2a,6a-cyclohexyl); 2.52-2.62 (m, 2H, H-2b,6b-cyclohexyl); 4.00 (s, 3H, CH₃O); 6.91 (s, 1H, H-5); 7.19 (dd, 1H, *J_{5',4'} =* 7.3 Hz, *J_{5',6'} =* 5.0 Hz, H-5'); 7.74 (m, 2H, H-*m*-Ph); 8.01 (m, 2H, H-o-Ph); 8.01 (dd, 1H, *J₄'_{,5'} =* 7.3 Hz, *J_{4',6'}* = 1.9 Hz, H-4'); 8.35 (dd, 1H, *J_{6',5'} =* 5.0 *Hz, J_{6',4'} =* 1.9 Hz, H-6'); 8.80 (s, 1H, H-2). HRMS (ESI) for C₂₄H₂₆O₂N₅Na⁺ (M+Na)⁺: calcd 400.2132; found 400.21287.

### 4-{4-[4-(3-Methoxypyridin-4-yl)-7H-pyrrolo[2,3-d]pyrimidin-6-yl]phenyl}tetrahydro-2H-pyran-4-amine (76)

GP C starting from **VIIIb** (430 mg, 0.94 mmol) and 3-methoxypyridin-4-yl boronic acid (186 mg, 1.22 mmol) in DMF/H₂O mixture (3/1; 12.4 mL). Deprotection by GP H (HPFC, SiO₂, DCM/MeOH 0 → 10% followed by C-18, H₂O/MeOH 5 → 100%) gave compound **76** (151 mg, 40%) as a pale yellow solid.

¹H NMR (500 MHz, CD₃OD): 2.14 (ddd, 2H, *J_{gem}* = 13.8 Hz, *J_{3a,2a}* = 9.2 Hz, *J_{3a,2b}* = 3.9 Hz, H-3a,5a-C₅H₈O); 2.59 (dm, 2H, *J_{gem}* = 13.8 Hz, H-3b,5b-C₅H₈O); 3.56 (ddd, 2H, *J_{gem} =* 12.1 Hz, *J_{2a,3a}* = 9.2 Hz, *J_{2a,3b}* = 2.6 Hz, H-2a,6a-C₅H₈O); 3.95 (dt, 2H, *J_{gem}* = 12.1 Hz, *J_{2b,3b}* = *J_{2b,3a}* = 4.3 Hz, H-2b,6b-C₅H₈O); 3.98 (s, 3H, CH₃O); 6.90 (s, 1H, H-5); 7.63 (d, 1H, *J_{5',6'} =* 4.8 Hz, H-5'); 7.74 (m, 2H, H-m-Ph); 8.03 (m, 2H, H-o-Ph); 8.39 (d, 1H, *J_{6',5'} =* 4.8 Hz, H-6'); 8.57 (s, 1H, H-2'); 8.83 (s, 1H, H-2). HRMS (ESI) for C₂₅H₂₇O₂N₅Cl⁺ (M+H)⁺: calcd 402.1225; found 402.19263.

### 1-{4-[4-(5-Fluoro-2-methoxypyridin-3-yl)-7H-pyrrolo[2,3-d]pyrimidin-6-yl]phenyl}cyclohexan-1-amine (77)

GP C starting from **VIIIa** (185 mg, 0.33 mmol) and 5-fluoro-2-methoxypyridin-3-yl boronic acid (74 mg, 0.43 mmol) in toluene (5.5 mL). Deprotection by GP H (HPFC, SiO₂, DCM/MeOH 0 → 10% followed by C-18, H₂O/MeOH 5 → 100%) gave compound 77 (58 mg, 42%) as a pale yellow solid.

¹H NMR (500 MHz, CD₃OD): 1.41-1.62 (m, 4H, H-4,3a,5a-cyclohexyl); 1.66-1.83 (m, 4H, H-3b,5b,2a,6a-cyclohexyl); 2.17-2.27 (m, 2H, H-2b,6b-cyclohexyl); 3.96 (s, 3H, CH₃O); 6.81 (s, 1H, H-5); 7.65 (m, 2H, H-m-Ph); 7.83 (dd, 1H, *J_{4',F} =* 8.1 Hz, *J_{4',6'}* = 3.0 Hz, H-4'); 7.87 (m, 2H, H-o-Ph); 8.20 (d, 1H, *J_{6',4'}* = 3.0 Hz, H-6'); 8.76 (s, 1H, H-2). ¹⁹F NMR (470.4 MHz, CD₃OD): -136.51 (d, 1F, *J_{F,4'}* = 8.1 Hz, F-5'). HRMS (ESI) for C₂₄H₂₅ON₅F⁺ (M+H)⁺: calcd 418.4959; found 418.20383.

### 1-{4-[4-(2-Methoxy-6-methylpyridin-3-yl)-7H-pyrrolo[2,3-d]pyrimidin-6-yl]phenyl}cyclohexan-1-amine (78)

GP C starting from **VIIIa** (250 mg, 0.40 mmol) and 2-methoxy-6-methylpyridin-3-yl boronic acid (88 mg, 0.53 mmol) in DMF (5.5 mL). Deprotection by GP H (HPFC, SiO₂, DCM/MeOH 0 → 10% followed by C-18, H₂O/MeOH 5 → 100%) gave compound **78** (117 mg, 71%) as a pale yellow solid.

¹H NMR (500 MHz, DMSO-d₆): 1.26 (m, 1H, H-4a-cyclohexyl); 1.39-1.47 (m, 2H, H-3a,5a-cyclohexyl); 1.50-1.61 (m, 3H, H-2a,6a, 4b); 1.65-1.76 (m, 2H, H-3b,5b-cyclohexyl); 1.76-1.85 (m, 2H, H-2b,6b-cyclohexyl); 2.50 (s, 3H, CH₃-6'); 3.91 (s, 3H, CH₃O-2'); 6.82 (s, 1H, H-5); 7.04 (bdq, 1H, *J_{5',4'} =* 7.4 Hz, *J_{5',CH3} =* 0.7 Hz, H-5'); 7.61 (m, 2H, H-m-Ph); 7.88 (m, 2H, H-o-Ph); 7.91 (d, 1H, *J_{4',5'} =* 7.4 Hz, H-4'); 8.78 (s, 1H, H-2). HRMS (ESI) for C₂₅H₂₈ON₅⁺ (M+H)⁺: calcd 414.5325; found 414.22902.

### 4-{4-[4-(4,6-Dimethoxypyrimidin-5-yl)-7H-pyrrolo[2,3-d]pyrimidin-6-yl]phenyl}tetrahydro-2H-pyran-4-amine (79)

GP C starting from **VIIIb** (239 mg, 0.43 mmol) and 4,6-dimethoxypyrimidin-5-yl boronic acid (112mg, 0.61 mmol) in toluene (7.8 mL). Deprotection by GP H (HPFC, SiO₂, DCM/MeOH 0 → 10% followed by C-18, H₂O/MeOH 5 → 100%) gave compound 79 (67 mg, 19%) as a pale yellow solid.

¹H NMR (500 MHz, DMSO-d₆): 1.54 (dm, 2H, *J_{gem} =* 13.5 Hz, H-3a,5a-C₅H₈O); 1.98 (dm, 2H, *J_{gem}* = 13.5 Hz, *J_{3b,2a}* = 11.4 Hz, *J_{3b,2b}* = 4.6 Hz, H-3b,5b-C₅H₈O); 3.65 (ddd, 2H, *J_{gem}* = 11.1 Hz, *J_{2a,3b}* = 4.5 *Hz, J_{2a,3a} =* 2.8 Hz, H-2a,6a-C₅H₈O); 3.88 (s, 2×3H, CH₃O); 3.88 - 3.93 (m, 2H, H-2b,6b-C₅H₈O); 6.77 (s, 1H, H-5); 7.61 (m, 2H, H-m-Ph); 7.92 (m, 2H, H-o-Ph); 8.68 (s, 1H, H-2'); 8.79 (s, 1H, H-2); 12.63 (bs, 1H, NH). HRMS (ESI) for C₂₃H₂₄O₂N₅⁺ (M+H)⁺: calcd 433.4915; found 433.19839.

### 4-{4-[4-(4-Methoxy-2-methylpyrimidin-5-yl)-7H-pyrrolo[2,3-d]pyrimidin-6-yl]phenyl}tetrahydro-2H-pyran-4-amine (80)

GP D starting from **VIIIb** (230 mg, 0.48 mmol) and 4-methoxy-2-methylpyrimidin-5-yl boronic acid (106 mg, 0.62 mmol) in DMF (9.5 mL). Deprotection by GP H (HPFC, SiO₂, DCM/MeOH 0 → 10% followed by C-18, H₂O/MeOH 5 → 100%) gave compound **80** (72 mg, 36%) as a pale yellow solid.

¹H NMR (600.1 MHz, DMSO-d₆): 1.55 (dm, 2H, *J_{gem} =* 13.4 Hz, H-3a,5a-C₅H₈O); 1.98 (ddd, 2H, *J_{gem}* = 13.4 Hz, *J_{3b,2b}* = 11.4 Hz, *J_{3b,2a}* = 4.6 Hz, H-3b,5b-C₅H₈O); 2.66 (s, 3H, CH₃); 3.64 (bddd, 2H, *J_{gem}* = 11.1 *Hz, J_{2a,3b}* = 4.4 Hz, *J_{2a,3a}* = 2.7 Hz, H-2a,6a-C₅H₈O); 3.88 (td, 2H, *J_{gem}* = *J_{2b,3b}* = 11.3 Hz, *J_{2b,3a}* = 2.3 Hz, H-2b,6b-C₅H₈O); 4.01 (s, 3H, CH₃O); 6.95 (s, 1H, H-5); 7.62 (m, 2H, H-m-Ph); 7.94 (m, 2H, H-o-Ph); 8.73 (s, 1H, H-6'); 8.83 (s, 1H, H-2); 12.67 (vbs, 1H, NH). HRMS (ESI) for C₂₃H₂₅O₂N₆⁺ (M+H)⁺: calcd 417.20335; found 417.20327.

### 1-{4-[4-(4-Methoxy-2-methylpyrimidin-5-yl)-7H-pyrrolo[2,3-d]pyrimidin-6-yl]phenyl}cyclohexan-1-amine (81)

GP D starting from **VIIIa** (350 mg, 0.73 mmol) and 4-methoxy-2-methylpyrimidin-5-yl boronic acid (134 mg, 0.80 mmol) in DMF (12.3 mL). Deprotection by GP H (HPFC, SiO₂, DCM/MeOH 0 → 10% followed by C-18, H₂O/MeOH 5 → 100%) gave compound 81 (150 mg, 49%) as a pale yellow solid.

¹H NMR (600.1 MHz, DMSO-d₆): 1.27 (m, 1H, H-4a-cyclohexyl); 1.41-1.50 (m, 2H, H-3a,5a-cyclohexyl); 1.51-1.62 (m, 3H, H-2a,6a,4b-cyclohexyl); 1.68-1.87 (m, 4H, H-3b,5b,2b,6b-cyclohexyl); 2.66. (s, 3H, CH₃); 4.01 (s, 3H, CH₃O); 6.93 (s, 1H, H-5); 7.64 (m, 2H, H-*m*-Ph); 7.91 (m, 2H, H-*o*-Ph); 8.73 (s, 1H, H-6'); 8.82 (s, 1H, H-2). HRMS (ESI) for C₂₄H₂₇ON₆⁺ (M+H)⁺: calcd 415.22409; found 415.22396.

### 4-{4-[4-Chloro-7-[(2-(trimethylsilyl)ethoxy)methyl]-7H-pyrrolo[2,3-d]pyrimidin-6-yl]-3-fluorophenyl} tetrahydro-2H-pyran-4-amine (VIIIf)

GP G starting from **VII** (500 mg, 1.22 mmol) and 4-(4-bromo-3-fluorophenyl)tetrahydro-2*H*-pyran-4-amine (616 mg, 2.24 mmol), (HPFC, SiO₂, DCM/MeOH 0 → 5%) gave compound **VIIIf** (131 mg, 32%) as a yellowish solid.

¹H NMR (401 MHz, Chloroform-*d*): -0.11 (s, 9H, 3× -CH₃); 0.89-0.77 (m, 2H, H-5'); 1.53 (s, 2H, - NH₂); 1.70-1.60 (m, 2H, H-2a,6a-C₅H₈O); 2.18 (ddd, *J* = 13.5, 11.0, 4.7 Hz, 2H, H-2b,6b-C₅H₈O); 3.60-3.47 (m, 2H, H-4'); 3.87-3.77 (m, 2H, H-3a,5a-C₅H₈O); 3.92 (td, *J =* 11.3, 2.3 Hz, 2H, H-3b,5b-C₅H₈O); 5.60 (s, 2H, H-2'); 6.73 (d, *J =* 1.3 Hz, 1H, H-5); 7.44-7.32 (m, 2H, H-o-Ph); 7.67 (t, *J =* 8.0 Hz, 1H, H-m-Ph); 8.66 (s, 1H, H-2). HRMS (ESI) for C₂₃H₃₁O₂N₄ClFSi⁺ (M+H)⁺: calcd 477.18834; found 477.18846.

### 4-{3-Fluoro-4-[4-(4-methoxypyrimidin-5-yl)-7H-pyrrolo[2,3-d]pyrimidin-6-yl]phenyl}tetrahydro-2H-pyran-4-amine (82)

GP D starting from **VIIIf** (93 mg, 0.19 mmol) and 4-methoxy-5-(4,4,5,5-tetramethyl-1,3,2-dioxaborolan-2-yl)pyrimidine (71 mg, 0.29 mmol) in DMF (13.8 mL). Deprotection by GP H (HPFC, SiO₂, DCM/MeOH 0 → 10% followed by C-18, H₂O/MeOH 5 → 100%) gave compound 82 (65 mg, 79%) as a pale yellow solid.

¹H NMR (600.1 MHz, CD₃OD): 1.78 (dm, 2H, *J_{gem}* = 13.8 Hz, H-3a,5a-C₅H₈O); 2.21 (ddd, 2H, *J_{gem} =* 13.8 Hz, *J_{3b,2b}* = 9.8 Hz, *J_{3b,2a}* = 4.2 Hz, H-3b,5b-C₅H₈O); 3.73 (dt, 2H, *J_{gem}* = 11.8 Hz, *J_{2a,3b}* = *J_{2a,3a}* = 4.4 Hz, H-2a,6a-C₅H₈O); 3.95 (ddd, 2H, *J_{gem}* = 11.8 Hz, *J_{2b,3b}* = 9.8 Hz, *J_{2b,3a}* = 2.7 Hz, H-2b,6b-C₅H₈O); 4.12 (s, 3H, CH₃O); 6.92 (d, 1H, *J_{5,F} =* 2.2 Hz, H-5); 7.50 (dd, 1H, *J_{3,F} =* 13.7 Hz, *J_{3,5} =* 1.9 Hz, H-3-Ph); 7.51 (dd, 1H, *J_{5,6} =* 8.3 Hz, *J_{5,3} =* 1.9 Hz, H-5-Ph); 7.89 (t, 1H, *J_{6,5} = J_{6,F} =* 8.3 Hz, H-6-Ph); 8.84 (s, 1H, H-6'); 8.85 (s, 1H, H-2); 8.92 (s, 1H, H-2'). ¹⁹F NMR (470.4 MHz, CD₃OD): -110.68 (ddd, 1F, *J_{F,3} =* 13.8 Hz, *J_{F,6} =* 8.2 Hz, *J_{F,5}* = 2.4 Hz, F-2-Ph). HRMS (ESI) for C₂₂H₂₂O₂N₆F⁺ (M-H)⁺: calcd 421.17828; found 421.17820.

### Tert-butyl (1-{4-[4-(chloro)-7-[(2-(trimethylsilyl)ethoxy)methyl]-7H-pyrrolo[2,3-d]pyrimidin-6-yl]-3-fluorophenyl}cyclohexyl)carbamate (VIIIe)

GP G starting from **VII** (230 mg, 0.56 mmol) and *tert*-butyl 1-(4-bromo-3-fluorophenyl)cyclohexylcarbamate (289 mg, 0.78 mmol), (HPFC, SiO₂, cHex/EtOAc 0 → 90%) gave compound **VIIIe** (168 mg, 52%) as a brown oil.

¹H NMR (401 MHz, Chloroform-*d*): -0.09 (s, 9H, 3x CH₃); 0.93-0.76 (m, 2H, H-5'); 1.48-1.31 (m, 11H, CH₃-tBu and H-4a,4b cyclohexyl); 1.81-1.56 (m, 6H, H-2b,3a,3b, 5a,5b,6b cyclohexyl); 2.36-2.14 (m, 2H, H-2a,6a cyclohexyl); 3.60-3.51 (m, 2H, H-4'); 4.91 (bs, 1H, NH); 5.60 (s, 2H, H-2'); 6.74 (d, *J* = 1.4 Hz, 1H); 7.35-7.25 (m, 2H, H-o-Ph); 7.65 (t, *J* = 7.9 Hz, 1H, H-*m*-Ph); 8.68 (s, 1H, H-2). ¹⁹F NMR (376 MHz, Chloroform-d): -113.55. HRMS (ESI) for C₂₉H₄₁O₃N₄CIFSI (M+H)⁺: calcd 575.26150; found 575.26174.

### 1-{3-Fluoro-4-[4-(4-methoxypyrimidin-5-yl)-7H-pyrrolo[2,3-d]pyrimidin-6-yl]phenyl}cyclohexan-1-amine (83)

GP D starting from **VIIIe** (121 mg, 0.19 mmol) and 4-methoxy-5-(4,4,5,5-tetramethyl-1,3,2-dioxaborolan-2-yl)pyrimidine (66 mg, 0.28 mmol) in DMF (3.8 mL). Deprotection by GP H (HPFC, SiO₂, DCM/MeOH 0 → 10% followed by C-18, H₂O/MeOH 5 → 100%) gave compound **83** (65 mg, 76%) as a pale yellow solid.

¹H NMR (600.1 MHz, DMSO-d₆): 1.28 (m, 1H, H-4a-cyclohexyl); 1.43-1.49 (m, 2H, H-3a,5a-cyclohexyl); 1.51-1.57 (m, 2H, H-2a,6a-cyclohexyl); 1.60 (m, 1H, H-4b-cyclohexyl); 1.69-1.83 (m, 4H, H-3b,5b,2b,6b-cyclohexyl); 4.02 (s, 3H, CH₃O); 6.84 (d, 1H, *J_{5,F} = 2.6* Hz, H-5); 7.50 (dd, 1H, *J_{5,6}* = 8.2 Hz, *J_{5,3} =* 1.8 Hz, H-5-Ph); 7.54 (dd, 1H, *J_{3,F} =* 13.9 Hz, *J_{3,5} =* 1.8 Hz, H-3-Ph); 7.93 (t, 1H, *J_{6,F} = J_{6,5} =* 8.3 Hz, H-6-Ph); 8.87 (d, 1H, *J_{6,2'}* = 0.5 Hz, H-6'); 8.89 (s, 1H, H-2); 8.97 (d, 1H, *J_{2,6'} =* 0.5 Hz, H-2'). ¹⁹F NMR (470.4 MHz, DMSO-d₆): -109.58 (ddd, 1F, *J_{F,3} =* 13.9 Hz, *J_{F,6} =* 8.4 Hz, *J_{F,5}* = 2.7 Hz, F-2-Ph). HRMS (ESI) for C₂₃H₂₄ON₆F⁺ (M-H)⁺: calcd 419.19901; found 419.19892.

### 1-{4-[4-(2,6-Dimethoxypyridin-3-yl)-7H-pyrrolo[2,3-d]pyrimidin-6-yl]phenyl}cyclohexan-1-amine (84)

GP D starting from **VIIIa** (300 mg, 0.55 mmol) and (2,6-dimethoxypyridin-3-yl)boronic acid (148 mg, 0.81 mmol) in DMF (5.0 mL). Deprotection by GP H (HPFC, SiO₂, DCM/MeOH 0 → 10% followed by C-18, H₂O/MeOH 5 → 100%) gave compound **84** (51 mg, 22%) as a white solid.

¹H NMR (500 MHz, DMSO-d₆): 1.28 (m, 1H, H-4a-cyclohexyl); 1.40-1.49 (m, 2H, H-3a,5a-cyclohexyl); 1.51-1.62 (m, 3H, H-2a,6a,4b-cyclohexyl); 1.66-1.78 (m, 2H, H-3b,5b-cyclohexyl); 1.78-1.90 (m, 2H, H-2b,6b-cyclohexyl); 3.97 (s, 3H, CH₃O-2'); 3.98 (s, 3H, CH₃O-6'); 6.58 (d, 1H, *J_{5',4'}* = 8.1 Hz, H-5'); 6.86 (s, 1H, H-5); 7.63 (m, 2H, H-m-Ph); 7.89 (m, 2H, H-o-Ph); 8.02 (d, 1H, *J_{4',5'} = 8.1* Hz, H-4'); 8.76 (s, 1H, H-2); 12.52 (bs, 1H, NH). HR MS (ESI) for C₂₅H₂₈O₂N₅ [M + H]⁺: calcd 430.22375; found 430.22343; for C₂₅H₂₇O₂N₅Na [M + Na]⁺: calcd 452.20570; found 430.20540.

### 1-{4-[4-(2-Methoxy-6-trifluoromethylpyridin-3-yl)-7H-pyrrolo[2,3-d]pyrimidin-6-yl]phenyl}cyclohexan-1-amine (85)

GP D starting from **VIIIa** (300 mg, 0.54 mmol) and (2-methoxy-6-trifluoromethylpyridin-3-yl)boronic acid (179 mg, 0.81 mmol) in DMF (5.0 mL). Deprotection by GP H (HPFC, SiO₂, DCM/MeOH 0 - 10% followed by C-18, H₂O/MeOH 5 → 100%) gave compound **85** (56 mg, 22%) as a yellow solid.

¹H NMR (500 MHz, DMSO-d₆): 1.28 (m, 1H, H-4a-cyclohexyl); 1.41-1.50 (m, 2H, H-3a,5a-cyclohexyl); 1.51-1.63 (m, 3H, H-2a,6a,4b-cyclohexyl); 1.67-1.78 (m, 2H, H-3b,5b-cyclohexyl); 1.78-1.87 (m, 2H, H-2b,6b-cyclohexyl); 3.98 (s, 3H, CH₃O-2'); 6.95 (s, 1H, H-5); 7.64 (m, 2H, H-m-Ph); 7.96 (d, 1H, *J_{5',4'} = 7.6* Hz, H-5'); 7.93 (m, 2H, H-o-Ph); 8.28 (d, 1H, *J_{4',5'} =* 7.6 Hz, H-4'); 8.85 (s, 1H, H-2), 12.67 (vbs, 1H, NH). ¹⁹F NMR (470.4 MHz, DMSO-d₆): -66.77 (s, 3F, CF₃). HR MS (ESI) for C₂₅H₂₅ON₅F₃ [M + H]⁺: calcd 468.20057; found 468.20062; for C₂₅H₂₄ON₅F₃Na [M + Na]⁺: calcd 490.18252; found 490.18277.

### 1-{4-[4-(4-Methoxypyrimidin-5-yl)-7H-pyrrolo[2,3-d]pyrimidin-6-yl]-phenyl}cyclohexan-1-amine (86)

GP D starting from **VIIIa** (300 mg, 0.54 mmol) and (4-methoxypyrimidin-5-yl)boronic acid (125 mg, 0.81 mmol) in DMF (5.0 mL). Deprotection by GP H (HPFC, SiO₂, DCM/MeOH 0 → 10% followed by C-18, H₂O/MeOH 5 → 100%) gave compound **86** (36 mg, 17%) as a yellow solid.

¹H NMR (500 MHz, DMSO-d₆): 1.28 (m, 1H, H-4a-cyclohexyl); 1.40-1.50 (m, 2H, H-3a,5a-cyclohexyl); 1.51-1.65 (m, 3H, H-2a,6a,4b-cyclohexyl); 1.66-1.79 (m, 2H, H-3b,5b-cyclohexyl); 1.79-1.89 (m, 2H, H-2b,6b-cyclohexyl); 4.03 (s, 3H, CH₃O-4'); 6.96 (s, 1H, H-5); 7.64 (m, 2H, H-m-Ph); 7.93 (m, 2H, H-o-Ph); 8.85 (s, 2H, H-2,6'); 8.97 (s, 1H, H-2'). HR MS (ESI) for C₂₃H₂₅ON₆ [M + H]⁺: calcd 401.20844; found 401.20849.

### Tert-butyl (2-{4-[4-chloro-7-[(2-(trimethylsilyl)ethoxy)methyl]-7H-pyrrolo[2,3-d]pyrimidin-6-yl]phenyl}propan-2-yl)carbamate (VIIIc)

GP G starting from **VII** (1.5 g, 3.67 mmol) and *tert*-butyl 2-(4-bromophenyl)propan-2-ylcarbamate (916 mg, 2.93 mmol), (HPFC, SiO₂, cHex/EtOAc 0 → 90%) gave compound **VIIIc** (623 mg, 33%) as a yellow oil.

¹H NMR (400 MHz, Chloroform-d): 0.00 (s, 9H, 3×CH₃); 1.05-0.92 (m, 2H, H-5'); 1.44 (s, 9H, CH₃-tBu); 1.69 (s, 6H, 2×CH₃); 3.85-3.72 (m, 2H, H-4'); 5.65 (s, 2H, H-2'); 6.71 (s, 1H, H-5); 7.58-7.49 (m, 2H, H-o-Ph); 7.82-7.71 (m, 2H, H-m-Ph); 8.68 (s, 1H, H-2). HR MS (ESI) for C₂₆H₃₈O₃N₄ClSi [M + H]⁺: calcd 517.23962; found 517.24001; for C₂₆H₃₇O₃N₄ClNaSi [M + Na]⁺: calcd 539.22157; found 539.22182.

### 2-{4-[4-(2,6-Dimethoxypyridin-3-yl)-7H-pyrrolo[2,3-d]pyrimidin-6-yl]phenyl}-propan-2-amine (87)

GP D starting from **VIIIc** (300 mg, 0.58 mmol) and (2,6-dimethoxypyridin-3-yl)boronic acid (159 mg, 0.87 mmol) in DMF (5.0 mL). Deprotection by GP H (HPFC, SiO₂, DCM/MeOH 0 → 10% followed by C-18, H₂O/MeOH 5 → 100%) gave compound 87 (109 mg, 52%) as a yellow solid.

¹H NMR (500 MHz, DMSO-d₆): 1.39 (s, 6H, (CH₃)₂C);); 3.97 (s, 3H, CH₃O-2'); 3.98 (s, 3H, CH₃O-6'); 6.58 (d, 1H, *J_{5',4'} =* 8.1 Hz, H-5'); 6.86 (s, 1H, H-5); 7.62 (m, 2H, H-m-Ph); 7.88 (m, 2H, H-o-Ph); 8.02 (d, 1H, *J_{4',5'}* = 8.1 Hz, H-4'); 8.76 (s, 1H, H-2). HR MS (ESI) for C₂₂H₂₄O₂N₅ [M + H]⁺: calcd 390.19245; found 390.19224.

### Tert-butyl (3-{4-[4-(chloro)-7-[(2-(trimethylsilyl)ethoxy)methyl]-7H-pyrrolo[2,3-d]pyrimidin-6-yl]phenyl}pentan-3-yl)carbamate (VIIId)

GP G starting from **VII** (389 mg, 0.95 mmol) and *tert*-butyl 3-(4-bromophenyl)pentan-3-ylcarbamate (388 mg, 1.14 mmol), (HPFC, SiO₂, cHex/EtOAc 0 → 90%) gave compound **VIIId** (120 mg, 23%) as a yellow oil.

¹H NMR (400 MHz, Chloroform-d): -0.03 (s, 9H, 3×CH₃); 0.79 (t, *J* = 7.4 Hz, 6H, 2x CH₃); 1.00-0.93 (m, 2H); 1.26 (t, *J =* 7.1 Hz, 4H, 2×CH₂); 1.51-1.34 (m, 9H, CH₃-tBu); 3.78-3.71 (m, 2H, H-4'); 5.63 (s, 2H, H-2'); 6.70 (s, 1H, H-5); 7.49-7.45 (m, 2H, H-o-Ph); 7.78-7.71 (m, 2H, H-m-Ph); 8.66 (s, 1H, H-2). HR MS (ESI) for C₂₈H₄₂O₃N₄ClSi [M + H]⁺: calcd 545.2644; found 545.2651.

### 3-{4-[4-(2,6-Dimethoxypyridin-3-yl)-7H-pyrrolo[2,3-d]pyrimidin-6-yl]phenyl}pentan-3-amine (88)

GP D starting from **VIIId** (120 mg, 0.22 mmol) and (2,6-dimethoxypyridin-3-yl)boronic acid (68 mg, 0.38 mmol) in DMF (5.0 mL). Deprotection by GP H (HPFC, SiO₂, DCM/MeOH 0 → 10% followed by C-18, H₂O/MeOH 5 → 100%) gave compound **88** (49 mg, 53%) as a yellow solid.

¹H NMR (500 MHz, DMSO-d₆): 0.66 (t, 6H, (C*H₃*CH₂)₂C); 1.64 and 1.80 (2×m, 2×2H, (CH₃C*H₂*)₂C); 3.97 (s, 3H, CH₃O-2'); 3.98 (s, 3H, CH₃O-6'); 6.58 (d, 1H, *J_{5',4'}* = 8.1 Hz, H-5'); 6.88 (s, 1H, H-5); 7.51 (m, 2H, H-m-Ph); 7.92 (m, 2H, H-o-Ph); 8.02 (d, 1H, *J_{4',5'} =* 8.1 Hz, H-4'); 8.76 (s, 1H, H-2); 12.52 (bs, 1H, NH). HR MS (ESI) for C₂₄H₂₈O₂N₅ [M + H]⁺: calcd 418.22375; found 418.22375.

### 4-{3-Fluoro-4-[4-(2-methoxypyridin-3-yl)-7H-pyrrolo[2,3-d]pyrimidin-6-yl]phenyl}tetrahydro-2H-pyran-4-amine (89)

GP D starting from **VIIIf** (110 mg, 0.226 mmol) and (2-methoxypyridin-3-yl)boronic acid (55 mg, 0.36 mmol) in DMF (4.5 mL). Deprotection by GP H (HPFC, C-18, H₂O/MeOH 10 → 100 %) gave compound **89** (21 mg, 10%) as a pale yellow solid.

¹H NMR (401 MHz, Methanol-*d*₄): 1.80 (dt, *J* = 13.3, 3.2 Hz, 2H, H-2b,6b-C₅H₈O); 2.23 (ddd, *J* = 13.8, 9.8, 4.3 Hz, 2H, H-2a,6a-C₅H₈O); 3.75 (dt, *J* = 11.8, 4.3 Hz, 2H, H-3b,5b-C₅H₈O); 3.96 (ddd, *J* = 12.1, 9.7, 2.7 Hz, 2H, H-3a,5a-C₅H₈O); 4.01 (s, 3H, CH₃O); 6.88 (d, *J =* 2.4 Hz, 1H, H-5); 7.21 (dd, *J* = 7.3, 5.0 Hz, 1H, H-3-Ph); 7.48 (dd, *J* = 9.5, 1.8 Hz, 1H, H-5-Ph); 7.51 (dd, *J* = 4.3, 1.9 Hz, 1H, H-5'); 7.90 (t, *J =* 8.3 Hz, 1H, H-6-Ph); 8.04 (dd, *J* = 7.3, 1.9 Hz, 1H, H-4'); 8.37 (dd, *J* = 5.0, 2.0 Hz, 1H, H-6'); 8.82 (s, 1H, H-2). ¹⁹F NMR (377 MHz, Methanol-*d*₄): -115.01 (ddd, *J =* 13.9, 8.3, 2.5 Hz). HR MS (ESI) for C₂₃H₂₃O₂N₅F [M + H]⁺: calcd 420.18303; found 420.18296.

### Tert-butyl {4-[5-(4-chloro-7-{(2-(trimethylsilyl)ethoxy)methyl}-7H-pyrrolo[2,3-d]pyrimidin-6-yl)pyridin-2-yl]tetrahydro-2H-pyran-4-yl}carbamate (VIIIh)

GP G starting from **VII** (700 mg, 1.7 mmol) and *tert*-butyl (4-(5-bromopyridin-2-yl)tetrahydro-2*H-*pyran-4-yl)carbamate (766 mg, 2.15 mmol), (HPFC, SiO₂, cHex/EtOAc 0 → 100 %- C-18, H₂O/MeOH 0 → 100 %) gave compound **VIIIh** (250 mg, 26%) as a yellowish solid.

¹H NMR (401 MHz, Chloroform-*d*) δ -0.04 (s, 9H, 3×CH₃), 1.00-0.93 (m, 2H, H-5'), 1.36 (s, 9H, C-(CH₃)₃), 2.19 (d, *J =* 13.8 Hz, 2H, H-2b,6b-C₅H₈O), 2.36 (ddd, *J =* 13.8, 11.1, 4.7 Hz, 2H, H-2a,6a-C₅H₈O), 3.77-3.71 (m, 2H, H-4'), 3.81 (td, *J =* 11.5, 2.2 Hz, 2H, H-3b,5b-C₅H₈O), 3.90 (ddd, *J =* 11.9, 4.6, 2.9 Hz, 2H, H-3a,5a-C₅H₈O), 5.13 (s, 1H, H-NH), 5.61 (s, 2H, H-2'), 6.75 (s, 1H, H-5), 7.56 (dd, *J =* 8.3, 0.9 Hz, 1H, H-5"-Py), 8.16 (dd, *J =* 8.3, 2.3 Hz, 1H, H-4"-Py), 8.67 (s, 1H, H-2), 8.91 (dd, *J* = 2.3, 0.8 Hz, 1H, H-2"-Py). HR MS (ESI) for C₂₇H₃₉O₄N₅ClSi [M + H]⁺: calcd 560.24544; found 560.24554.

### 4-{5-[4-(4-Methoxypyrimidin-5-yl)-7H-pyrrolo[2,3-d]pyrimidin-6-yl]pyridin-2-yl}tetrahydro-2H-pyran-4-amine (90)

GP D starting from **VIIIh** (138 mg, 0.235 mmol) and 4-methoxy-5-(4,4,5,5-tetramethyl-1,3,2-dioxaborolan-2-yl)pyrimidine (88 mg, 0.352 mmol) in DMF (4.7 mL). Deprotection by GP H (HPFC, C-18, H₂O/MeOH 10 → 100 %) gave compound **90** (82 mg, 79%) as a pale yellow solid.

¹H NMR (401 MHz, Methanol-*d*₄) δ 1.85 (d, *J =* 13.5 Hz, 2H, H-3a,5a-C₅H₈O), 2.36 (ddd, *J =* 14.1, 10.0, 4.3 Hz, 2H, H-3b,5b-C₅H₈O), 3.76 (dt, *J =* 11.7, 4.3 Hz, 2H, H-2a,6a-C₅H₈O), 4.01 (ddd, *J =* 12.2, 10.0, 2.7 Hz, 2H, H-2b,6b-C₅H₈O), 4.14 (s, 3H, CH₃O), 6.97 (s, 1H, H-5), 7.62 (t, *J* = 8.4 Hz, 1H, H-5-Ph), 7.69 (dd, *J =* 14.1, 1.9 Hz, 1H, H-2-Ph), 7.74 (dd, *J =* 8.2, 1.9 Hz, 1H, H-6-Ph), 8.84 (s, 1H, H-6'),8.85 (s, 1H, H-2), 8.93 (s, 1H, H-2'). ¹⁹F NMR (377 MHz, Methanol-*d*₄) δ -112.10 (dd, *J* = 14.0, 8.5 Hz). HR MS (ESI) for C₂₁H₂₂O₂N₇ [M + H]⁺: calcd 404.18295; found 404.18312.

### Tert-butyl {4-[4-(4-chloro-7-(2-(trimethylsilyl)ethoxy)methyl)-7H-pyrrolo[2,3-d]pyrimidin-6-yl)-2-fluorophenyl]tetrahydro-2H-pyran-4-yl}carbamate (VIIIg)

GP G starting from **VII** (550 mg, 1.34 mmol) and *tert*-butyl (4-(4-bromo-2-fluorophenyl)tetrahydro-2H-pyran-4-yl)carbamate (766 mg, 2.052 mmol), (HPFC, SiO₂, cHex/EtOAc 0 → 100 % - C-18, H₂O/MeOH 0 → 100 %) gave compound **VIIIg** (178 mg, 23%) as a yellowish solid.

¹H NMR (401 MHz, Chloroform-*d*) δ -0.01 (s, 9H, 3×CH₃), 1.05-0.96 (m, 2H, H-5'), 1.37 (s, 9H, C-(CH₃))₃, 2.26 (ddd, *J =* 13.6, 10.5, 5.4 Hz, 2H, H-2a,6a-C₅H₈O), 2.44 (d, *J =* 13.6 Hz, 2H, H-2b,6b-C₅H₈O), 3.81-3.74 (m, 2H, H-4'), 3.96-3.82 (m, 4H, H-3a,3b,5a,5b-C₅H₈O), 5.13 (s, 1H, H-NH), 5.64 (s, 2H, H-2'), 6.74 (s, 1H, H-5), 7.52 (t, *J =* 8.4 Hz, 1H, H-o-Ph), 7.58 (dd, *J =* 9.0, 1.7 Hz, 1H, H-*m-*Ph), 7.62-7.59 (m, 1H, H-m-Ph), 8.68 (s, 1H, H-2). ¹⁹F NMR (376 MHz, Chloroform-*d*) δ -112.19. HR MS (ESI) for C₂₈H₃₉O₄N₄ClFSi [M + H]⁺: calcd 577.24076; found 577.24091.

### 4-{2-Fluoro-4-[4-(4-methoxypyrimidin-5-yl)-7H-pyrrolo[2,3-d]pyrimidin-6-yl]phenyl}tetrahydro-2H-pyran-4-amine (91)

GP D starting from **VIIIh** (179 mg, 0.312 mmol) and 4-methoxy-5-(4,4,5,5-tetramethyl-1,3,2-dioxaborolan-2-yl)pyrimidine (116 mg, 0.49 mmol) in DMF (6.2 mL). Deprotection by GP H (HPFC, C-18, H₂O/MeOH 10 → 100 %) gave compound **91** (88 mg, 67%) as a pale yellow solid.

¹H NMR (401 MHz, Methanol-*d*₄) δ 1.76 (dddd, *J =* 13.6, 4.8, 3.0, 1.4 Hz, 2H, H-3a,5a-C₅H₈O), 2.37 (ddd, *J =* 13.6, 9.4, 4.2 Hz, 2H, H-3b,5b-C₅H₈O), 3.73 (dt, *J =* 11.7, 4.5 Hz, 2H, H-2a,6a-C₅H₈O), 3.95 (ddd, *J =* 12.0, 9.4, 2.9 Hz, 2H, H-2b,6b-C₅H₈O), 4.12 (s, 3H, CH₃O), 7.01 (s, 1H, H-5), 7.70 (dd, *J =* 8.3, 0.9 Hz, 1H, H-5-Py), 8.26 (dd, *J =* 8.4, 2.4 Hz, 1H, H-6-Py), 8.83 (d, *J =* 0.6 Hz, 1H, H-6'), 8.84 (s, 1H, H-2), 8.92 (d, *J =* 0.6 Hz, 1H, H-2'), 9.08 (dd, *J =* 2.4, 0.8 Hz, 1H, H-2-Py). HR MS (ESI) for C₂₂H₂₂O₂N₆F [M + H]⁺: calcd 421.17828; found 421.17845.

### Results of Biological Activity Screening

The inhibitory activity of target compounds was tested on full-length human CDK9 using Mobility Shift Assay and NanoLuc^{®}-CDK9 Fusion Vector using NanoBRET^{™} TE Intracellular Kinase Assay, K-8. Results are summarized in Table 1 and confirm very high potency of all tested CDK9 inhibitors.

**Table 1. Inhibition of human CDK9/Cyc T1 (cell-based assay)**

| **Compound** | **CDK-9 inhibition** | **Compound** | **CDK-9 inhibition** |
|---|---|---|---|
| **1** | **B** | **48** | **C** |
| **2** | C | **49** | B |
| **3** | A | **50** | B |
| **4** | B | **51** | B |
| **5** | B | | |
| **6** | C | **52** | C |
| **7** | C | **53** | C |
| **8** | B | **54** | C |
| **9** | A | **55** | D |
| **10** | D | **56** | A |
| **11** | D | **57** | A |
| **12** | B | **58** | A |
| **13** | B | **59** | A |
| **14** | A | **60** | C |
| **15** | A | **61** | A |
| **16** | B | **62** | B |
| **17** | C | **63** | A |
| **18** | A | **64** | A |
| **19** | D | **65** | A |
| **20** | B | **66** | A |
| **21** | B | **67** | B |
| **22** | B | **68** | C |
| **23** | B | **69** | B |
| **24** | C | **70** | B |
| **25** | B | **71** | B |
| **26** | C | **72** | B |
| **27** | C | **73** | B |
| **28** | B | **74** | A |
| **29** | B | **75** | A |
| **30** | B | **76** | A |
| **31** | B | **77** | A |
| **32** | C | **78** | B |
| **33** | B | **79** | B |
| **34** | C | **80** | B |
| **35** | B | **81** | B |
| **36** | C | **82** | B |
| **37** | C | **83** | B |
| **38** | B | **84** | A |
| **39** | C | **85** | D |
| **40** | B | **86** | A |
| **41** | B | **87** | A |
| **42** | B | **88** | B |
| **43** | C | **89** | B |
| **44** | B | **90** | B |
| **45** | D | **91** | B |
| **46** | C | KB0742 | C |
| **47** | C | | |

CDK9/Cyc T1 IC₅₀ values: A: [<20] nM, B: [20-100] nM, C: [100-500] nM, D: [500-1000] nM, E: [>1000] nM

Kinase selectivity of selected compounds against closely related Cyclin Dependent Kinases was determined using Mobility Shift Assay performed by Carna Biosciences. Data are shown in table 2, the minimum required selectivity is 20 fold in the enzymatic assay.

**Table 2. Kinase Selectivity of CDK9/CycT1 vs closely related CDKs**

| **Compoun d** | **CDK2/Cyc A2** | **CDK4/Cyc D3** | **CDK5/p2 5** | **CDK6/Cyc D3** | **CDK7/CycH/MA T1** |
|---|---|---|---|---|---|
| **9** | C | C | B | B | B |
| **63** | A | A | A | A | A |
| **69** | A | B | A | A | A |
| **71** | A | C | A | A | A |
| **72** | B | C | A | A | A |
| **80** | A | B | A | A | A |
| **81** | A | A | A | A | A |
| **82** | B | A | A | A | A |
| **83** | A | A | A | A | A |
| **86** | A | B | A | A | A |
| **89** | A | A | A | A | A |
| **KB0742** | B | A | A | A | A |

| | | | | | |
|---|---|---|---|---|---|
| A: >100 fold; B: >50 fold; C: 20-50 fold; Tested kinases: CDK2/CycA2, CDK4/Cyc D3, CDK5/p25, CDK6/CycD3, CDK7/CycH/MAT1 | | | | | |

The potential of selected inhibitors to treat cancer was first demonstrated in vitro on a small panel of leukemia cell lines, many of which are dependent on Mcl-1, a downstream target for CDK9.

**Table 3. in vitro cytotoxicity against human cancer cell lines. Data are expressed as IC₅₀ values (µM)**

| **Compound** | MV-4-11 | Kasumi-1 | | KG-1 | OCI AML-3 | HL-60 |
|---|---|---|---|---|---|---|
| **9** | A | B | B | C | C | A |
| **63** | A | A | A | B | C | A |
| **69** | C | D | C | E | C | B |
| **71** | B | A | B | B | B | A |
| **72** | C | C | B | D | C | C |
| **80** | C | C | THP-1 D | D | D | E |
| **81** | C | C | D | D | D | D |
| **82** | C | C | C | C | C | A |
| **83** | B | C | B | C | C | C |
| **86** | A | A | A | A | A | A |
| **89** | B | C | C | C | C | A |
| KB0742 | E | E | E | E | E | E |

| | | | | | | |
|---|---|---|---|---|---|---|
| A = <0.1 µM; B = 0.1-0.3 µM; C = 0.3-0.5 µM; D = 0.5-1.0 µM; E = > 1 µM. Tested human cancer cell lines: MV-4-11, Kasumi-1, THP-1, KG-1, OCI AML-3, HL-60. | | | | | | |

Compound **9** was further tested on a panel of 160 human cancer cell lines (ProLiFiler^{™} service by Reaction Biology, Table 4) proving that its therapeutic potential can be extended to solid tumors.

**Table 4. In vitro cytotoxic activity agains a panel of 160 human cancer cell lines**

| **Cell line** | **Disease** | **IC₅₀ [µM]** |
|---|---|---|
| 786-O | Renal cell carcinoma | 0.062 |
| A172 | Glioblastoma | 0.029 |
| A2058 | Melanoma | 0.031 |
| A-253 | Squamous cell carcinoma | 0.089 |
| A2780 | Ovarian endometrioid adenocarcinoma | 0.017 |
| A375 | Amelanotic melanoma | 0.026 |
| A427 | Lung adenocarcinoma | 0.058 |
| A431 | Squamous cell carcinoma | 0.036 |
| A498 | Renal cell carcinoma | 0.027 |
| A549 | Lung adenocarcinoma | 0.023 |
| AN3-CA | Endometrial adenocarcinoma | 0.041 |
| AsPC-1 | Pancreatic ductal adenocarcinoma | 0.039 |
| BEN | Non-small cell lung carcinoma | 0.058 |
| BT-20 | Invasive ductal carcinoma | 0.118 |
| BV-173 | Chronic myeloid leukemia | 0.049 |
| BxPC-3 | Pancreatic ductal adenocarcinoma | 0.063 |
| Caki-1 | Clear cell renal cell carcinoma | 0.029 |
| Caki-2 | Papillary renal cell carcinoma | 0.101 |
| Cal27 | Squamous cell carcinoma | 0.036 |
| Calu-6 | Lung adenocarcimona | 0.024 |
| Colo 205 | Colon adenocarcinoma | 0.022 |
| COR-L279 | Small cell lung carcinoma | 0.035 |
| COV434 | Ovarian small cell carcinoma | 0.026 |
| Detroit562 | Pharyngeal squamous cell carcinoma | 0.026 |
| DLD-1 | Colon adenocarcinoma | 0.037 |
| DU-145 | Prostate carcinoma | 0.034 |
| DV90 | Lung adenocarcinoma | 0.039 |
| EBC-1 | Lung squamous cell carcinoma | 0.112 |
| EFM-19 | Ductal breast carcinoma | 0.089 |
| EFM-192A | Breast carcinoma | 0.116 |
| EFO-27 | Ovarian mucinous papillary adenocarcinoma, metastatic site: Omentum | 0.032 |
| EPLC-272H | Epidermoid lung carcinoma | 0.032 |
| FaDu | Squamous Cell Carcinoma | 0.110 |
| GCT | Histiocytoma | 0.030 |
| H4 | Astrocytoma (Neuroglioma) | 0.060 |
| HCC 1569 | Ductal breast carcinoma | 0.054 |
| HCC202 | Ductal breast carcinoma | 0.090 |
| HCC38 | Ductal breast carcinoma | 0.063 |
| HCC827 | Lung adenocarcinoma (non-small cell lung cancer) | 0.043 |
| HCT116 | Colorectal carcinoma | 0.020 |
| HCT-15 | Colon adenocarcinoma | 0.066 |
| HEC-1-A | Endometrial adenocarcinoma | 0.064 |
| HEC-1-B | Endometrial adenocarcinoma | 0.046 |
| HeLa | Human papillomavirus-related endocervical adenocarcinoma | 0.027 |
| Hep3B2.1-7 | Childhood hepatocellular carcinoma | 0.057 |
| HL-60 | Adult acute myeloid leukemia | 0.066 |
| Hs 746T | Gastric adenocarcinoma | 0.043 |
| HT-1080 | Fibrosarcoma | 0.119 |
| HT-29 | Colon adenocarcinoma | 0.050 |
| HuH7 | Adult hepatocellular carcinoma | 0.037 |
| Hutu 80 | Duodenal adenocarcinoma | 0.089 |
| Ishikawa | Endometrial adenocarcinoma | 0.040 |
| J82 | Bladder carcinoma | 0.080 |
| JIMT-1 | Ductal breast carcinoma | 0.031 |
| Jurkat | Childhood T cell acute lymphoblastic leukemia | 0.072 |
| JVM-3 | B-cell prolymphocytic leukemia | 0.012 |
| K562 | Chronic myeloid leukemia | 0.111 |
| KARPAS 299 | Anaplastic large cell lymphoma | 0.034 |
| Kato III | Gastric signet ring cell adenocarcinoma | 0.034 |
| KG-1 | Adult acute myeloid leukemia | 0.087 |
| KG-1a | Adult acute myeloid leukemia | 0.163 |
| KMS-11 | Plasma cell myeloma | 0.047 |
| KMS-12-BM | Plasma cell myeloma | 0.097 |
| KP-4 | Pancreatic carcinoma | 0.096 |
| LN229 | Glioblastoma | 0.099 |
| LnCap | Prostate carcinoma | 0.039 |
| LOU-NH91 | Lung squamous cell carcinoma | 0.085 |
| LOVO | Colon adenocarcinoma | 0.022 |
| LP-1 | Plasma cell myeloma | 0.046 |
| M07e | Childhood acute megakaryoblastic leukemia | 0.042 |
| MCAS | Ovarian mucinous cystadenocarcinoma | 0.010 |
| MCF-7 | Invasive ductal carcinoma | 0.012 |
| MDA MB 231 | Breast adenocarcinoma | 0.035 |
| MDA MB 435 | Amelanotic melanoma | 0.091 |
| MDA-MB-468 | Breast adenocarcinoma | 0.030 |
| MEC-1 | Chronic lymphocytic leukemia | 0.067 |
| MG63 | Osteosarcoma | 0.096 |
| Mia PaCA 2 | Pancreatic ductal adenocarcinoma | 0.020 |
| MKN-1 | Gastric adenosquamous carcinoma | 0.071 |
| MKN-45 | Gastric adenocarcinoma | 0.025 |
| MM.1S | Plasma cell myeloma | 0.034 |
| MOLM-13 | Adult Acute Myeloid Leukemia | 0.028 |
| MOLT-4 | Adult T acute lymphoblastic leukemia | 0.031 |
| MV4-11 | Childhood acute monocytic leukemia | 0.021 |
| NALM-6 | Adult B acute lymphoblastic leukemia | 0.020 |
| NCI-H1048 | Lung small cell carcinoma | 0.049 |
| NCI-H1299 (H1299) | Non-small cell lung cancer | 0.035 |
| NCI-H1437 | Non-small cell lung cancer | 0.049 |
| NCI-H1563 | Non-small cell lung cancer | 0.064 |
| NCI-H1573 | Lung adenocarcinoma | 0.045 |
| NCI-H1581 | Non-small cell lung cancer | 0.078 |
| NCI-H1703 | Squamous cell lung carcinoma | 0.088 |
| NCI-H1838 | Non-small cell lung cancer | 0.033 |
| NCI-H2009 | Lung adenocarcinoma | 0.081 |
| NCI-H2110 | Non-small cell lung cancer | 0.117 |
| NCI-H2228 (H2228) | Non-small cell lung cancer | 0.098 |
| NCI-H226 | Squamous Cell Carcinoma; Mesothelioma | 0.080 |
| NCI-H2286 | Small cell lung cancer | 0.038 |
| NCI-H292 | Lung mucoepidermoid carcinoma | 0.043 |
| NCI-H441 | Lung papillary adenocarcinoma | 0.136 |
| NCI-H460 (H460) | Lung large cell carcinoma | 0.043 |
| NCI-H82 | Lung small cell carcinoma | 0.099 |
| NCI-H838 | Non-small cell lung cancer | 0.036 |
| NCI-N87 | Gastric tubular adenocarcinoma | 0.031 |
| OCI-AML3 | Adult acute myeloid leukemia | 0.032 |
| OCI-AML5 | Adult acute myeloid leukemia | 0.024 |
| OCI-LY19 | Diffuse large B-cell lymphoma germinal center B-cell type | 0.024 |
| OPM-2 | Plasma cell myeloma | 0.061 |
| OV56 | Ovarian serous adenocarcinoma | 0.030 |
| OVCAR-3 | Ovarian serous adenocarcinoma | 0.066 |
| OVK18 | Ovarian endometrioid adenocarcinoma | 0.044 |
| P31/FUJ | Childhood acute myeloid leukemia | 0.035 |
| PANC-1 | Pancreatic ductal adenocarcinoma | 0.072 |
| PC3 | Prostate carcinoma | 0.041 |
| PC-9 | Lung adenocarcinoma | 0.097 |
| PSN1 | Pancreatic adenocarcinoma | 0.100 |
| Raji | EBV-related Burkitt lymphoma | 0.052 |
| RD | Rhabdomyosarcoma | 0.037 |
| RERF-LC-Ad2 | Lung adenocarcimona | 0.096 |
| RERF-LC-MS | Lung adenocarcimona | 0.091 |
| RKO | Colon carcinoma | 0.021 |
| RL95-2 | Endometrial adenosquamous carcinoma | 0.017 |
| RPMI 8226 | Plasma cell myeloma | 0.069 |
| RT-4 | Bladder carcinoma (papilloma) | 0.101 |
| Saos-2 EC | Osteosarcoma | 0.029 |
| SCLC-21H | Small cell lung carcinoma | 0.055 |
| SCH | Gastric choriocarcinoma | 0.065 |
| SiHa | Squamous cell carcinoma | 0.046 |
| SJSA-1 | Osteosarcoma | 0.033 |
| SK.BR-3 | Breast adenocarcinoma | 0.076 |
| SK-ES-1 | Ewing sarcoma | 0.064 |
| SK-LU-1 | Lung adenocarcimona | 0.040 |
| SK-MEL-3 | Malignant melanoma | 0.138 |
| SK-N-FI | Neuroblastoma | 0.068 |
| SK-N-MC | Askin tumor (Ewing's sarcoma family) | 0.048 |
| SK-N-SH | Neuroblastoma | 0.024 |
| SK-OV3 | Ovarian serous cystadenocarcinoma | 0.038 |
| SNU-1 | Gastric adenocarcinoma | 0.029 |
| SNU-16 | Gastric adenocarcinoma | 0.045 |
| SNU-216 | Gastric adenocarcinoma | 0.040 |
| SNU840 | Malignant ovarian Brenner tumor | 0.029 |
| SU-DHL-5 | Diffuse large B-cell lymphoma germinal center B-cell type | 0.018 |
| SU-DHL-6 | Diffuse large B-cell lymphoma germinal center B-cell type | 0.053 |
| SW-1783 | Anaplastic astrocytoma | 0.097 |
| SW480 | Colon adenocarcinoma | 0.030 |
| SW620 | Colon adenocarcinoma | 0.030 |
| SW948 | Colon adenocarcinoma | 0.028 |
| T-24 | Bladder carcinoma | 0.051 |
| T-47D | Invasive ductal carcinoma | 0.066 |
| T84 | Colon adenocarcinoma | 0.070 |
| T98G | Glioblastoma | 0.014 |
| U118MG | Glioblastoma (Astrocytoma) | 0.031 |
| U251MG | Glioblastoma (Astrocytoma) | 0.032 |
| U-266 | Plasma cell myeloma | 0.122 |
| U2OS | Osteosarcoma | 0.026 |
| U87MG | Glioblastoma | 0.063 |
| U-937 | Adult histiocytic lymphoma | 0.025 |
| UACC-62 | Melanoma | 0.039 |
| UM-UC-3 | Bladder carcinoma | 0.011 |
| ZR-75-1 | Invasive ductal carcinoma | 0.033 |

Toxicity to a panel of 7 non-cancer cell lines was evaluated using the same methodology. Most of the tested compounds show high selectivity index (>100) with respect to MV-4-11 leukemia target cells.

**Table 5. Selectivity index - target leukemia MV-4-11 cell line vs non-cancer cell lines**

| **Compound** | Papi | IPrO | NHDF | HME | BEAS-2B | HIEC-6 | PBMC^{a} |
|---|---|---|---|---|---|---|---|
| **9** | D | D | D | B | D | D | E |
| **63** | A | A | A | A | A | A | C |
| **69** | A | A | A | B | A | A | B |
| **71** | A | A | A | A | A | A | E |
| **72** | B | A | C | B | D | A | E |
| **80** | A | A | B | B | A | A | C |
| **81** | B | A | C | A | C | B | D |
| **82** | A | A | A | A | A | A | C |
| **83** | C | B | C | A | B | B | E |
| **86** | A | A | B | A | B | A | D |
| **89** | A | B | B | A | C | C | C |
| KB0742 | E | D | E | nt | D | C | D |

A: SI >200; B: SI >150; C: SI >100; D: SI >50; E: SI >10. ^{a} 4 h washout to mimic short exposure, readout after 24 h. List of cell lines: BEAS-2B = Human bronchial epithelial cells; IPrO = Immortalized primary ovary cells; Papi = Pancreatic primary cells, hTERT-immortalized; HME = Human mammary epithelial cell line, hTERT-immortalized; NHDF = Normal human dermal fibroblasts; PBMC = Peripheral Blood Mononuclear Cell

Pharmacokinetic characteristics of selected compounds were determined in male CD-1 mice following intravenous (IV) and peroral (PO) administration by Bienta/Enamine. Levels of the test compound were determined by LC-MS/MS in the blood plasma samples over time after a single dose. All our tested compounds show sufficient bioavailability after PO administration and reasonably short half-lives necessary to reduce toxicity originating from sustained target suppresion.

**Table 6. In vivo Pharmacokinetic data in CD-1 mice**

| | **9** | **18** | **63** | **71** | **82** |
|---|---|---|---|---|---|
| Half-life (IV), min | 332 | 184 | 38 | 82 | 18 |
| Half-life (PO), min | 284 | 170 | 66 | 91 | 61 |
| Tₘₐₓ (min) | 480 | 240 | 120 | 120 | 30 |
| Clearance (ml/min/kg) | 48 | 180 | 140 | 140 | 160 |
| Bioavailability (%) | 46 | 33 | 25 | 18 | 85 |

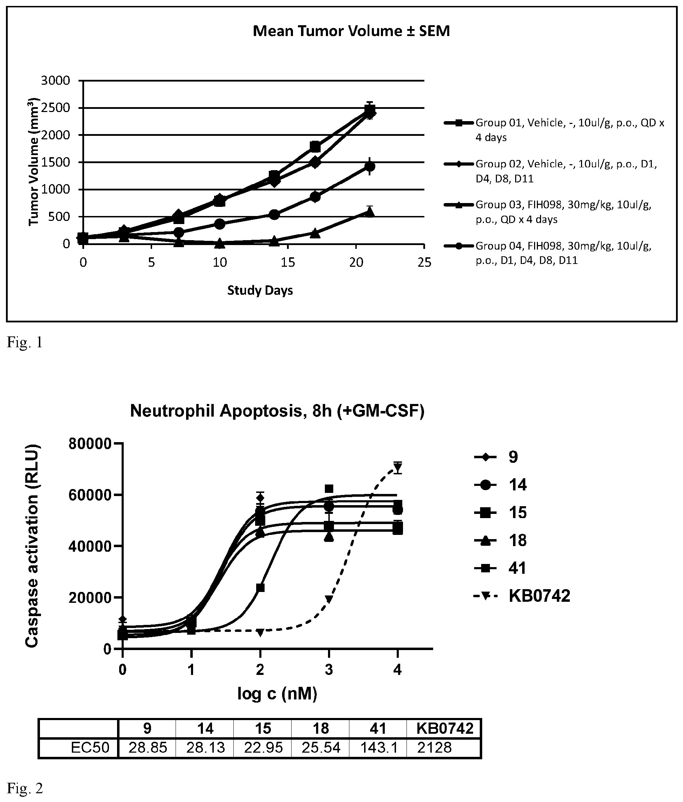

Preliminary in vivo efficacy study of compound 9 in mouse subcutaneous MV-4-11 xenograft model in female NOD/SCID mice was performed by CrownBioscience. The data shown in Fig. 1 confirm very high efficacy of compound 9, which significantly reduced tumour growth after PO administration of 30 mg/kg for two weeks (tumour size reduction by 98% in QDx4 regimen; and by 56% in TW regimen).

The ability of CDK9 inhibitors to induce apoptosis in neutrophils was demonstrated in vitro using neutrophils freshly isolated from peripheral blood of healthy human donors with use of magnetic separation (EasySep^{™}). (Fig. 2, Table 7)

**Table 7. Neutrophil apoptosis (Caspase Activation)**

| **Compound** | EC₅₀ (nM) |
|---|---|
| **9** | 29 |
| **14** | 28 |
| **15** | 23 |
| **18** | 26 |
| **41** | 143 |
| **KB0742** | 2128 |

### Description of the methods:

### Cell culture

MV-4-11, OCI-AML-3, KG-1, HL-60, CCRF-CEM, THP-1, Kasumi-1 cancer cell lines were purchased either from ATCC or DSMZ. Non-tumorigenic primary immortalized epithelial cells (BEAS-2B, iPRO, HME, Papi) were a kind gift from Z. Kečkešová except for HIEC-6 which was from ATCC. HEK-293 and NHDF cells were also from ATCC. PBMC were isolated from peripheral blood of healthy volunteers. Cells were grown the media recommended by the manufacturer (DMEM, RPMI-1640, Ham-F12) supplemented with 10% (v/v) fetal calf serum and 2 mM glutaMax,^{™} or eventually, commercial supplements containing growth factors (primary cells). The cells were grown under a humidified atmosphere containing 5% CO₂ at 37 °C. Cell lines were routinely tested for the absence of Mycoplasma infection.

### NanoBRET intracellular kinase assay

To quantify the intracellular affinity of a test compounds to the full-length protein kinase CDK9/Cyclin T1 expressed in living cells, we used NanoBRET^{™} Target Engagement Intracellular Kinase Assay (CDK9/Cyclin T1 TE Assay, Promega) according to the manufacturer's protocol with minor modifications. Briefly, HEK293-T cells were transiently transfected using DNA:lipid complex prepared in the following ratios: 9.0 µg/mL of Transfection Carrier DNA (Promega), 0.9 µg/mL CCNT1 Expression Vector (Promega) and 0.1 µg/mL NanoLuc-CDK9 Fusion Vector (Promega) in 1 mL of assay medium (Opti-MEM^{®} I Reduced Serum Medium, no phenol red,Gibco^{™}). The DNA mixture was mixed thoroughly. Next, 30 µl of FuGENE^{®} HD Transfection Reagent (Promega) was added, mixed gently by tube inversion and incubated for 20 minutes at RT to form DNA:lipid complexes. In the meantime, HEK293-T passaged one day prior to the transfection, were trypsinized, centrifuged and resuspended in assay medium containing 1% FBS to obtain 3 x 10⁵ cells/mL. The DNA:lipid complex at a 20:1 ratio was then added to the cell suspension and 20 µL of the mixture was seeded into each well of a white 384-well plate (Nunc). After 24 h incubation at 37°C, 5% CO₂ in a humidified tissue culture incubator, tested compounds were dispensed to the cells using ECHO 650 liquid handler (Beckman Coulter) and the plate was incubated in humidified tissue culture incubator for 1 hour. After that, 1 µl of Tracer 8 (Promega) diluted in Tracer Dilution Buffer (Promega) at final concentration of 60 nM was added by Mantis^{®} liquid dispenser (Formulatrix^{®}) and the plate was incubated for the next 45 minutes in an incubator. The plate was then equilibrated to RT for 15 minutes after which 10 µL of Substrate: Inhibitor master mix containing 1:166 ratio of NanoBret^{™} Nano-Glo^{®} Substrate (Promega) and 1:500 Exracellular NanoLuc^{®} Inhibitor (Promega) in the assay medium were added to each well by Mantis^{®}. To reach consistent signal, the plate was then incubated for the additional 15 minutes at RT. BRET signal at donor (450 nm) and acceptor (610 nm) emission wavelength was measured using Tecan Spark^{®} multimode reader. The control samples in the absence of Tracer 8 were used for background correction. BRET ratio was calculated using NanoBRET^{™} ratio equation, including background correction: [(Acceptor sample / Donor sample) - (Acceptor no-tracer sample / Donor no-tracer sample) x 1 000. GraphPad Prism software 5 was employed to determine the IC₅₀ values using the non-linear regression model.

### Kinase Selectivity - Mobility Shift Assay

Kinase selectivity against closely related Cyclin dependent kinases 2, 4, 5, 6 and 7 was tested using Mobility Shift Assay performed by Carna Biosciences, Inc using following procedure:

The 4× Substrate/ATP/Metal solution was prepared with kit buffer (20 mmol/L HEPES, 0.01% Triton X-100, 5 mmol/L DTT, pH7.5), and 2x kinase solution was prepared with assay buffer (20 mmol/L HEPES, 0.01% Triton X-100, 1 mmol/L DTT, pH7.5).

The 5 µL of 4× compound solution, 5 µL of 4× Substrate/ATP/Metal solution, and 10 µL of 2× kinase solution were mixed and incubated in a well of polypropylene 384 well microplate for 1 or 5 hour(s)* at room temperature. (*; depend on kinase)
70 µL of Termination Buffer (127 mM HEPES, 0.01% Triton X-100, 26.7 mM EDTA-2Na, 1% DMSO, pH7.5) was added to the well.

The reaction mixture was applied to LabChipTM system (Revvity) or BioPhase^{™} 8800 system (AB Sciex), and the product and substrate peptide peaks were separated and quantitated.

The kinase reaction was evaluated by the product ratio calculated from peak heights(when using LabChip^{™} system) or area (when using BioPhase^{™} 8800 system) of product(P) and substrate(S) peptides (P/(P+S)).

The readout value of reaction control (complete reaction mixture) was set as a 0% inhibition, and the readoutvalue of background (Enzyme(-)) was set as a 100% inhibition, then the percent inhibition of each test solutionwas calculated.

IC50 value was calculated from concentration vs.%Inhibition curves by fitting to a four parameter logistic curve.

### Cytotoxicity assays

The toxicity of the compounds was determined as follows: 384-well white plates (NUNC) were pre-spotted with the concentrated DMSO stock solutions of the compounds with use of Echo^{®} 550 dispenser so that the final inhibitor concentration in the well was 0.01-50 000 nM depending on cell type. Then, 20 µl of the cell suspension was added to reach a concentration of 2 x 10⁵ / ml (cell lines) or 2 x 10⁶ (PBMC). After 24 hours, 20 µl of CellTiter-Glo^{®} reagent (Promega) was added to each well and luminescence was measured according to the manufacturer's protocol. A non-linear regression analysis was performed using GraphPad Prism 5 software to calculate IC₅₀ values.

### 4h-washout modification

In addition to the standard workflow where the compound is present throughout the incubation period, toxicity was also measured after a 4h-pulse treatment after which the compound-containing media was replaced by compound-free medium for the remainder of the incubation period (24h in total). The washout was done by centrifugation (450 x g, 5 min), pipetting off the medium and resuspension of the pellet in an equal volume of fresh medium (all twice). The washing step simulates in vivo conditions where CDK9 inhibitors such as AZD4573 and VIP152 are rapidly metabolized and excreted from the body after a single intravenous infusion, as described in clinical studies (Barlaam B. et al. J. Med. Chem. 2020, 63, 15564-15590; Licking U. et al. J. Med. Chem. 2021, 64, 11651-11674). Cytotoxicity was evaluated 20 hours after washing the compounds using Cell Titer-Glo^{®} reagent as described above.

### Neutrophil isolation

Neutrophils were isolated from the peripheral blood of healthy volunteers by immunomagnetic negative selection with use of EasySep Direct Neutrophil Isolation kit (StemCell Technologies, Cat.No.19666). Briefly, approx. 10 mL of whole venous blood was drawn in K₂EDTA tube, mixed gently and transferred into a 50 mL Falcon tube. 500 µL of antibody cocktail and 500 µL of magnetic beads were added, the tube was gently mixed and left for 5 min at RT. The volume was topped up to 50 mL with PBS containing 1 mM EDTA and mixed with pipette. The sample was incubated for 10 min in the magnet, the supernatant containing the desired population was removed and the purification procedure was repeated once more with another aliquot of magnetic beads. Neutrophil containing supernatant was transferred, cells were counted and resuspended in RPMI-1640 medium with 10%FBS at concentration of 2 × 10⁶/ml.

### Apoptosis evaluation

Cells were seeded in 384-well white plates (NUNC) at 20000 cells per well in 10 µL of culture media. Compounds were added from 10 mM DMSO stocks by digital dispenser d300e (TECAN) to reach indicated final concentrations. After 8 h, 10 µL of caspase3/7-Glo^{™} reagent (Promega) was added and the luminescence was measured. Dose response curves were plotted against log c (compounds) and EC₅₀ values were calculated by non-linear regression method using GraphPad Prism.

### PBMC isolation

Peripheral blood mononuclear cells (PBMC) were isolated from the peripheral blood of healthy volunteers by Ficoll-Paque density gradient with use of SepMate^{™} tubes. Briefly, ~10 mL blood was diluted with 25 mL PBS containing 2%FBS and gently layered over 15 mL of Ficoll-Paque^{™} (Sigma) in a SepMate tube (Stem Cell Technologies). Following sample centrifugation at 500 × g for 30 min at RT (no brakes), the supernatant was collected in a separate 50 mL Falcon tube and topped up to 50 mL with PBS containing 2%FBS. After another round of centrifugation (300 × g, 10 min, RT), 10 mL of RBC lysis buffer (150 mM NH₄Cl, 10 mM KHCO₃, 0.1 mM EDTA) was added to the pellet, resuspended and incubated for 5 min, to lyse remaining red blood cells. The sample was centrifuged (300 × g, 10 min, RT) and washed with PBS + 2% FBS. The pellet containing PBMC was resuspended in RPMI-1640 complete culture media (1% penicillin/streptomycin, 10% FBS), counted and aliquots were frozen at -80 °C. Prior to the experimentation, a frozen aliquot was thawed, live cells were counted and the cells were diluted to a seeding concentration of 2 × 10⁶/mL.

### Industrial Applicability

The compounds disclosed in this patent are useful as active pharmaceutical substances effective against cancer, neutrophilic diseases and other diseases connected with overexpression of CDK9 and oncoproteins MCL-1 and MYC.

## Claims

1. A compound of general formula I 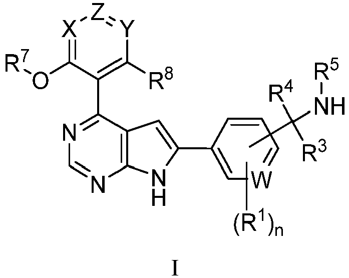
W is selected from CH, CR¹ or N;
R¹ is on each occurence independently selected from halogen, C1-C4 alkyl, hydroxy, C1-C4 alkoxy; or R¹ forms a ring with R³ or R⁵ as described herein below;
n = 0, 1 or 2;
R³ is selected from C1-C6 alkyl, C1-C3 haloalkyl, C1-C3 hydroxyalkyl, C3-C8 cycloalkyl, C4-C8 cycloalkylalkyl, hydrogen;
or R³ and R¹ which is in a position adjacent to the position of the substituent -C(R³)(R⁴)-NH(R⁵), together with the carbon atoms linking them, form a 5- to 8-membered saturated cycloalkyl or heterocycloalkyl ring, wherein the heterocycloalkyl ring contains 1 or 2 heteroatoms selected from N, O, S, and wherein the ring formed by R³ and R¹ together with the carbon atoms linking them can optionally be substituted by one, two or three substituents selected from halogen (preferably fluorine), C1-C4 alkyl, amino, hydroxy, C1-C4 alkoxy;
R⁴ is selected from C1-C6 alkyl, C3-C8 cycloalkyl, C4-C8 cycloalkylalkyl, hydrogen;
or R³ and R⁴ together with the carbon atom to which they are bound form a 3- to 8-membered cycloalkyl or heterocycloalkyl or spirocycloalkyl ring, wherein the heterocycloalkyl ring contains 1 or 2 heteroatoms selected from N, O, S, and wherein the ring formed by R³ and R⁴ together with the carbon atom to which they are bound can optionally be substituted by one, two or three substituents selected from halogen (preferably fluorine), C1-C4 alkyl, amino, hydroxy, C1-C4 alkoxy;
or R³ and R⁵ together with the carbon and nitrogen atom linking them form a 5- to 8-membered (preferably 5- to 6-membered) saturated heterocycloalkyl ring, optionally containing 1 or 2 further heteroatoms selected from N, O, S, and optionally substituted by one, two or three substituents selected from halogen (preferably fluorine), C1-C4 alkyl, amino, hydroxy, C1-C4 alkoxy;
R⁵ is selected from C1-C6 alkyl, C1-C4 haloalkyl (preferably trifluoromethyl), C3-C8 cycloalkyl, C4-C8 cycloalkylalkyl, hydrogen;
or R⁵ and R¹ when R¹ is in a position adjacent to the position of the substituent -C(R³)(R⁴)-NH(R⁵), together with the carbon and nitrogen atoms linking them, form a 5- to 8-membered (preferably 5-to 6-membered) heterocycloalkyl ring, optionally containing 1 or 2 further heteroatoms selected from N, O, S, and optionally substituted by one, two or three substituents selected from halogen (preferably fluorine), C1-C4 alkyl, amino, hydroxy, C1-C4 alkoxy;
X, Y and Z are selected from CH, C-R⁶ and N, wherein one or two of X, Y, Z are N;
R⁶ is selected from halogen, C1-C4 alkyl, C1-C4 halogenalkyl, C1-C4 alkoxy, C1-C4 halogenalkoxy and hydrogen;
R⁷ is selected from C1-C4 alkyl, trifluoromethyl, C3-C6 cycloalkyl;
R⁸ is selected from C1-C4 alkyl, C1-C4 alkoxy, trifluoromethyl, hydrogen;
and pharmaceutically acceptable salts thereof.

2. The compound according to claim 1, wherein the substituent -C(R³)(R⁴)-NH(R⁵) is bound in position meta or para relative to the pyrrole ring attachment.

3. The compound according to claim 1 or 2, wherein
- X and Y are N and Z is C, or
- X is N and Y and Z are C, or
- Z is N and X and Y are C.

4. The compound according to any one of claims 1 to 3, wherein R¹ is independently selected from fluorine, hydroxy, C1-C4 alkyl, C1-C4 alkoxy, and/or n is 1.

5. The compound according to any one of claims 1 to 4, wherein R³ is selected from methyl, ethyl, n-propyl, 2-methylpropyl, butyl, i-propyl, trifluoromethyl, hydroxymethyl, cyclopropyl, cyclobutyl, cyclopentyl, cyclohexyl, cyclobutylmethyl and trifluoromethyl, and R⁴ is hydrogen; or
R³ and R⁴ are selected from methyl, ethyl, n-propyl, i-propyl, butyl and 2-methylpropyl.

6. The compound according to any one of claims 1 to 3, wherein R³ and R¹ which is in a position adjacent to the position of the substituent -C(R³)(R⁴)-NH(R⁵), together with the carbon atoms linking them, form a 5- or 6-membered cycloalkyl, or a heterocycloalkyl ring containing 1 or 2 heteroatoms selected from N, O, S, and preferably having 6 atoms,
wherein the ring formed by R³ and R¹ together with the carbon atoms linking them is optionally substituted by one, two or three substituents, selected from fluorine and C1-C3 alkyl, such as methyl.

7. The compound according to any one of claims 1 to 4, wherein R³ and R⁵ together with the carbon and nitrogen atom to which they are attached form a 5- to 6-membered heterocycloalkane ring, optionally containing 1 or 2 further heteroatoms selected from N, O, S, wherein the ring may optionally be substituted by one, two or three substituents selected from halogen, methyl, amino, hydroxy, methoxy.

8. The compound according to any one of claims 1 to 6, wherein R⁵ is selected from hydrogen, methyl, ethyl, cyclopropyl, i-propyl and butyl.

9. The compound according to any one of claims 1 to 3 and 5 to 6, wherein R⁵ and R¹ when R¹ is in a position adjacent to the position of the substituent -C(R³)(R⁴)-NH(R⁵), together with the carbon and nitrogen atoms linking them, form a 5- to 6-membered saturated heterocycloalkane ring, optionally containing 1 or 2 further heteroatoms selected from N, O, S, and optionally substituted by one, two or three substituents selected from halogen and C1-C4 alkyl.

10. The compound according to any one of claims 1 to 9 for use as a medicament.

11. The compound according to any one of claims 1 to 9 for use in the treatment of tumor, non-tumor and/or cancer disease associated with cell hyperproliferation related to overexpression of antiapoptotic factors MCL-1 and cMYC, or for the treatment of neutrophilic diseases.

12. The compound according to any one of claims 1 to 9 for use in the treatment of leukemias, solid tumors, lymphomas, neutrophilic dermatoses or netrophilic diseases.

13. The compound according to any of claims 1 to 9 for use in the treatment of a leukemia selected from Acute Myelocytic Leukemia (AML), Relapsed/Refractory Chronic Lymphocytic Leukemia (CLL) and Chronic Myelomonocytic Leukemia (CMML); or of a lymphoma selected from Hodgkin lymphoma; diffuse large B-cell lymphoma, high-grade B-cell lymphoma, cutaneous T-cell lymphoma; Extranodal Marginal Zone B-Cell Lymphoma (Mucosa-Associated Lymphoid Tissue or MALT-Lymphoma) and Peripheral T-Cell Lymphoma (PTCL); or of a solid tumor selected from sarcoma, breast (Human Epidermal Growth Factor Receptor 2 Negative Breast Cancer, Triple-negative breast cancer), lung, ovarian, melanoma and pancreatic cancers including Castration-Resistant Prostate Cancer.

14. The compound according to any of claims 1 to 9 for use in the treatment of a neutrophilic disease selected from autoimmune disease like rheumatoid arthritis, systemic lupus erythematosus or antiphospholipid syndrome, systemic sclerosis and Sjögren's syndrome; or of an inflammatory disease selected from cystic fibrosis, severe asthma, chronic obstructive pulmonary disease, bronchiectasis, diabetic nephropathy, regeneration after myocardial infarction and acute liver failure; or of a neutrophilic dermatose selected from psoriasis, prurigo nodularis, palmoplantar pustulosis, hidradenitis suppurativa and pyoderma gangrenosum.

15. A pharmaceutical preparation comprising at least one compound according to any one of claims 1 to 9 and at least one pharmaceutically acceptable excipient, carrier and/or solvent.
